(19) Europäisches Patentamt
European Patent Office
Office européen des brevets

(11) EP 1 458 723 B1

(12) **EUROPEAN PATENT SPECIFICATION**

(45) Date of publication and mention of the grant of the patent:
**15.10.2008 Bulletin 2008/42**

(21) Application number: **02797421.1**

(22) Date of filing: **18.12.2002**

(51) Int Cl.:
*C07D 491/00* (2006.01) *A61K 31/40* (2006.01)
*A61P 35/00* (2006.01)

(86) International application number:
**PCT/US2002/040598**

(87) International publication number:
**WO 2003/062241 (31.07.2003 Gazette 2003/31)**

(54) **FUSED HETEROCYCLIC SUCCINIMIDE COMPOUNDS AND ANALOGS THEREOF, MODULATORS OF NUCLEAR HORMONE RECEPTOR FUNCTION**

KONDENSIERTE HETEROCYCLISCHE SUCCINIMIDVERBINDUNGEN UND DEREN ANALOGA, MODULATOREN DER FUNKTION VON NUKLEÄREN HORMONREZEPTOREN

COMPOSES DE SUCCINIMIDE HETEROCYCLIQUES LIES PAR FUSION ET SUBSTANCES ANALOGUES, MODULATEURS DE LA FONCTION DU RECEPTEUR HORMONAL NUCLEAIRE

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **19.12.2001 US 25116**

(43) Date of publication of application:
**22.09.2004 Bulletin 2004/39**

(60) Divisional application:
**07015374.7 / 1 854 798**

(73) Proprietor: **Bristol-Myers Squibb Company**
**Princeton NJ 08543-4000 (US)**

(72) Inventors:
- **SALVATI, Mark, E.**
  **Lawrenceville, NJ 08648 (US)**
- **BALOG, James, Aaron**
  **Lambertville, NJ 08530 (US)**
- **PICKERING, Dacia, A.**
  **Lawrenceville, NJ 08648 (US)**
- **GIESE, Soren**
  **New Hope, PA 18938 (US)**
- **FURA, Aberra**
  **Lawrencville, NJ 08648 (US)**
- **LI, Wenying**
  **Middletown, CT 06457 (US)**
- **PATEL, Ramesh, N.**
  **Bridgewater, NJ 08807 (US)**
- **HANSON, Ronald, L.**
  **Morris Plains, NJ 07950 (US)**
- **MITT, Toomas**
  **Plainsboro, NJ 08536 (US)**
- **ROBERGE, Jacques**
  **Princeton, NJ 08540 (US)**
- **CORTE, James, R.**
  **Lawrenceville, NJ 08648 (US)**
- **SPERGEL, Steven, H.**
  **Warrington, PA 18966 (US)**
- **RAMPULLA, Richard, A.**
  **Flemington, NJ 08822 (US)**
- **MISRA, Raj**
  **Hopewell, NJ 08525 (US)**
- **XIAO, Hai-Yun**
  **Princeton, NJ 08540 (US)**

(74) Representative: **Vossius & Partner**
**Siebertstrasse 4**
**81675 München (DE)**

(56) References cited:

| | |
|---|---|
| **WO-A-02/00617** | **WO-A-02/24702** |
| **WO-A-91/06297** | **US-A- 3 215 597** |
| **US-A- 3 261 845** | **US-A- 3 320 270** |
| **US-A- 4 892 943** | **US-A- 6 090 837** |
| **US-B1- 6 384 050** | **US-B1- 6 448 284** |

- **PATENT ABSTRACTS OF JAPAN vol. 012, no. 446 (C-546), 24 November 1988 (1988-11-24) & JP 63 170383 A (NIPPON ZEON CO LTD; others: 01), 14 July 1988 (1988-07-14)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- CH. GROGAN: "bicyclic imides and isoindolines." JOURNAL OF MEDICINAL CHEMISTRY., vol. 6, no. 6, 1963, pages 802-805, XP002351028 USAMERICAN CHEMICAL SOCIETY. WASHINGTON.
- EARL BOCKSTAHLER: "7-oxabicyclo[2.2.1.] heptane-2,3-dicarboxi mides with anticonvulsant activity." JOURNAL OF MEDICINAL CHEMISTRY., vol. 11, no. 3, 1968, pages 603-606, XP002351029 USAMERICAN CHEMICAL SOCIETY. WASHINGTON.
- ANTONIO GRONDIN: "benzotriazole maleimide as a bifunctional reactant for sers." JOURNAL OF THE CHEMICAL SOCIETY, PERKIN TRANSACTIONS 2., no. 2, 2001, pages 2136-2141, XP002351030 GBCHEMICAL SOCIETY. LETCHWORTH.
- CHRISTOPHER WALTER: "free-energy profile for a host accelarated diels-alder reaction:" ANGEWANDTE CHEMIE. INTERNATIONAL EDITION., vol. 34, no. 2, 1995, pages 217-219, XP002351031 DEWILEY VCH VERLAG, WEINHEIM.
- DATABASE HCAPLUS [Online] WARD ET AL.: 'Diels-Alder reactions of 2H-thiopyrans', XP002963880 Retrieved from STN Database accession no. 1990:440505 & TETRAHEDRON LETTERS vol. 31, no. 6, 1990, pages 845 - 848

## Description

The present invention relates to two fused cyclic compounds, to their use in the treatment of nuclear hormone receptor-associated conditions such as cancer, and to pharmaceutical compositions containing such compounds.

Nuclear hormone receptors (NHR's) constitute a large super-family of ligand-dependent and sequence-specific transcription factors. Members of this family influence transcription either directly, through specific binding to the promoter target genes (Evans, in Science 240: 889-895 (1988)), or indirectly, via protein-protein interactions with other transcription factors (Jonat et al., Cell 62: 1189-1204 (1990), Schuele et al., Cell 62: 1217-1226 (1990), and Yang-Yen et al., Cell 62: 1205-1215 (1990)). The nuclear hormone receptor super-family (also known in the art as the "steroid/thyroid hormone receptor super-family") includes receptors for a variety of hydrophobic ligands, including cortisol, aldosterone, estrogen, progesterone, testosterone, vitamine D3, thyroid hormone and retinoic acid (Evans, 1988, **supra**). In addition to these conventional nuclear hormone receptors, the super-family contains a number of proteins that have no known ligands, termed orphan nuclear hormone receptors (Mangelsdorf et al., Cell 83: 835-839 (1995), O'Malley et al., Mol. Endocrinol. 10: 1293 (1996), Enmark et al., Mol. Endocrinol. 10, 1293-1307 (1996) and Giguere, Endocrin. Rev. 20, 689-725 (1999)). The conventional nuclear hormone receptors are generally transactivators in the presence of ligand, and can either be active repressors or transcriptionally inert in the absence of ligand. Some of the orphan receptors behave as if they are transcriptionally inert in the absence of ligand. Others, however, behave as either constitutive activators or repressors. These orphan nuclear hormone receptors are either under the control of ubiquitous ligands that have not been identified, or do not need to bind to a ligand to exert these activities.

In common with other transcription factors, the nuclear hormone receptors have a modular structure, being comprised of three distinct domains: an N-terminal domain of variable size containing a transcriptional activation function AF-1, a highly conserved DNA binding domain and a moderately conserved ligand-binding domain. The ligand-binding domain is not only responsible for binding the specific ligand but also contains a transcriptional activation function called AF-2 and a dimerisation domain (Wurtz et al., Nature Struc. Biol. 3, 87-94 (1996), Parker et al., Nature Struc. Biol. 3, 113-115 (1996) and Kumar et al., Steroids 64, 310-319 (1999)). Although the overall protein sequence of these receptors can vary significantly, all share both a common structural arrangement indicative of divergence from an ancestral archetype, and substantial homology (especially, sequence identity) at the ligand-binding domain.

The steroid binding nuclear hormone receptors (SB-NHR's) comprise a sub-family of nuclear hormone receptors. These receptors are related in that they share a stronger sequence homology to one another, particularly in the ligand binding domain (LBD), than to the other members of the NHR super-family (Evans, 1988, supra) and they all utilize steroid based ligands. Some examples of this sub-family of NHR's are the androgen receptor (AR), the estrogen receptor (ER), the progesterone receptor (PR), the glucocorticoid receptor (GR), the mineralocorticoid receptor (MR), the aldosterone receptor (ALDR) and the steroid and xenobiotic receptor (SXR) (Evans et al., WO 99/35246). Based on the strong sequence homology in the LBD, several orphan receptors may also be members of the SB-NHR sub-family.

Consistent with the high sequence homology found in the LBD for each of the SB-NHR's, the natural ligands for each are derived from a common steroid core. Examples of some of the steroid based ligands utilized by members of the SB-NHR's include cortisol, aldosterone, estrogen, progesterone, testosterone and dihydrotestosterone. Specificity of a particular steroid based ligand for one SB-NHR versus another is obtained by differential substitution about the steroid core. High affinity binding to a particular SB-NHR, coupled with high level specificity for that particular SB-NHR, can be achieved with only minor structural changes about the steroid core (e.g., Waller et al., Toxicol. Appl. Pharmacol. 137, 219-227 (1996) and Mekenyan et al., Environ. Sci. Technol. 31, 3702-3711 (1997), binding affinity for progesterone towards the androgen receptor as compared to testosterone).

Numerous synthetically derived steroidal and non-steroidal agonists and antagonists have been described for the members of the SB-NHR family. Many of these agonist and antagonist ligands are used clinically in man to treat a variety of medical conditions. RU486 is an example of a synthetic agonist of the PR, which is utilized as a birth control agent (Vegeto et al., Cell 69: 703-713 (1992)), and Flutamide is an example of an antagonist of the AR, which is utilized for the treatment of prostate cancer (Neri et al., Endo. 91, 427-437 (1972)). Tamoxifen is an example of a tissues specific modulator of the ER function that is used in the treatment of breast cancer (Smigel, J. Natl. Cancer Inst. 90, 647-648 (1998)). Tamoxifen can function as an antagonist of the ER in breast tissue while acting as an agonist of the ER in bone (Grese et al., Proc. Natl. Acad. Sci. USA 94,14105-14110 (1997)). Because of the tissue selective effects seen for Tamoxifen, this agent and agents like it are referred to as "partial-agonist" or "partial-antagonist". In addition to synthetically derived non-endogenous ligands, non-endogenous ligands for NHR's can be obtained from food sources (Regal et al., Proc. Soc. Exp. Biol. Med. 223, 372-378 (2000) and Hempstock et al., J. Med. Food 2, 267-269 (1999)). The flavanoid phytoestrogens are an example of an unnatural ligand for SB-NHR's that are readily obtained from a food source such as soy (Quella et al., J. Clin. Oncol. 18, 1068-1074 (2000) and Banz et al., J. Med. Food 2, 271-273 (1999)). The ability to modulate the transcriptional activity of individual NHR is by the addition of a small molecule ligand, makes them ideal targets for the development of pharmaceutical agents for a variety of disease states.

As mentioned above, non-natural ligands can be synthetically engineered to serve as modulators of the function

of NHR's. In the case of SB-NHR's, engineering of an unnatural ligand can include the identification of a core structure which mimics the natural steroid core system. This can be achieved by random screening against several SB-NHR's or through directed approaches using the available crystal structures of a variety of NHR ligand binding domains (Bourguet et al., Nature 375, 377-382 (1995), Brzozowski, et al., Nature 389, 753-758 (1997), Shiau et al., Cell 95, 927-937 (1998) and Tanenbaum et al., Proc. Natl. Acad. Sci. USA 95, 5998-6003 (1998)). Differential substitution about such a steroid mimic core can provide agents with selectivity for one receptor versus another. In addition, such modifications can be employed to obtain agents with agonist or antagonist activity for a particular SB-NHR. Differential substitution about the steroid mimic core can result in the formation of a series of high affinity agonists and antagonists with specificity for, for example, ER versus PR versus AR versus GR versus MR. Such an approach of differential substitution has been reported, for example, for quinoline based modulators of steroid NHR in J. Med. Chem., 41, 623 (1999); WO 9749709; US 5696133; US 5696130; US 5696127; US 5693647; US 5693646; US 5688810; US 5688808 and WO 9619458, all incorporated herein by reference.

Ch. Grogan describes bicyclic imides and isoindolines in the J. Med. Chem., Vol. 6, No. 6, 1963, pages 802-805. The compounds of the present invention comprise a core which serves as a steroid mimic, and are useful as modulators of the function of steroid binding nuclear hormone receptors, as well as other NHR is as described following.

## Summary of the Invention

The present invention provides fused cyclic compounds which are especially useful as modulators of nuclear hormone receptor function.

The present invention relates to a compound having the formula:

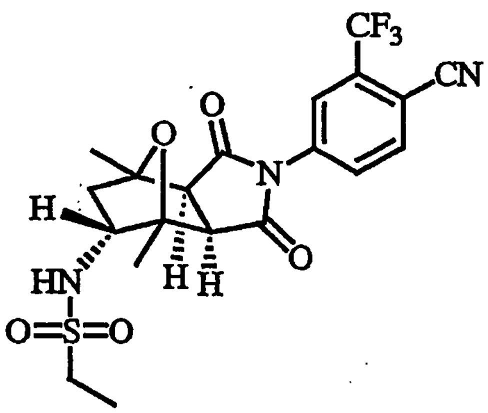

In a further aspect the present invention relates to a compound having the formula:

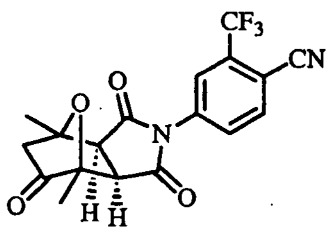

Pharmaceutical compositions comprising at least one of the above mentioned compounds or a pharmaceutically acceptable salt, or solvate thereof and a pharmaceutically acceptable carrier are also disclosed. The pharmaceutical composition can optionally further comprise another anti-cancer agent.

The above mentioned compounds, a pharmaceutically acceptable salt, or solvate thereof are particularly useful for treating a condition or disorder selected from the group consisting of proliferate diseases, cancers, benign prostate hypertrophia, adenomas and neoplasies of the prostate, benign or malignant tumor cells containing the androgen receptor, heart disease, angiogenic conditions or disorders, hirsutism, acne, hyperpilosity, inflammation, immune modulation, seborrhea, endometriosis, polycystic ovary syndrome, androgenic alopecia, hypogonadism, osteoporosis, suppressing spermatogenisis, libido, cachexia, anorexia, inhibition of muscular atrophy in ambulatory patients, androgen supplementation for age related decreased testosterone levels in men, cancers expressing the estrogen receptor, prostate cancer, breast cancer, endometrial cancer, hot flushes, vaginal dryness, menopause, amennoreahea, dysmennoreahea, contraception, pregnancy termination, cancers containing the progesterone receptor, cyclesynchrony, meniginoma, fibroids, labor induction, autoimmune diseases, Alzheimer's disease, psychotic disorders, drug dependence, non-insulin dependent Diabetes Mellitus, dopamine receptor mediated disorders, congestive heart failure, disregulation of cholesterol homeostasis, and attenuating the metabolism of a pharmaceutical agent, preferably prostate cancer.

Analogs of the presently claimed compounds have the formula I:

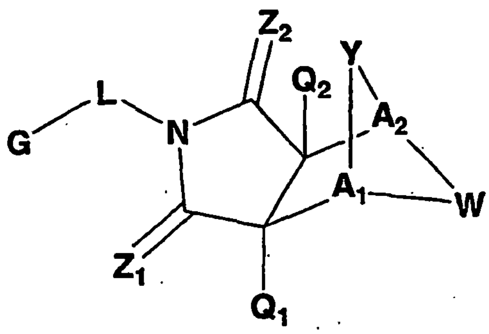

(I)

These compounds of the formula (I), with the exception of the two claimed compounds, are not subject matter of the present invention.

As used in formula I, and throughout the specification, the symbols have the following meanings unless otherwise indicated, and are, for each occurrence, independently selected:

G is an aryl or heterocyclo (e.g., heteroaryl) group, where said group is mono- or polycyclic, and which is optionally substituted at one or more positions, preferably with hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC=O$,$R^1C=O$, $R^1C=S$, $R^1HNC=O$, $R^1R^2NC=O$, $HOCR^3R^{3'}$, nitro, $R^1OCH_2$, $R^1O$, $NH_2$, $NR^4R^5$, $SR^1$, $S=OR^1$, $SO_2R^1$, $SO_2OR^1$, $SO_2NR^1R^{1'}$, $(R^1O)(R^{1'}O)P=O$, oxo, $(R^1)(R^{1'})P=O$, or $(R^{1'})(NHR^1)P=O$;

$Z_1$ is O, S, NH, or $NR^6$;

$Z_2$ iS O, S, NH, or $NR^6$;

$A_1$ is $CR^7$ or N;

$A_2$ is $CR^7$ or N; .

Y is J-J'-J" where J is $(CR^7R^{7'})n$ and n = 0-3, J' is a bond or O, S, S=O, $SO_2$, NH, $NR^7$, C=O, OC=O, $NR^1C=O$, $CR^7R^{7'}$, $C=CR^8R^{8'}$, $R^2P=O$, $R^2P=S$, $R^2OP=O$, $R^2NHP=O$, $OP=OOR^2$, $OP=ONHR^2$, $OP=OR^2$, $OSO_2$, $C=NR^7$, NHNH, $NHNR^6$, $NR^6NH$, N=N, cycloalkyl or substituted cycloalkyl, cycloalkenyl or substituted cycloalkenyl, heterocyclo or substituted heterocyclo or aryl or substituted aryl, and J" is $(CR^7R^{7'})n$ and n = 0-3, where Y is not a bond (i.e., if J' is a bond, then in at least one of J or J" (each defined as $(CR^7R^{7'})n$), n is not zero);

W is $CR^7R^{7'}$-$CR^7R^{7'}$, $CR^8=CR^{8'}$, $CR^7R^{7'}$-C=O, C=O-C=O, $CR^7R^{7'}$-$C=CH_2$, $C=CH_2$-$C=CH_2$, $CR^7R^{7'}$-$C=NR^1$, $C=NR^1$-$C=NR^1$, $NR^9$-$CR^7R^{7'}$, $N=CR^8$, N=N, $NR^9$-$NR^{9'}$, S-$CR^7R^{7'}$, SO-$CR^7R^{7'}$, $SO_2$-$CR^7R^{7'}$, cycloalkyl or substituted cycloalkyl, cycloalkenyl or substituted cycloalkenyl, heterocyclo or substituted heterocyclo, or aryl or substituted aryl, wherein when W is not $NR^9$-$CR^7R^{7'}$, $N=CR^8$, N=N, $NR^9$-$NR^{9'}$, S-$CR^7R^{7'}$, SO-$CR^7R^{7'}$, $SO_2$-$CR^7R^{7'}$, or heterocyclo or substituted heterocyclo, then J' must be O, S, S=O, $SO_2$, NH, $NR^7$, OC=O, $NR^1C=O$, $OP=OOR^2$, $OP=ONHR^2$, $OSO_2$, NHNH, $NHNR^6$, $NR^6NH$, or N=N; or

when W is $CR^7R^{7'}$-$CR^7R^{7'}$, the $R^7$ and $R^{7'}$ substituents in each occurrence may be taken together to form a substituted or unsubstituted carbocyclic or substituted or unsubstituted heterocyclic ring system which can be formed by any combination of $R^7$ and $R^{7'}$ attached to the same carbon atom;

$Q_1$ is H, alkyl or substituted alkyl, alkenyl or substituted alkenyl, cycloalkyl or substituted cycloalkyl, cycloalkenyl or substituted cycloalkenyl, heterocycloalkyl or substituted heterocycloalkyl, arylalkyl or substituted arylalkyl, alkynyl or substituted alkynyl, aryl or substituted aryl, heterocyclo (e.g., heteroaryl) or substituted

heterocyclo (e.g., substituted heteroaryl), halo, CN, $R^1OC{=}O$, $R^4C{=}O$, $R^5R^6NC{=}O$, $HOCR^7R^{7'}$, nitro, $R^1OCH_2$, $R^1O$, $NH_2$, $C{=}OSR^1$, $SO_2R^1$ or $NR^4R^5$;

$Q_2$ is H, alkyl or substituted alkyl, alkenyl or substituted alkenyl, cycloalkyl or substituted cycloalkyl, cycloalkenyl or substituted cycloalkenyl, heterocycloalkyl or substituted heterocycloalkyl, arylalkyl or substituted arylalkyl, alkynyl or substituted alkynyl, aryl or substituted aryl, heterocyclo (e.g., heteroaryl) or substituted heterocyclo (e.g., substituted heteroaryl), halo, CN, $R^1OC{=}O$, $R^4C{=}O$, $R^5R^6NC{=}O$, $HOCR^7R^{7'}$, nitro, $R^1OCH_2$, $R^1O$, $NH_2$, $C{=}OSR^1$, $SO_2R^1$ or $NR^4R^5$;

L is a bond, $(CR^7R^{7'})n$, NH, $NR^5$, NH $(CR^7R^{7'})n$, or $NR^5(CR^7R^{7'})n$, where n = 0-3;

$R^1$ and $R^{1'}$ are each independently H, alkyl or substituted alkyl, alkenyl or substituted alkenyl, alkynyl or substituted alkynyl, cycloalkyl or substituted cycloalkyl, cycloalkenyl or substituted cycloalkenyl, heterocyclo or substituted heterocyclo, cycloalkylalkyl, or substituted cycloalkyalkyl, cycloalkenylalkyl or substituted cycloalkenylalkyl, heterocycloalkyl or substituted heterocycloalkyl, aryl or substituted aryl, arylalkyl or substituted arylalkyl;

$R^2$ is alkyl or substituted alkyl, alkenyl or substituted alkenyl, alkynyl or substituted alkynyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl;

$R^3$ and $R^{3'}$ are each independently H, alkyl or substituted alkyl, alkenyl or substituted alkenyl, alkynyl or substituted alkynyl, cycloalkyl or substituted cycloalkyl, cycloalkenyl, or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, halo, CN, hydroxylamine, hydroxamide, alkoxy or substituted alkoxy, amino, $NR^1R^2$, thiol, alkylthio or substituted alkylthio;

$R^4$ is H, alkyl or substituted alkyl, alkenyl or substituted alkenyl, alkynyl or substituted alkynyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, $R^1C{=}O$, $R^1OC{=}O$, $R^1NHC{=}O$, $SO_2OR^1$, $SO_2R^1$ or $SO_2NR^1R^{1'}$;

$R^5$ is alkyl or substituted alkyl, alkenyl or substituted alkenyl, alkynyl or substituted alkynyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, $R^1C{=}O$, $R^1NHC{=}O$, $SO_2R^1$, $SO_2OR^1$, or $SO_2NR^1R^{1'}$;

$R^6$ is alkyl or substituted alkyl, alkenyl or substituted alkenyl, alkynyl or substituted alkynyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, CN, OH, $OR^1$, $R^1C{=}O$, $R^1NHC{=}O$, $SO_2R^1$, $SO_2OR^1$, or $SO_2NR^1R^{1'}$;

$R^7$ and $R^{7'}$ are each independently H, alkyl or substituted alkyl, alkenyl or substituted alkenyl, alkenyl or substituted alkenyl, alkynyl or substituted alkynyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, halo, CN, $OR^4$, nitro, hydroxylamine, hydroxylamide, amino, $NHR^4$, $NR^2R^5$, $NR^5R^5$, $NOR^1$, thiol, alkylthio or substituted alkylthio, $HOC{=}O$, $R^1C{=}O$, $R^1(C{=}O)O$, $R^1OC{=}O$, $R^1NHC{=}O$, $NH_2C{=}O$, $SO_2R^1$, $SOR^1$, $PO_3R^1R^{1'}$, $R^1R^{1'}NC{=}O$, $C{=}OSR^1$, $SO_2R^1$, $SO_2OR^1$, or $SO_2NR^1R^{1'}$, or, wherein $A_1$ or $A_2$ contains a group $R^7$ and W contains a group $R^7$, said $R^7$ groups of $A_1$ or $A_2$ and W together form a heterocyclic ring;

$R^8$ and $R^{8'}$ are each independently H, alkyl or substituted alkyl, alkenyl or substituted alkenyl, alkynyl or substituted alkynyl, cycloalkyl or substituted cycloalkyl, cycloalkenyl or substituted cycloalkenyl, heterocyclo or substituted heterocyclo, cycloalkylalkyl or substituted cycloalkyalkyl, cycloalkenylalkyl or substituted cycloalkenylalkyl, heterocycloalkyl or substituted heterocycloalkyl, aryl or substituted aryl, arylalkyl or substituted arylalkyl, nitro, halo, CN, $OR^1$, amino, $NHR^4$, $NR^2R^5$, $NOR^1$, alkylthio or substituted alkylthio, $C{=}OSR^1$, $R^1OC{=}O$, $R^1C{=}O$, $R^1NHC{=}O$, $R^1R^{1'}NC{=}O$, $SO_2OR^1$, $S{=}OR^1$, $SO_2R^1$, $PO_3R^1R^{1'}$, or $SO_2NR^1R^{1'}$; and

$R^9$ and $R^{9'}$ are each independently H, alkyl or substituted alkyl, alkenyl or substituted alkenyl, alkynyl or substituted alkynyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or sub-

stituted arylalkyl, CN, OH, $OR^1$, $R^1C{=}O$, $R^1OC{=}O$, $R^1NHC{=}O$, $SO_2R^1$, $SO_2OR^1$, or $SO_2NR^1R^{1'}$.

Preferably, the claimed compounds are monomeric, and are not comprised within other oligomers or polymers.

The claimed compounds and salts thereof comprise a core which can serve as a steroid mimic (and do not require the presence of a steroid-type (e.g., cyclopentanoperhydrophenanthrene analog) structure).

The following are definitions of terms used in the present specification. The initial definition provided for a group or term herein applies to that group or term throughout the present specification individually or as part of another group, unless otherwise indicated.

The terms "alkyl" and "alk" refers to a straight or branched chain alkane (hydrocarbon) radical containing from 1 to 12 carbon atoms, preferably 1 to 6 carbon atoms. Exemplary such groups include, but are not limited to, methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, and the like. "Substituted alkyl" refers to an alkyl group substituted with one or more substituents, preferably 1 to 4 substituents, at any available point of attachment. Exemplary substituents include but are not limited to one or more of the following groups: halo (e.g., a single halo substituent or multiple halo substitutents forming, in the latter case, groups such as a perfluoroalkyl group or an alkyl group bearing $Cl_3$ or $CF_3$), alkoxy, alkylthio, hydroxy, carboxy (i.e., -COOH), alkoxycarbonyl, alkylcarbonyloxy, amino (i.e., $-NH_2$), carbamoyl or substituted carbomoyl, carbamate or substituted carbamate, urea or substituted urea, amidinyl or substituted amidinyl, thiol (i.e., -SH), aryl, heterocycle, cycloalkyl, heterocycloalkyl, -S-aryl, -S-heterocycle, -S=O-aryl, -S=O-heterocycle, arylalkyl-O-, $-S(O)_2$-aryl, $-S(O)_2$-heterocycle, $-NHS(O)_2$-aryl, $-NHS(O)_2$-heterocycle, $-NHS(O)_2NH$-aryl, $-NHS(O)_2NH$-heterocycle $-P(O)_2$-aryl, $-P(O)_2$-heterocycle, $-NHP(O)_2$-aryl, $-NHP(O)_2$-heterocycle, $-NHP(O)_2NH$-aryl, $-NHP(O)_2NH$-heterocycle, -O-aryl, -O-heterocycle, -NH-aryl, -NH-heterocycle, -NHC=O-aryl, -NHC=O-alkyl, -NHC=O-heterocycle, -OC=O-aryl, -OC=O-heterocycle, -NHC=ONH-aryl, -NHC=ONH-heterocycle, -OC=OO-aryl, -OC=OO-heterocycle, -OC=ONH-aryl, -OC=ONH-heterocycle, -NHC=OO-aryl, -NHC=OO-heterocycle, -NHC=OO-alkyl, -C=ONH-aryl, -C=ONH-heterocycle, -C=OO-aryl, -C=OO-heterocycle, $-N(alkyl)S(O)_2$-aryl, $-N(alkyl)S(O)_2$-heterocycle, $-N(alkyl)S(O)_2NH$-aryl, $-N(alkyl)S(O)_2NH$-heterocycle, $-N(alkyl)P(O)_2$-aryl, $-N(alkyl)P(O)_2$-heterocycle, $-N(alkyl)P(O)_2NH$-aryl, $-N(alkyl)P(O)_2NH$-heterocycle, -N(alkyl)-aryl, -N(alkyl)-heterocycle, -N(alkyl)C=O-aryl, -N(alkyl)C=O-heterocycle, -N(alkyl)C=ONH-aryl, -N(alkyl)C=ONH-heterocycle, -OC=ON(alkyl)-aryl, -OC=ON(alkyl)-heterocycle, -N(alkyl)C=OO-aryl, -N(alkyl)C=OO-heterocycle, -C=ON(alkyl)-aryl, -C=ON(alkyl)-heterocycle, $-NHS(O)_2N(alkyl)$-aryl, $-NHS(O)_2N(alkyl)$-heterocycle, $-NHP(O)_2N(alkyl)$-aryl, $NHP(O)_2N(alkyl)$-heterocycle, -NHC=ON(alkyl)-aryl, -NHC=ON(alkyl)-heterocycle, $-N(alkyl)S(O)_2N(alkyl)$-aryl, $-N(alkyl)S(O)_2N(alkyl)$-heterocycle, $-N(alkyl)P(O)_2N(alkyl)$-aryl, $-N(alkyl)P(O)_2N(alkyl)$-heterocycle, -N(alkyl)C=ON(alkyl)-aryl, and -N(alkyl)C=ON(alkyl)-heterocycle. In the aforementioned exemplary substitutents, in each instance, groups such as "alkyl", "aryl" and "heterocycle" can themselves be optionally substituted; for example, "alkyl" in the group "NCH=OO-alkyl" recited above can be optionally substituted so that both "NHC=OO-alkyl" and "NHC=OO-substituted alkyl" are exemplary substitutents. Exemplary alkyl substituents also include groups such as "T" and "T-$R^{12}$" (which are defined below), especially for substituted alkyl, groups within $A_1$ or $A_2$.

The term "alkenyl" refers to a straight or branched chain hydrocarbon radical containing from 2 to 12 carbon atoms and at least one carbon-carbon double bond. Exemplary such groups include ethenyl or allyl. "Substituted alkenyl" refers to an alkenyl group substituted with one or more substituents, preferably 1 to 4 substituents, at any available point of attachment. Exemplary substituents include, but are not limited to, alkyl or substituted alkyl, as well as those groups recited above as exemplary alkyl substituents.

The term "alkynyl" refers to a straight or branched chain hydrocarbon radical containing from 2 to 12 carbon atoms and at least one carbon to carbon triple bond. Exemplary such groups include ethynyl. "Substituted alkynyl" refers to an alkynyl group substituted with one or more substituents, preferably 1 to 4 substituents, at any available point of attachment. Exemplary substituents include, but are not limited to, alkyl or substituted alkyl, as well as those groups recited above as exemplary alkyl substituents.

The term "cycloalkyl" refers to a fully saturated cyclic hydrocarbon group containing from 1 to 4 rings and 3 to 8 carbons per ring. Exemplary such groups include cyclopropyl, cyclobutyl, cyclopentyl, cyclohexyl, etc. "Substituted cycloalkyl" refers to a cycloalkyl group substituted with one or more substituents, preferably 1 to 4 substituents, at any available point of attachment. Exemplary substituents include, but are not limited to, nitro, cyano, alkyl or substituted alkyl, as well as those groups recited above as exemplary alkyl substituents, and as previously mentioned as preferred aryl substituents in the definition for G. Exemplary substituents also include spiro-attached or fused cyclic substituents, especially cycloalkenyl or substituted cycloalkenyl.

The term "cycloalkenyl" refers to a partially unsaturated cyclic hydrocarbon group containing 1 to 4 rings and 3 to 8 carbons per ring. Exemplary such groups include cyclobutenyl, cyclopentenyl, cyclohexenyl, etc. "Substituted cycloalkenyl" refers to a cycloalkenyl group substituted with one more substituents, preferably 1 to 4 substituents, at any available point of attachment. Exemplary substituents include but are not limited to nitro, cyano, alkyl or substituted alkyl, as well as those groups recited above as exemplary alkyl substituents, and as previously mentioned as preferred aryl substituents in the definition for G. Exemplary substituents also include spiro-attached or fused cyclic substituents,

especially cycloalkyl or substituted cycloalkyl.

The terms "alkoxy" or "alkylthio" refer to an alkyl group as described above bonded through an oxygen linkage (-O-) or a sulfur linkage (-S-), respectively. The terms "substituted alkoxy" or "substituted alkylthio" refer to a substituted alkyl group as described above bonded through an oxygen or sulfur linkage, respectively.

The term "alkoxycarbonyl" refers to an alkoxy group bonded through a carbonyl group.

The term "alkylcarbonyl" refers to an alkyl group bonded through a carbonyl group. The term "alkylcarbonyloxy" refers to an alkylcarbonyl group bonded through an oxygen linkage.

The terms "arylalkyl", "substituted arylalkyl," "cycloalkylalkyl," "substituted cycloalkylalkyl," "cycloalkenylalkyl", "substituted cycloalkenylalkyl", "heterocycloalkyl" and "substituted heterocycloalkyl" refer to aryl, cycloalkyl, cycloalkenyl and heterocyclo groups bonded through an alkyl group, substituted on the aryl, cycloalkyl, cycloalkenyl or heterocyclo and/or the alkyl group where indicated as "substituted."

The term "aryl" refers to cyclic, aromatic hydrocarbon groups which have 1 to 5 aromatic rings, especially monocyclic or bicyclic groups such as phenyl, biphenyl or naphthyl. Where containing two or more aromatic rings (bicyclic, etc.), the aromatic rings of the aryl group may be joined at a single point (e.g., biphenyl), or fused (e.g., naphthyl, phenanthrenyl and the like). "Substituted aryl" refers to an aryl group substituted by one or more substituents, preferably 1,2,3,4 or 5 substituents, at any point of attachment. Exemplary substituents include, but are not limited to, nitro, cycloalkyl or substituted cycloalkyl, cycloalkenyl or substituted cycloalkenyl, cyano, alkyl-$S(O)_m$- (m=0, 1 or 2), alkyl or substituted alkyl, as well as those groups recited above as exemplary alkyl substituents and as previously mentioned as preferred aryl substituents in the definition for G. Exemplary substituents also include fused cyclic substituents, such as heterocyclo or cycloalkenyl, or substituted heterocyclo or cycloalkenyl, groups (e.g., thereby forming a fluoroenyl, tetrahydronapthalenyl, or dihydroindenyl group).

"Carbamoyl" refers to the group -CONH- which is bonded on one end to the remainder of the molecule and on the other to hydrogen or an organic moiety (such as alkyl, substituted alkyl, aryl, substituted aryl, heterocycle, alkylcarbonyl, hydroxyl and substituted nitrogen). "Carbamate" refers to the group -O-CO-NH- which is bonded on one end to the remainder of the molecule and on the other to hydrogen or an organic moiety (such as those listed above). "Urea" refers to the group -NH-CO-NH-which is bonded on one end to the remainder of the molecule and on the other to hydrogen or an organic moiety (such as those listed above). "Amidinyl" refers to the group $-C(=NH)(NH_2)$. "Substituted carbamoyl," "substituted carbamate," "substituted urea" and "substituted amidinyl" refer to carbamoyl, carbamate, urea or amidinyl groups as described above in which one more of the hydrogen groups are replaced by an organic moiety (such as those listed above).

The terms "heterocycle", heterocyclic" and "heterocyclo" refer to fully saturated, or partially or fully unsaturated, including aromatic (i.e., "heteroaryl") cyclic groups (for example, 3 to 7 membered monocyclic, 7 to 11 membered bicyclic, or 10 to 16 membered tricyclic ring systems) which have at least one heteroatom in at least one carbon atom-containing ring. Each ring of the heterocyclic group containing a heteroatom may have 1, 2, 3, or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. (The term "heteroarylium" refers to a heteroaryl group bearing a quaternary nitrogen atom and thus a positive charge.) The heterocyclic group may be attached to the remainder of the molecule at any heteroatom or carbon atom of the ring or ring system. It is understood that, where W or W' are cycloalkyl or substituted cycloalkyl, cycloalkenyl or substituted cycloalkenyl, heterocyclo or substituted heterocyclo, or aryl or substituted aryl, that A1 and A2 can be separately bonded to different (such as adjacent) ring atoms on said groups. Exemplary monocyclic heterocyclic groups include ethylene oxide, azetidinyl, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, hexahydrodiazepinyl, 4-piperidonyl, pyridyl, pyrazinyl, pyrimidinyl, pyridazinyl, triazinyl, triazolyl, tetrazolyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, and the like. Exemplary bicyclic heterocyclic groups include indolyl, isoindolyl, benzothiazolyl, benzodioxolyl, benzoxazolyl, benzoxadiazolyl, benzothienyl, quinuclidinyl, quinolinyl, tetrahydroisoquinolinyl, isoquinolinyl, benzimidazolyl, benzopyranyl, indolizinyl, benzofuryl, benzofurazanyl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]pyridinyl] or furo[2,3-b]pyridinyl), dihydrobenzodioxinyl, dihydrodioxidobenzothiophenyl, dihydroisoindolyl, dihydroindolyl, dihydroquinolinyl, dihydroquinazolinyl (such as 3,4-dihydro-4-oxo-quinazolinyl), triazinylazepinyl, tetrahydroquinolinyl and the like. Exemplary tricyclic heterocyclic groups include carbazolyl, benzidolyl, phenanthrolinyl, dibenzofuranyl, acridinyl, phenanthridinyl, xanthenyl and the like.

"Substituted heterocycle," "substituted heterocyclic," and "substituted heterocyclo" (such as "substituted heteroaryl") refer to heterocycle, heterocyclic or heterocyclo groups substituted with one or more substituents, preferably 1 to 4 substituents, at any available point of attachment. Exemplary substituents include, but are not limited to, cycloalkyl or substituted cycloalkyl, cycloalkenyl or substituted cycloalkenyl, nitro, oxo (i.e., = O), cyano, alkyl-$S(O)_m$- (m = 0, 1 or 2), alkyl or substituted alkyl, as well as those groups recited above as exemplary alkyl substituents, and as previously

mentioned as preferred heterocyclo substituents in the definition for G.

The term "quaternary nitrogen" refers to a tetravalent positively charged nitrogen atom including, for example, the positively charged nitrogen in a tetraalkylammonium group (e.g., tetramethylammonium, N-methylpyridinium), the positively charged nitrogen in protonated ammonium species (e.g., trimethyl-hydroammonium, N-hydropyridinium), the positively charged nitrogen in amine N-oxides (e.g., N-methyl-morpholine-N-oxide, pyridine-N-oxide), and the positively charged nitrogen in an N-amino-ammonium group (e.g., N-aminopyridinium).

The terms "halogen" or "halo" refer to chlorine, bromine, fluorine or iodine.

The terms "hydroxylamine" and "hydroxylamide" refer to the groups OH-NH- and OH-NH-CO-, respectively.

When a functional group is termed "protected", this means that the group is in modified form to mitigate, especially preclude, undesired side reactions at the protected site. Suitable protecting groups for the methods and compounds described herein include, without limitation, those described in standard textbooks, such as Greene, T. W. et al., Protective Groups in Organic Synthesis, Wiley, N.Y. (1991).

When a term such as "(CRR)n" is used, it denotes an optionally substituted alkyl chain existing between the two fragments to which it is bonded, the length of which chain is defined by the range described for the term n. An example of this is n=0-3, implying from zero to three (CRR) units existing between the two fragments, which are attached to the primary and terminal (CRR) units. In the situation where the term n is set to zero (n = 0) then a bond exists between the two fragments attached to (CRR).

Unless otherwise indicated, any heteroatom with unsatisfied valences is assumed to have hydrogen atoms sufficient to satisfy the valences.

Divalent groups, such as those in the definition of W (e.g., $NR^9$-$CR^7R^{7'}$), may be bonded in either direction to the remainder of the molecule (e.g,

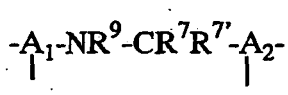

or,

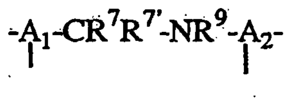

for the aforementioned group within the definition of W).

Carboxylate anion refers to a negatively charged group $-COO^-$.

The claimed compounds form salts which are also within the scope of this invention. Reference to a compound of the formula I herein is understood to include reference to salts thereof, unless otherwise indicated. The term "salt(s)", as employed herein, denotes acidic and/or basic salts formed with inorganic and/or organic acids and bases. In addition, when a compound of formula I contains both a basic moiety, such as but not limited to a pyridine or imidazole, and an acidic moiety such as but not limited to a carboxylic acid, zwitterions ("inner salts") may be formed and are included within the term "salt(s)" as used herein. Pharmaceutically acceptable (i.e., non-toxic, physiologically acceptable) salts are preferred, although other salts are also useful, e.g., in isolation or purification steps which may be employed during preparation. Salts of the compounds of the formula I may be formed, for example, by reacting the compound of the formula I with an amount of acid or base, such as an equivalent amount, in a medium such as one in which the salt precipitates or in an aqueous medium followed by lyophilization.

The compounds of formula I which contain a basic moiety, such as but not limited to an amine or a pyridine or imidazole ring, may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorated, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, hydroxyethanesulfonates (e.g., 2-hydroxyethanesulfonates), lactates, maleates, methanesulfonates, naphthalenesulfonates (e.g., 2-naphthalenesulfonates), nicotinates, nitrates, oxalates, pectinates, persulfates, phenylpropionates (e.g., 3-phenylpropionates), phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

The compounds of formula I which contain an acidic moiety, such but not limited to a carboxylic acid, may form

salts with a variety of organic and inorganic bases. Exemplary basic salts include ammonium salts, alkali metal salts such as sodium, lithium and potassium salts, alkaline earth metal salts such as calcium and magnesium salts, salts with organic bases (for example, organic amines) such as benzathines, dicyclohexylamines, hydrabamines (formed with N, N-bis(dehydroabietyl)ethylenediamine), N-methyl-D-glucamines, N-methyl-D-glycamides, t-butyl amines, and salts with amino acids such as arginine, lysine and the like. Basic nitrogen-containing groups may be quaternized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

Solvates of the compounds of the invention are also contemplated herein. Solvates of the compounds of formula I include, for example, hydrates.

Compounds of the formula I, and salts thereof, may exist in their tautomeric form (for example, as an amide or imino ether). All such tautomeric forms are contemplated herein as part of the present invention.

All stereoisomers of the present compounds (for example, those which may exist due to asymmetric carbons on various substituents), including enantiomeric forms and diastereomeric forms, are contemplated within the scope of this invention. Individual stereoisomers of the compounds of the invention may, for example, be substantially free of other isomers (e.g., as a pure or substantially pure optical isomer having a specified activity), or may be admixed, for example, as racemates or with all other, or other selected, stereoisomers. The chiral centers may have the $S^-$ or $R^-$ configuration as defined by the IUPAC 1974 Recommendations. The racemic forms can be resolved by physical methods, such as, for example, fractional crystallization, separation or crystallization of diastereomeric derivatives or separation by chiral column chromatography. The individual optical isomers can be obtained from the racemates by any suitable method, including without limitation conventional methods, such as, for example, salt formation with an optically active acid followed by crystallization.

All configurational isomers of the compounds of the present invention are contemplated, either in admixture or in pure or substantially pure form. The definition of compounds of the present invention embraces both cis (Z) and trans (E) alkene isomers, as well as cis and trans isomers of cyclic hydrocarbon or heterocyclo rings. In certain cases, for example, the exo or endo conformation can be preferred for the fused ring system bonded to G-L in formula I. For example, for androgen receptor antagonists (or selective androgen receptor modulators), where Y is O or $NR^7$, the exo configuration can be preferred, while for most other definitions of Y, the endo configuration can be preferred. As can be appreciated, the preferred configuration can be a function of the particular compound and its preferred activity. Separation of configurational isomers can be achieved by any suitable method, such as column chromatography.

Throughout the specifications, groups and substituents thereof may be chosen to provide stable moieties and compounds.

Embodiments indicated herein as exemplary or preferred are intended to be illustrative and not limiting.

## Methods of Preparation

The compounds of the present invention and analogs thereof may be prepared by methods such as those illustrated in the following Schemes I to XI. Solvents, temperatures, pressures, and other reaction conditions may readily be selected by one of ordinary skill in the art. Starting materials are commercially available or readily prepared by one of ordinary skill in the art. Combinatorial techniques may be employed in the preparation of compounds, for example, where the intermediates possess groups suitable for these techniques. See the following which describe other methods which may be employed in the preparation of compounds of the present invention and analogs thereof, Li, et al., Eur. J. Org. Chem. 9, 1841-1850 (1998); Li, Y-Q, Synlett. 5, 461-464 (1996); Thiemann, et al., Bull. Chem. Soc. Jpn. 67, 1886-1893 (1994); Tsuge et al., Heterocycles 14, 423-428 (1980); Ward et al., Can J. Chem. 75, 681-693 (1997); Ward et al., Can J. Chem. 69, 1487-1497 (1991); Ward et al., Tetrahedron Lett. 31, 845-848 (1990); Fleming et al., J. Org. Chem. 44, 2280-2282 (1979); Jankowski et al., J. Organomet. Chem. 595, 109-113 (2000); Keglevich et al., J. Organomet. Chem. 579, 182-189 (1999); Keglevich et al., J. Organomet. Chem. 570, 49-539 (1998); Jankowski et al., Hetroat. Chem. 7, 369-374 (1996); Jankowski et al., J. Am. Chem. Soc. 113, 7011-7017 (1991); Quin et al., Tetrahedron Lett. 31, 6473-6476 (1990); Quin et al., J. Org. Chem. 59, 120-129 (1994); Quin et al., J. Org. Chem. 58, 6212-6216 (1993); Quin et al., Phosphorous, Sulfur Silicon Relat. Elem. 63, 349-362 (1991); Quin et al., Hetroat. Chem. 2, 359-367 (1991); Hussong et al., Phosphorus Sulfur. 25, 201-212 (1985); Quin et al., J. Org. Chem. 51, 3341-3347 (1986); Myers et al., J. Am. Chem. Soc. 114, 5684-5692 (1992); Myers et al., J. Am. Chem. Soc. 113, 6682-6683 (1991); Shen et al., US Patent No. 5817679; Cordone et al., J. Am. Chem. Soc. 111, 5969-5970 (1989); Jung et al., J. Chem. Soc. Commun. 630-632 (1984); Lay et al., J. Am. Chem. Soc. 104, 7658-7659 (1982); Gonzalez et al., J. Am. Chem. Soc. 117, 3405-3421 (1995); Kreher et al., Chem. Ber. 125, 183-189 (1992); Simig et al., Synlett. 7, 425-426 (1990); Sha et al., J. Org. Chem. 55, 2446-2450 (1990); Drew et al., J. Chem. Soc., Perkin Trans. 17, 1277-1284 (1985); Kreher et al., Anorg. Chem., Org Chem. 31B, 599-604 (1976); Avalos et al., Tetrahedron Lett. 39, 9301-9304 (1998); Gousse et al., Macromolecules 31, 314-321 (1998); Mikhailyuchenko et al., Khim. Geterotsikl. Soedin. 6, 751-758 (1993); Lubowitz et al., US Patent

No. 4476184; Padwa et al., J. Org. Chem. 61, 3706-3714 (1996); Schlessinger et al., J. Org. Chem. 59, 3246-3247 (1994); Buchmeiser et al., WO Publication No. 9827423; Tanabe *et al.,* Japanese Patent Document JP 07144477; Mochizucki *et al.,* Japanese Patent Document JP 63170383; Hosoda *et al.,* Japanese Patent Document JP 62053963; Onaka *et al.,* Japanese Patent Document JP 62053964; Kato *et al.,* Japanese Patent Document JP 53086035; Kato *et al.,* Japanese Patent Document JP 51088631; Tottori *et al.,* Japanese Patent Document JP 49124225; Augustin *et al.,* German Patent Document DD101271; Title *et al.,* French Patent Document FR 2031538; Gousse et al., Polym. Int. 48, 723-731 (1999); Padwa et al., J. Org. Chem. 62, 4088-4096 (1997); Theurillat-Moritz et al., Tetrahedron: Asymmetry 7, 3163-3168 (1996); Mathews et al., J. Carbohydr. Chem. 14, 287-97 (1995); Srivastava et al., Natl. Acad. Sci. Lett. (India) 15, 41-44 (1992); Mayorga et al., Rev. Cubana Quim. 4, 1-6 (1988); Kondoli et al., J. Chem. Res., Synop. 3, 76 (1987); Primelles et al., Cent. Azucar 7-14 (1985); Solov'eva et al., Khim. Geterotsikl. Soedin. 5, 613-15 (1984); Liu et al., Yaoxue Xuebao 18, 752-759 (1983); Joshi et al., Indian J. Chem, Sect. B. 22B, 131-135 (1983); Amos et al., WO Publication No. 9829495; Odagiri et al., US Patent No. 4670536; Gallucci *et al.,* European Patent Document EP 355435; Redmore, D. US Patent No. 3821232; Nakano et al., Heterocycles 35, 37-40 (1993); Tomisawa et al., Chem. Pharm. Bull. 36, 1692-1697 (1988); Krow et al., J. Heterocycl. Chem. 22, 131-135 (1985); Krow et al., J. Org. Chem. 47, 1989-1993 (1982); Liu et al., Yaoxue Xuebao 18, 752-759 (1983); Nishikawa *et al., Yaoxue Xuebao* JP 01061457; and/or Rice et al., J. Med. Chem. 11, 183-185 (1968).

All documents cited in the present specification, such as those cited in this "Methods of Preparation" as well as other sections herein, are incorporated herein by reference in their entirety. Reference to any document herein is not to be construed as an admission that such document is prior art.

## Scheme I

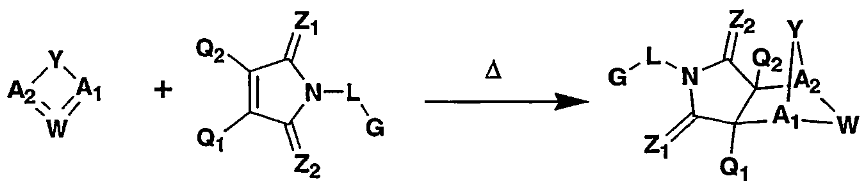


**II** **III** **IV**

As illustrated in Scheme **I,** a diene of formula **II** can be reacted with a dienophile of formula **III,** under conditions readily selected by one skilled in the art (such as by the addition of heat ("Δ")), to obtain a compound of formula **IV,** which is a compound of formula **I**. An intermediate diene of formula **II** can be obtained from commercial sources or readily made by one skilled in the art, for example, in accordance with the following literature documents and the references found therein: Hofman et al., J. Agric. Food Chem. 45, 898-906 (1997); Baciocchi et al., J. Chem. Soc., Perkin Trans. 2 8, 821-824 (1975); Wu et al., J. Heterocycles 38, 1507-1518 (1994); Yin et al., Tetrahedron Lett. 38, 5953-5954 (1997); Mic'ovic' et al., Tetrahedron 20, 2279-2287 (1964); Gorbunova et al., J. Org. Chem.. 35, 1557-1566 (1999); Rassu et al., Chem. Soc. Rev. 29, 109-118 (2000); Kaberdin et al., Russ. Chem. Rev.. 68, 765-779 (1999); Barluenga et al., Aldrichimica Acta 32, 4-15 (1999); Bogdanowicz-Szwed et al., Pol. Wiad. Chem. 52, 821-842 (1998); Casiraghi et al., Adv. Asymmetric Synth. 3, 113-189 (1998); and/or Baeckvall et al., Chem. Rev. 98, 2291-2312 (1998). An intermediate dieneophile of formula **III** can be obtained from commercial sources or readily made by one skilled in the art, for example, in accordance with the following literature references and the references found therein: Deshpande et al., Heterocycles 51, 2159-2162 (1999); Seijas et al., J. Chem. Res., Synop. 7, 420-421 (1999); Langer et al., Eur. J. Org. Chem. 7, 1467-1470 (1998); Kita *et al.,* Japanese Patent Document JP 09194458; Lopez-Alvarado et al., J. Org. Chem. 61, 5865-5870 (1996); Condon et al., US Patent No. 5523277; Sasakihara *et al.,* Japanese Patent Document JP 04290868; Igarashi *et al.,* Japanese Patent Document JP 04149173; Aoyama *et al.,* Japanese Patent Document JP 04134063; Aoyama *et al.,* Japanese Patent Document JP 04134062; Pastor et al., J. Org. Chem. 53, 5776-5779 (1988); and/or Takahashi et al., Chem. Lett. 6, 1229-1232 (1987).

## Scheme II

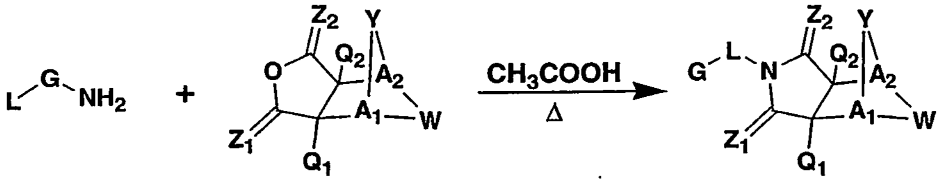


V VI IV

As illustrated in Scheme **II,** compounds of formula **I** can be obtained by reaction of a primary amine of formula **V** with a substituted anhydride-like intermediate of formula **VI,** for example, in a solvent such as acetic acid with or without heating, to yield a compound of formula **IV,** which is a compound of formula **I.** Primary amines of formula **V** can be obtained from commercial sources or readily synthesized by one skilled in the art. Anhydride-like agents of formula **VI** can be obtained from commercial sources or readily synthesized by one skilled in the art. The documents listed following describe exemplary approaches for the synthesis of intermediates of formula **VI** as well as synthetic approaches which can be applied to the synthesis of compounds of formula **IV** (all incorporated herein by reference in their entirety): Kohler, E. P.; Tishler, M.; Potter, H.; Thompson, H. T. J. Am. Chem. Soc. 1939, 1057-1061; Yur'ev, Y. K.; Zefirov, N. S. J. Gen. Chem. U.S.S.R. (Engl. Transl.) 1961, 31, 772-5; Norman G. Gaylord US Patent No. 3,995,099; Schueler, P. E.; Rhodes, Y. E. J. Org. Chem. 1974,39,2063-9; Ishitobi, H.; Tanida, H; Tsuji, T. Bull. Client. Soc. Japan 1971, 44, 2993-3000; Stájer, G.; Virág, M.; Szabó, A. E.; Bernáth, G.; Sohár, P.; Sillanpää, R. Acta. Chem. Scand. 1996, 50, 922-30; Hart, H.; Ghosh, T. Tetrahedron Lett. 1988,29,881-884; Kato, M.; Yamamoto, S.; Yoshihara, T.; Furuichi, K; Miwa, T. Chem. Lett. 1987, 1823-1826; Kottwitz, J.; Vorbrüggen, H. Synthesis 1995, 636-637; Creary, X. J. Org. Chem. 1975, 40, 3326-3331; Alder, K.; Ache, H.-J.; Flock, F. H. Chem. Ber. 1960, 93, 1888-1895; Toder, B. H.; Branca, S. J.; Dieter, R. K.; Smith, A. B. III Synth. Commun. 1975, 5, 435-439; Sprague, P. W.; Heikes, J. E.; Gougoutas, J. Z.; Malley, M. F.; Harris, D. N.; and/or Greenberg, R. J. Med. Chem. 1985, 28, 1580-1590.

The aforementioned approach(es) can be applied in a combinatorial fashion, for example, by utilizing a multi-well reaction block such as is described in Waldemar Ruediger, Wen-Jeng Li, John W., Allen Jr., and Harold N. Weller III, US Patent No. 5,961,925, Apparatus for Synthesis of Multiple Organic Compounds With Pinch Valve Block (incorporated herein by reference in its entirety). By utilizing the above-mentioned multi-well reaction block, one can, for example, perform multiples of 96 reactions at a time. Solvent can then be removed from the reaction tubes without removal from the reaction block and the crude products can be precipitated using a base such as sodium bicarbonate. The precipitates can be collected by filtration of the reaction block and then the desired products can be transferred directly to 96 well plates for screening. In this fashion, a large array of compounds of formula **I** can be synthesized, and tests conducted as desired by an automated approach.

## Scheme III

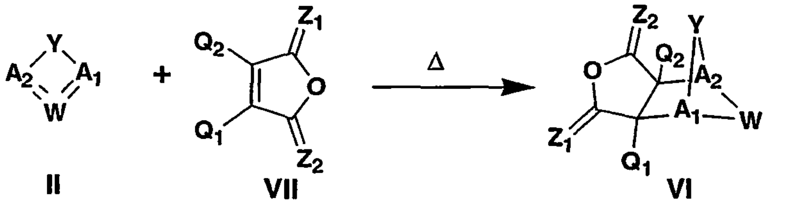


II VII VI

Scheme **III** describes a method for preparing an intermediate compound of formula **VI** which can be used to synthesize a compound of formula **I,** as described in Scheme **II.** As described in Scheme **III,** a diene of formula **II** can be reacted with a dieneophile of formula **VII** to yield the intermediate of formula **VI.** The methods applied to obtain such a transformation are analogous to those described in Scheme **I.**

## Scheme IV

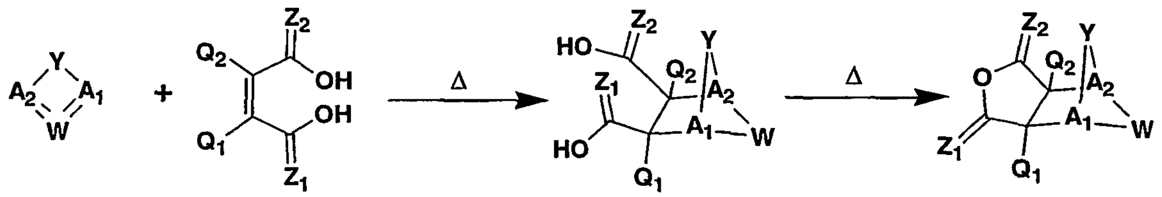


II VIII IX VI

Scheme **IV** describes a method for preparing an intermediate compound of formula **VI** which can be used to synthesize a compound of formula **I,** as described in Scheme **II.** As shown in Scheme **IV,** a diene of formula **II** can be reacted with a dieneophile of formula **VIII** to yield the intermediate of formula **IX.** The intermediate of formula **IX** can be dehydrated to an anhydride-like intermediate of formula **VI.** Dehydration of the bis-acid intermediate of formula **IX** to can be achieved by a variety of methods, such as those known to one skilled in the art and described in the following documents and the references embodied therein: Sprague et al., J. Med. Chem. 28, 1580-1590 (1985); and/or Retemi et al., J. Org. Chem. 61, 6296-6301 (1996).

Schemes **I** to **IV** describe general methods for the synthesis of compounds of formula **I,** and intermediates thereof, in which substitution about the ring system is incorporated directly, for example, at the level of the intermediate diene, dienophile, anhydride-like intermediate and amine groups. In addition to these approaches, additional substitution can be incorporated onto an already-prepared compound of formula **I** by a variety of approaches to prepare other compounds of the formula **I.** Exemplary methods for further substitution are described in Schemes **V** to **XI.**

## Scheme V

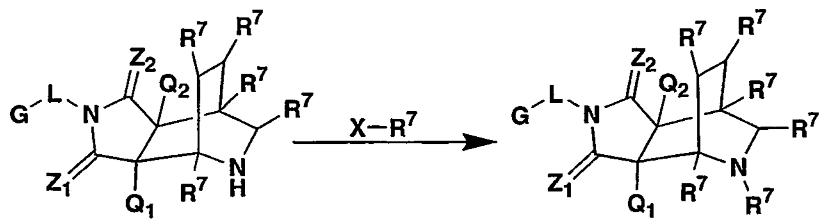


X XI

Scheme **V** describes one such approach to incorporating additional substitution into a structure of formula **I.** As illustrated in Scheme **V,** a compound of formula **X,** which is a compound of formula **I** where $A_1$ and $A_2$ are $CR^7$, W is NH-$CHR^7$ and Y is $CHR^7$-$CHR^7$, can be functionalized at the free amine of the group W by reaction with any of a variety of electrophilic agents such as acid halides or alkyl halides in the presence of base, for example, by methods known by one skilled in the art. In Scheme **V,** X is a leaving group, and a compound of formula **XI** is a compound of formula **I** where $A_1$ and $A_2$ are $CR^7$, W is $NR^7$-$CHR^7$ and Y is $CHR^7$-$CHR^7$.

## Scheme VI

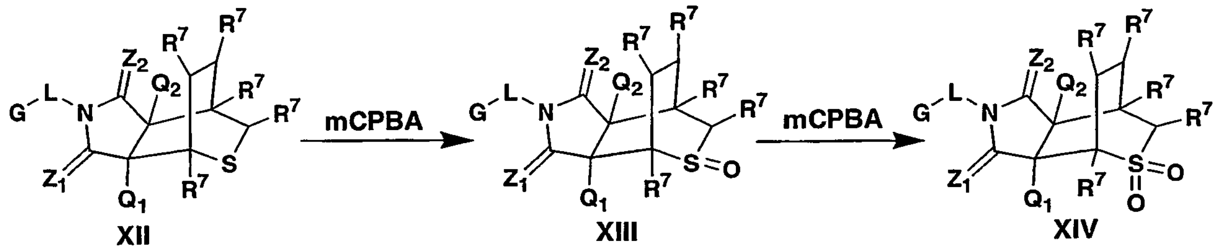


XII XIII XIV

Scheme **VI** describes an additional approach for further incorporating substitution onto a compound of formula **I.** As illustrated in Scheme **VI,** a compound of formula **XII,** which is a compound of formula **I** where $A_1$ and $A_2$ are $CR^7$, W is S-$CHR^7$ and Y is $CHR^7$-$CHR^7$, can be partially oxidized with an oxidizing agent such as mCPBA or other agents such as those known to one skilled in the art, to give the sulfoxide analog of formula **XIII,** which is a compound of formula **I** where $A_1$ and $A_2$ are $CR^7$, W is SO-$CHR^7$ and Y is $CHR^7$-$CHR^7$. Further treatment of a compound of formula **XIII** with an oxidizing agent such as mCPBA or other agents such as those known to one skilled in the art, can yield the sulphone analog of formula **XIV,** which is a compound of formula **I** where $A_1$ and $A_2$ are $CR^7$, W is $SO_2$-$CHR^7$ and Y is $CHR^7$-$CHR^7$. Alternatively, a compound of formula **XII** can be converted directly to a compound of formula **XIV** by prolonged treatment with an oxidizing agent, such as mCPBA, or with other agents such as those known to one skilled in the art.

## Scheme VII

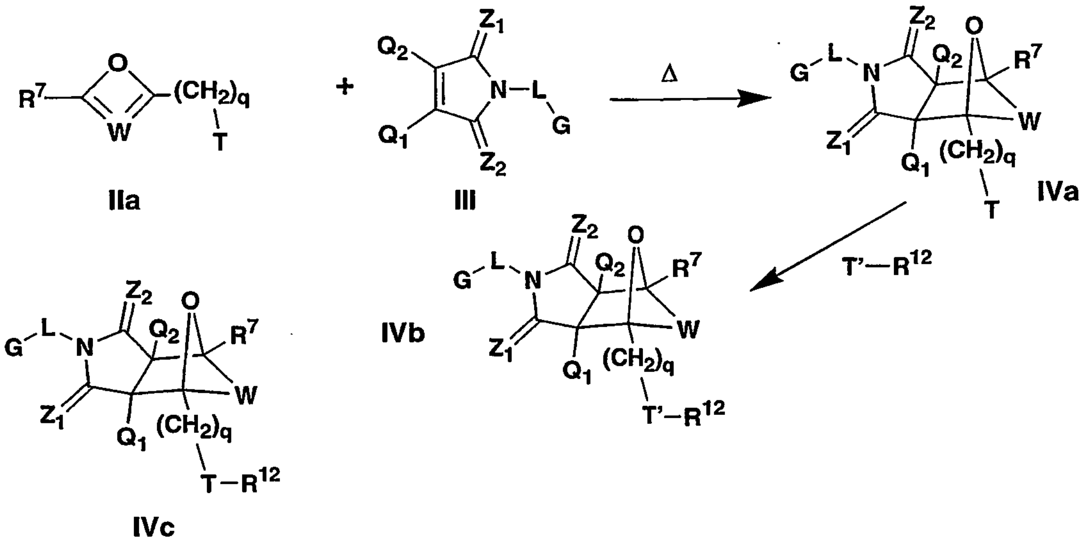


IIa III IVa IVb IVc

Scheme **VII** describes another approach to incorporating additional substitution onto a compound of formula **I.** As illustrated in Scheme **VII,** a diene of formula **IIa** can be reacted with a dienophile of formula **III,** as described in Scheme **I,** to yield a compound of formula **IVa,** which is a compound of formula **I** where Y is O, $A_2$ is $CR^7$ and $A_1$ is C-$(CH_2)_q$-T. The compound of formula **IVa** can be reacted with a reagent of formula $R^{12}$-T' to obtain a compound of formula **IVb** or **IVc** which are compounds of formula I where Y is O, $A_2$ is $CR^7$ and $A_1$ is C-$(CH_2)_q$-T'-$R^{12}$ or C-$(CH_2)_q$-T-$R^{12}$, respectively. The reagent $R^{12}$ -T' can be obtained from commercial sources or can readily be prepared by one skilled in the art.

In the above Scheme, $R^{12}$ has the same definition as $R^7$ defined earlier, q is zero or an integer from 0-8, and T is defined either as (1) a nucleophilic center such as, but not limited, to a nitrogen, oxygen or sulfur-containing group, capable of undergoing a nucleophilic substitution reaction with the leaving group T' or (2) a leaving group capable undergoing a nucleophilic substitution reaction with a nucleophilic group T' (such as, but not limited, to a nitrogen, oxygen or sulfur-containing nucleophilic group). T' has the same definition as T. In the present case, for example, a nucleophilic substitution reaction occurs when the attacking reagent (the nucleophile) brings an electron pair to the substrate, using

this pair to form the new bond, and the leaving group (the nucleofuge) comes away with the electron pair, leaving as an anionic intermediate. For a detailed discussion of the mechanism of aliphatic nucleophilic substitutions and a review of specific aliphatic nucleophilic substitution reactions see Advanced Organic Chemistry, Reactions, Mechanisms, and Structure, 4th Addition. Jerry March (Ed.), John Wiley & Sons, New York (1992) 293-500 and the references therein. Compounds of the formulae **IVa, IVb,** or **IVc** may, of course, be employed in the methods described herein (especially, in the treatment of nuclear hormone receptor-associated conditions) without undergoing further reaction of T or T'.

## Scheme VIII

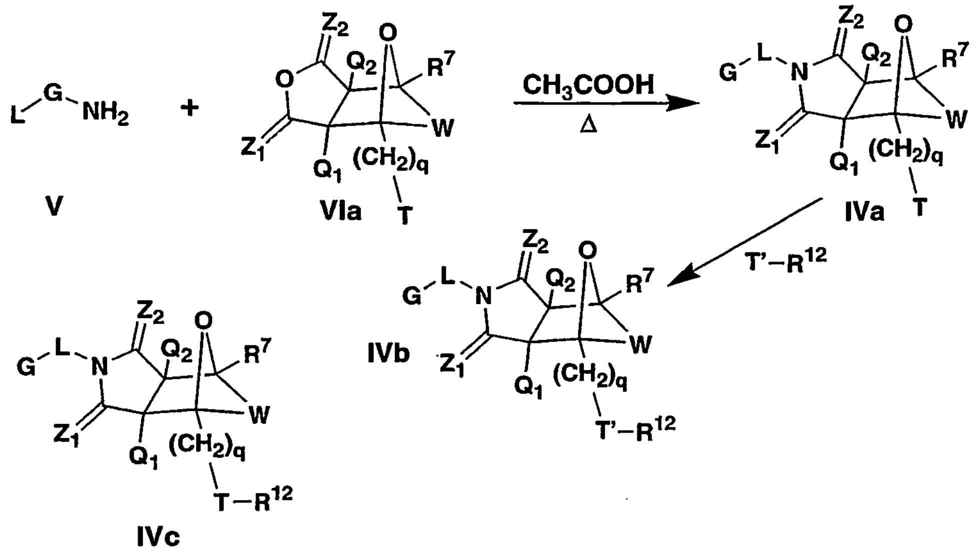


V VIa IVa IVb IVc

An alternate approach to compounds of formula **IVa, IVb** and **IVc** is illustrated in Scheme **VIII.** For this approach, techniques such as those described in Schemes **II, III** and **IV** can be applied to the preparation of an intermediate of formula **VIa,** where T and q are as defined in Scheme **VII.** The intermediate of formula **VIa** can be reacted with a substitited amine of formula **V,** as described in Scheme **II,** to yield the compound of formula **IVa,** which is a compound of formula **I** where Y is O, $A_2$ is $CR^7$ and $A_1$ is C-$(CH_2)_q$-T. The compound of formula **IVa** can be treated in the manner described in Scheme **VII** to obtain compounds of formula **IVb** or **IVc** which are compounds of formula **I** where Y is O, $A_2$ is $CR^7$ and $A_1$ is C-$(CH_2)_q$-T'-$R^{12}$ or C-$(CH_2)_q$-T-$R^{12}$, respectively.

## Scheme IX

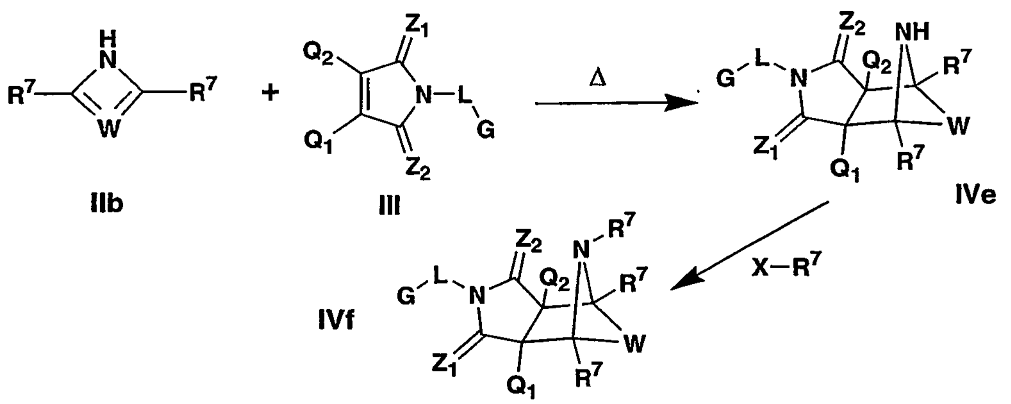


IIb III IVe IVf

Scheme **IX** describes another approach to incorporating further substitution onto a compound of formula **I.** As illustrated in Scheme **IX** (where X is a leaving group), a diene of formula **IIb** can be reacted with a dienophile of formula **III,** as described in Scheme **I,** to yield a compound of formula **IVe,** which is a compound of formula **I** where Y is NH, and $A_1$ and $A_2$ are $CR^7$. The compound of formula **IVe** can be functionalized at the free amine by reacting with a variety of electrophilic agents such as acid halides or alkyl halides in the presence of base, for example by methods known by one skilled in the art and described in Scheme **V,** to yield a compound of formula **IVf,** which is a compound of formula **I** where Y is $NR^7$ and $A_1$ and $A_2$ are $CR^7$.

## Scheme X

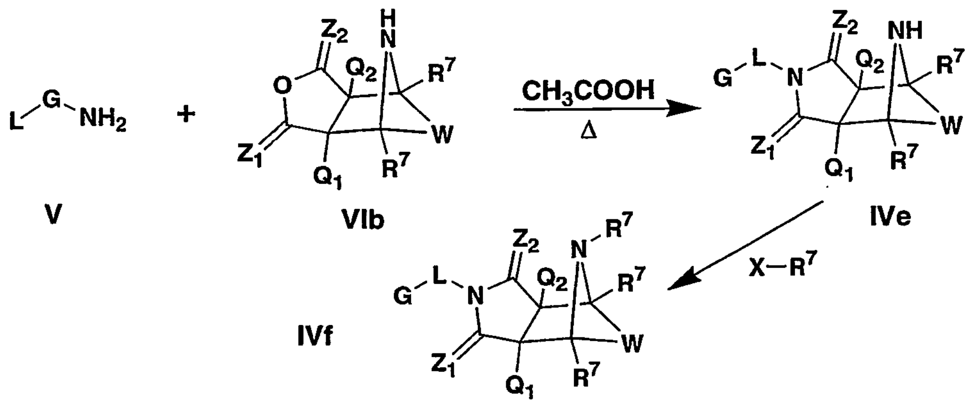


V VIb IVe IVf

An alternate approach to compounds of formula **IVe** and **IVf** is illustrated in Scheme **X.** For this approach, techniques as described in Schemes **II, III** and **IV** can be applied to the preparation of an intermediate of formula **VIb.** The intermediate of formula **VIb** can be reacted with a substituted amine of formula **V,** as described in Scheme **II,** to yield a compound of formula **IVe,** which is a compound of formula **I** where Y is NH, and $A_1$ and $A_2$ are $CR^7$. The latter intermediate can be treated in the manner described in Scheme **V** to obtain a compound of formula **IVf,** which is a compound of formula **I** where Y is $NR^7$, and $A_1$ and $A_2$ are $CR^7$.

## Scheme XI

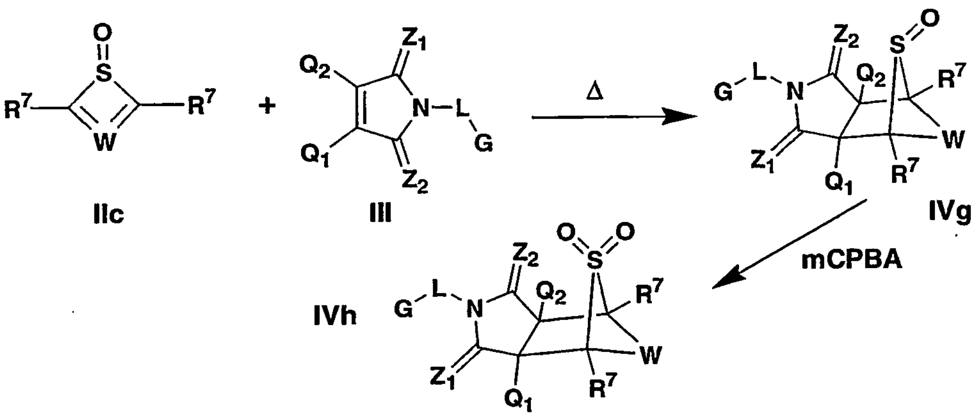


IIc III IVg IVh

Scheme **XI** describes another approach to incorporating additional substitution onto a compound of formula **I.** As illustrated in Scheme **XI,** a diene of formula **IIc** can be reacted with a dienophile of formula **III,** as described in Scheme **I,** to yield a compound of formula **IVg,** which is a compound of formula **I** where Y is SO and $A_1$ and $A_2$ are $CR^7$. A

compound of formula **IVg** can be treated with an oxidizing agent such as mCPBA, as described in Scheme **VI,** to yield a compound of formula **IVh,** which is a compound of formula **I** where Y is $SO_2$ and $A_1$ and $A_2$ are $CR^7$.

## Scheme XII

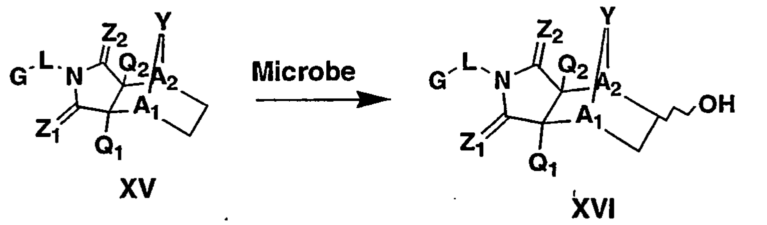


XV XVI

Scheme **XII** describes another approach to incorporating additional substitution onto a compound of formula **I.** As illustrated in Scheme **XII,** a compound of formula **XV,** which can be prepared in accordance with the above Schemes, can be incubated in the presence of a suitable enzyme or microorganism resulting in the formation of a hydroxylated analog of formula **XVI.** Such a process can be employed to yield regiospecific as well as enantiospecific incorporation of a hydroxyl group into a molecule of formula **XV** by a specific microorganism or by a series of different microorganisms. Such microorganisms can, for example, be bacterial, yeast or fungal in nature and can be obtained from distributors such as ATCC or identified for use in this method such as by methods known to one skilled in the art. Compound **XVI** is a compound of formula **I** where Y is as described above and $A_1$ and $A_2$ are preferably $CR^7$.

## Scheme XIII

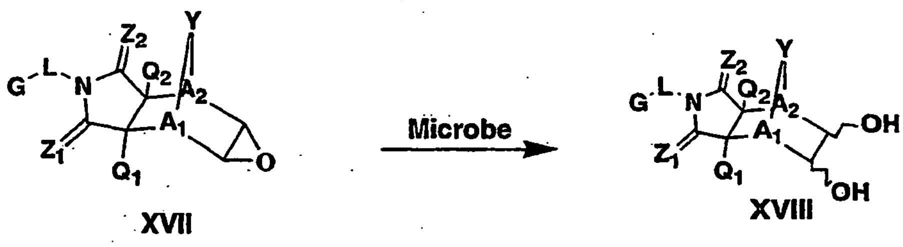


XVII XVIII

Scheme **XIII** describes another approach to incorporating additional substitution onto a compound of formula **I.** As illustrated in Scheme **XIII,** a compound of formula **XVII,** which can be prepared in accordance with the above Schemes, can be incubated in the presence of a suitable enzyme or microorganism resulting in the formation of a diol analog of formula **XVIII.** Such a process can be employed to yield regiospecific as well as enantiospecific transformation of a compound of formula **XVII** to a 1-2 diol of formula **XVIII** by a specific microorganism or by a series of different microorganisms. Such microorganisms can, for example, be bacterial, yeast or fungal in nature and can be obtained from distributors such as ATCC or identified for use in this method such as by methods known to one skilled in the art. Compound **XVIII** is a compound of formula I where **Y** is as described above and $A_1$ and $A_2$ are preferably $CR^7$.

The methods of Schemes **XII** and **XIII** are also described.

Thus, a method for the preparation of a compound of the following formula **XVI**, or salt thereof is disclosed.

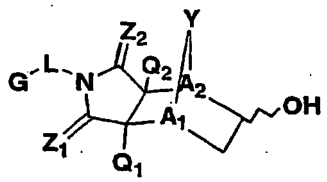

XVI

where the symbols are as defined herein,
comprising the steps of contacting a compound of the following formula XV, or salt thereof:

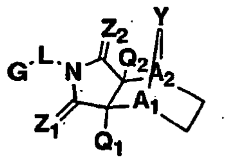

XV

where the symbols are as defined above;
with an enzyme or microorganism capable of catalyzing the hydroxylation of said compound XV to form said compound XVI, and effecting said hydroxylation.

A method for the preparation of a compound of the following formula XVIII, or salt thereof is also described.

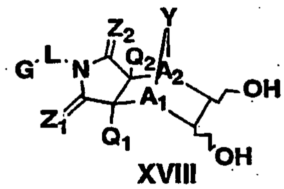

XVIII

where the symbols are as defined herein,
comprising the steps of contacting a compound of the following formula XVII, or salt thereof:

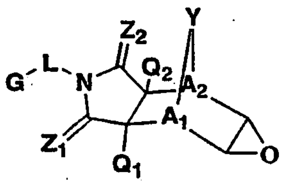

XVII

where the symbols are as defined above;
with an enzyme or microorganism capable of catalyzing the opening of the epoxide ring of compound XVII to form the diol of said compound XVIII, and effecting said ring opening and diol formation.

All stereoconfigurations of the unspecified chiral centers of the compounds of the formulae XV, XVI, XVII and XVIII are contemplated in the methods, either alone (that is, substantially free of other stereoisomers) or in admixture with other stereoisomeric forms. Conversion of one isomer selectively (e.g., hydroxylation of the exo isomer preferentially to hydroxylation of the endo isomer) when contacting an isomeric mixture is a preferred embodiment of the invention. Conversion to one isomer selectively (e.g., hydroxylation on the exo face "exo isomer" preferentially to the endo face "endo isomer" or regioselective opening of an epoxide to form only one of two possible regioisomers of a trans diol) is

preferred. Hydroxylation of an achiral intermediate to form a single optical isomer of the hydroxylated product is also preferred.

Resolution of a recemic mixture of an intermediate by selective hydroxylation, or epoxide ring opening and diol formation, to generate one of the two possible optical isomers is also preferred. The term "resolution" as used herein denotes partial, as well as, preferably, complete resolution.

The terms "enzymatic process" or "enzymatic method", as used herein, denote a process or method employing an enzyme or microorganism. The term "hydroxylation", as used herein, denotes the addition of a hydroxyl group to a methylene group as described above. Hydroxylation can be achieved, for example, by contact with molecular oxygen according to the methods of the present invention. Diol formation can be achieved, for example, by contact with water according to the methods disclosed herein. Use of "an enzyme or microorganism" in the present methods includes use of two or more, as well as a single, enzyme or microorganism.

The enzyme or microorganism employed herein can be any enzyme or microorganism capable of catalyzing the enzymatic conversions described herein. The enyzmatic or microbial materials, regardless of origin or purity, can be employed in the free state or immobilized on a support such as by physical adsorption or entrapment. Microorganisms or enzymes suitable for use in the present methods can be selected by screening for the desired activity, for example, by contacting a candidate microorganism or enzyme with a starting compound XV or XVII or salt thereof, and noting conversion to the corresponding compound XVI or XVIII or salt thereof. The enzyme may, for example, be in the form of animal or plant enzymes or mixtures thereof, cells of microorganisms, crushed cells, extracts of cells, or of synthetic origin.

Exemplary microorganisms include those within the genera: *Streptomyces* or *Amycolatopsis.* Particularly preferred microorganisms are those within the species *Streptomyces griseus,* especially *Streptomyces griseus* ATCC 10137, and *Amycolatopsis orientalis* such as ATCC 14930, ATCC 21425, ATCC 35165, ATCC 39444, ATCC 43333, ATCC 43490, ATCC 53550, ATCC 53630, and especially ATCC 43491. The term "ATCC" as used herein refers to the accession number of the American Type Culture Collection, 10801 University Blvd., Manassas Virginia 20110-2209, the depository for the organism referred to. It should be understood that mutants of these organisms are also contemplated by the present invention, for use in the methods described herein, such as those modified by the use of chemical, physical (for example, X-rays) or biological means (for example, by molecular biology techniques).

Preferred enzymes include those derived from microorganisms, particularly those microorganisms described above. Enzymes may be isolated, for example, by extraction and purification methods such as by methods known to those of ordinary skill in the art. An enzyme may, for example, be used in its free state or in immobilized form. One embodiment of the invention is that where an enzyme is adsorbed onto a suitable carrier, e.g., diatomaceous earth (porous Celite Hyflo Supercel), microporous polypropylene (Enka Accurel® polypropylene powder), or a nonionic polymeric adsorbent such as Amberlite® XAD-2 (polystyrene) or XAD-7 (polyacrylate) from Rohm and Haas Co. When employed to immobilize an enzyme, a carrier may control the enzyme particle size and prevent aggregation of the enzyme particles when used in an organic solvent. Immobilization can be accomplished, for example, by precipitating an aqueous solution of the enzyme with cold acetone in the presence of the Celite Hyflo Supercel followed by vacuum drying, or in the case of a nonionic polymeric adsorbent, incubating enzyme solutions with adsorbent on a shaker, removing excess solution and drying enzyme-adsorbent resins under vacuum. While it is desirable to use the least amount of enzyme possible, the amount of enzyme required will vary depending upon the specific activity of the enzyme used.

Hydroxylation as described above can occur *in vivo.* For example, liver enzyme can selectively, relative to the endo isomer, hydroxylate the exo isomer of a compound of the present invention. In conducting the methods outside the body, liver microsomal hydroxylase can be employed as the enzyme for catalysis.

These processes may also be carried out using microbial cells containing an enzyme having the ability to catalyze the conversions. When using a microorganism to perform the conversion, these procedures are conveniently carried out by adding the cells and the starting material to the desired reaction medium.

Where microorganisms are employed, the cells may be used in the form of intact wet cells or dried cells such as lyophilized, spray-dried or heat-dried cells, or in the form of treated cell material such as ruptured cells or cell extracts. Cell extracts immobilized on Celite® or Accurel® polypropylene as described earlier may also be employed. The use of generically engineered organisms is also contemplated. The host cell may be any cell, e.g. *Escherichia coli,* modified to contain a gene or genes for expressing one or more enzymes capable of catalysis as described herein.

Where one or more microorganisms are employed, the enzymatic methods may be carried out subsequent to the fermentation of the microorganism (two-stage fermentation and conversion), or concurrently therewith, that is, in the latter case, by *in situ* fermentation and conversion (single-stage fermentation and conversion).

Growth of the microorganisms can be achieved by one of ordinary skill in the art by the use of an appropriate medium. Appropriate media for growing microorganisms include those which provide nutrients necessary for the growth of the microbial cells. A typical medium for growth includes necessary carbon sources, nitrogen sources, and elements (e.g. in trace amounts). Inducers may also be added. The term "inducer", as used herein, includes any compound

enhancing formation of the desired enzymatic activity within the microbial cell.

Carbon sources can include sugars such as maltose, lactose, glucose, fructose, glycerol, sorbitol, sucrose, starch, mannitol, propylene glycol, and the like; organic acids such as sodium acetate, sodium citrate, and the like; and alcohols such as ethanol, propanol and the like.

Nitrogen sources can include N-Z amine A, corn steep liquor, soy bean meal, beef extracts, yeast extracts, molasses, baker's yeast, tryptone, nutrisoy, peptone, yeastamin, amino acids such as sodium glutamate and the like, sodium nitrate, ammonium sulfate and the like.

Trace elements can include magnesium, manganese, calcium, cobalt, nickel, iron, sodium and potassium salts. Phosphates may also be added in trace or, preferably, greater than trace amounts.

The medium employed can include more than one carbon or nitrogen source or other nutrient.

Preferred media for growth include aqueous media.

The agitation and aeration of the reaction mixture affects the amount of oxygen available during the conversion process when conducted, for example, in shake-flask cultures or fermentor tanks during growth of microorganisms.

Incubation of the reaction medium is preferably at a temperature between about 4 and about 60°C. The reaction time can be appropriately varied depending upon the amount of enzyme used and its specific activity. Reaction times may be reduced by increasing the reaction temperature and/or increasing the amount of enzyme added to the reaction solution.

It is also preferred to employ an aqueous liquid as the reaction medium, although an organic liquid, or a miscible or immiscible (biphasic) organic/aqueous liquid mixture, may also be employed. The amount of enzyme or microorganism employed relative to the starting material is selected to allow catalysis of the enzymatic conversions of the present invention.

Solvents for the organic phase of a biphasic solvent system may be any organic solvent immiscible in water, such as toluene, cyclohexane, xylene, trichlorotrifluoroethane and the like. The aqueous phase is conveniently of water, preferably deionized water, or a suitable aqueous buffer solution, especially a phosphate buffer solution. The biphasic solvent system preferably comprises between about 10 to 90 percent by volume of organic phase and between about 90 to 10 percent by volume of aqueous phase, and most preferably contains at or about 20 percent by volume of organic phase and at or about 80 percent by volume of the aqueous phase.

An exemplary embodiment of such processes starts with preparation of an aqueous solution of the enzyme(s) or microbes to be used. For example, the preferred enzyme(s) or microbes can be added to a suitable amount of an aqueous solvent, such as phosphate buffer or the like. This mixture is preferably adjusted to and maintained at a desired pH.

The compounds XVI and XVIII produced by the present processes can be isolated and purified, for example, by methods such as extraction, distillation, crystallization, and column chromatography.

## Use and Utility

The compounds of the present invention modulate the function of nuclear hormone receptors (NHR), and include compounds which are, for example, agonists, partial agonists, antagonists or partial antagonists of the androgen receptor (AR), the estrogen receptor (ER), the progesterone receptor (PR), the glucocorticoid receptor (GR), the mineralocorticoid receptor (MR), the steroid and xenobiotic receptor (SXR), other steroid binding NHR's, the Orphan receptors or other NHR's. Selective modulation of one such NHR relative to others within the NHR family is preferred. "Modulation" includes, for example, activation (e.g., agonist activity such as selective androgen receptor agonist activity) or inhibition (e.g., antagonist activity).

The present compounds are thus useful in the treatment of NHR-associated conditions. A "NHR-associated condition", as used herein, denotes a condition or disorder which can be treated by modulating the function of a NHR in a subject, wherein treatment comprises prevention (e.g., prophylactic treatment), partial alleviation or cure of the condition or disorder. Modulation may occur locally, for example, within certain tissues of the subject, or more extensively throughout a subject being treated for such a condition disorder.

The compounds of the present invention are useful for the treatment of a variety of conditions and disorders including, but not limited to, those described following:

Compounds of formula I can be applied as agonists, partial agonists, antagonists, or partial antagonists of the estrogen receptor, preferably selectively to that receptor, in an array of medical conditions which involve modulation of the estrogen receptor pathway. Applications of said compounds include but are not limited to: osteoporosis, hot flushes, vaginal dryness, prostate cancer, breast cancer, endometrial cancer, cancers expressing the estrogen receptor such as the aforementioned cancers and others, contraception, pregnancy termination, menopause, amennoreahea, and dysmennoreahea.

Compounds of formula I can be applied as agonists, partial agonists, antagonists or partial antagonists of the progesterone receptor, preferably selectively to that receptor, in an array of medical conditions which involve modulation

of the progesterone receptor pathway. Applications of said compounds include but are not limited to: breast cancer, other cancers containing the progesterone receptor, endometriosis, cachexia, contraception, menopause, cyclesynchrony, meniginoma, dysmennoreahea, fibroids, pregnancy termination, labor induction and osteoporosis.

Compounds of formula I can be applied as agonists, partial agonists, antagonists or partial antagonists of the glucocorticoid receptor, preferably selectively to that receptor, in an array of medical conditions which involve modulation of the glucocorticoid receptor pathway. Applications of said compounds include but are not limited to: inflammatory diseases, autoimmune diseases, prostate cancer, breast cancer, Alzheimer's disease, psychotic disorders, drug dependence, non-insulin dependent Diabetes Mellitus, and as dopamine receptor blocking agents or otherwise as agents for the treatment of dopamine receptor mediated disorders.

Compounds of formula I can be applied as agonists, partial agonists, antagonists or partial antagonists of the mineralocorticoid receptor, preferably selectively to that receptor, in an array of medical conditions which involve modulation of the mineralocorticoid receptor pathway. Applications of said compounds include but are not limited to: drug withdrawal syndrome and inflammatory diseases.

Compounds of formula I can be applied as agonists, partial agonists, antagonists or partial antagonists of the aldosterone receptor, preferably selectively to that receptor, in an array of medical conditions which involve modulation of the aldosterone receptor pathway. One application of said compounds includes but is not limited to: congestive heart failure.

Compounds of formula I can be applied as agonists, partial agonists, antagonists or partial antagonists of the androgen receptor, preferably selectively to that receptor, in an array of medical conditions which involve modulation of the androgen receptor pathway. Applications of said compounds include but are not limited to: hirsutism, acne, seborrhea, Alzheimer's disease, androgenic alopecia, hypogonadism, hyperpilosity, benign prostate hypertrophia, adenomas and neoplasies of the prostate (such as advanced metastatic prostate cancer), treatment of benign or malignant tumor cells containing the androgen receptor such as is the case for breast, brain, skin, ovarian, bladder, lymphatic, liver and kidney cancers, pancreatic cancers modulation of VCAM expression and applications therein for the treatment of heart disease, inflammation and immune modulations, modulation of VEGF expression and the applications therein for use as antiangiogenic agents, osteoporosis, suppressing spermatogenesis, libido, cachexia, endometriosis, polycystic ovary syndrome, anorexia, androgen supplement for age related decreased testosterone levels in men, male menopause, male hormone replacement, male and female sexual dysfunction, and inhibition of muscular atrophy in ambulatory patients. For example, pan AR modulation is contemplated, with prostate selective AR modulation ("SARM") being particularly preferred, such as for the treatment of early stage prostate cancers.

Compounds of formula I can be applied as (preferably, selective) antagonists of the mutated androgen receptor, for example, found in many tumor lines. Examples of such mutants are those found in representative prostate tumor cell lines such as LNCap, (T877A mutation, Biophys. Acta, 187, 1052 (1990)), PCa2b, (L701H & T877A mutations, J. Urol., 162, 2192 (1999)) and CWR22, (H874Y mutation, Mol. Eildo., 11, 450 (1997)). Applications of said compounds include but are not limited to: adenomas and neoplasies of the prostate, breast cancer and endometrial cancer.

Compounds of formula I can be applied as agonists, partial agonists, antagonists or partial antagonists of the steroid and xenobiotic receptor, preferably selectively to that receptor, in an array of medical conditions which involve modulation of the steroid and xenobiotic receptor pathway. Applications of said compounds include but are not limited to: treatment of disregulation of cholesterol homeostasis, attenuation of metabolism of pharmaceutical agents by co-administration of an agent (compound of the present invention) which modulates the P450 regulator effects of SXR.

Along with the aforementioned NHR, there also exist a number of NHR for which the activating or deactivating ligands may not be characterized. These proteins are classified as NHR due to strong sequence homology to other NHR, and are known as the Orphan receptors. Because the Orphan receptors demonstrate strong sequence homology to other NHR, compounds of formula I include those which serve as modulators of the function of the Orphan NHR. Orphan receptors which are modulated by NHR modulators such as compounds within the scope of formula I are exemplified, but not limited to, those listed in Table 1. Exemplary therapeutic applications of modulators of said Orphan receptors are also listed in Table 1, but are not limited to the examples therein.

**Table 1**. Exemplary Orphan nuclear hormone receptors, form (M = monomeric, D = heterodimeric, H = homodimeric), tissue expression and target therapeutic applications. (CNS=central nervous system)

| Receptor | Form | Tissue Expression | Target Therapeutic Application |
|---|---|---|---|
| NURR1 | M/D | Dopaminergic Neurons | Parkinson's Disease |
| RZRβ | M | Brain (Pituitary), Muscle | Sleep Disorders |
| RORα | M | Cerebellum, Purkinje Cells | Arthritis, Cerebellar Ataxia |

(continued)

| Receptor | Form | Tissue Expression | Target Therapeutic Application |
|---|---|---|---|
| NOR-1 | M | Brain, Muscle, Heart, Adrenal, Thymus | CNS Disorders, Cancer |
| NGFI-Bβ | M/D | Brain | CNS Disorders |
| COUP-Tfα | H | Brain | CNS Disorders |
| COUP-TFβ | H | Brain | CNS Disorders |
| COUP-TFγχ | H | Brain | CNS Disorders |
| Nur77 | H | Brain, Thymus, Adrenals | CNS Disorders |
| Rev-ErbAα | H | Muscle, Brain (Ubiquitous) | Obesity |
| HNF4α | H | Liver, Kidney, Intestine | Diabetes |
| SF-1 | M | Gonads, Pituitary | Metabolic Disorders |
| LXRα,β | D | Kidney (Ubiquitous) | Metabolic Disorders |
| GCNF | M/H | Testes, Ovary | Infertility |
| ERRα,β | M | Placenta, Bone | Infertility, Osteoporosis |
| FXR | D | Liver, Kidney | Metabolic Disorders |
| CARα | H | Liver, Kidney | Metabolic Disorders |
| PXR | H | Liver, Intestine | Metabolic Disorders |
| COUP-TF2 (ARP1) | D | Testis | Oncology/angiogenesis |
| RORbeta | M | CNS, retina, pineal gland | Metabolic Disorders |

The present invention thus provides the use of at least one compound of formula I for the manufacture of a medicament for the treatment of NHR-associated conditions. Other therapeutic agents such as those described below may be employed with the inventive compounds in the present methods (for example, separately, or formulated together as a fixed dose). In the methods such other therapeutic agent(s) can be administered prior to, simultaneously with or following the administration of the compound(s) of the present invention.

The present invention also provides pharmaceutical compositions comprising at least one of the claimed compounds capable of treating a NHR-associated condition in an amount effective therefor, and a pharmaceutically acceptable carrier (vehicle or diluent). The compositions of the present invention can contain other therapeutic agents as described below, and can be formulated, for example, by employing conventional solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation.

It should be noted that the compounds of the present invention are, without limitation as to their mechanism of action, useful in treating any of the conditions or disorders listed or described herein such as inflammatory diseases or cancers, or other proliferate diseases, and in compositions for treating such conditions or disorders. Such conditions and disorders include, without limitation, any of those described previously, as well as those described following such as: maintenance of muscle strength and function (e.g., in the elderly); reversal or prevention of frailty or age-related functional decline ("ARFD") in the elderly (e.g., sarcopenia); treatment of catabolic side effects of glucocorticoids; prevention and/or treatment of reduced bone mass, density or growth (e.g., osteoporosis and osteopenia); treatment of chronic fatigue syndrome (CFS); chronic malagia; treatment of acute fatigue syndrome and muscle loss following elective surgery (e.g., post-surgical rehabilitation); acceleration of wound healing; accelerating bone fracture repair (such as accelerating the recovery of hip fracture patients); accelerating healing of complicated fractures, e.g. distraction osteogenesis; in joint replacement; prevention of post-surgical adhesion formation; acceleration of tooth repair or growth; maintenance of sensory function (e.g., hearing, sight, olefaction and taste); treatment of periodontal disease; treatment of wasting secondary to fractures and wasting in connection with chronic obstructive pulmonary disease (COPD), chronic liver disease, AIDS, weightlessness, cancer cachexia, burn and trauma recovery, chronic catabolic state (e.g., coma), eating disorders (e.g., anorexia) and chemotherapy; treatment of cardiomyopathy; treatment of thrombocytopenia; treatment of growth retardation in connection with Crohn's disease; treatment of short bowel syndrome; treatment of irritable bowel syndrome; treatment of inflammatory bowel disease; treatment of Crohn's disease and ulcerative colits; treatment of complications associated with transplantation; treatment of physiological short stature including growth hormone deficient children and short stature associated with chronic illness; treatment of obesity and growth retardation associated

with obesity; treatment of anorexia (e.g., associated with cachexia or aging); treatment of hypercortisolism and Cushing's syndrome; Paget's disease; treatment of osteoarthritis; induction of pulsatile growth hormone release; treatment of osteochondrodysplasias; treatment of depression, nervousness, irritability and stress; treatment of reduced mental energy and low self-esteem (e.g., motivation/assertiveness); improvement of cognitive function (e.g., the treatment of dementia, including Alzheimer's disease and short term memory loss); treatment of catabolism in connection with pulmonary dysfunction and ventilator dependency; treatment of cardiac dysfunction (e.g., associated with valvular disease, myocardial infarction, cardiac hypertrophy or congestive heart failure); lowering blood pressure; protection against ventricular dysfunction or prevention of reperfusion events; treatment of adults in chronic dialysis; reversal or slowing of the catabolic state of aging; attenuation or reversal of protein catabolic responses following trauma (e.g., reversal of the catabolic state associated with surgery, congestive heart failure, cardiac myopathy, burns, cancer, COPD etc.); reducing cachexia and protein loss due to chronic illness such as cancer or AIDS; treatment of hyperinsulinemia including nesidioblastosis; treatment of immunosuppressed patients; treatment of wasting in connection with multiple sclerosis or other neurodegenerative disorders; promotion of myelin repair; maintenance of skin thickness; treatment of metabolic homeostasis and renal homeostasis (e.g., in the frail elderly); stimulation of osteoblasts, bone remodeling and cartilage growth; regulation of food intake; treatment of insulin resistance, including NIDDM, in mammals (e.g., humans); treatment of insulin resistance in the heart; improvement of sleep quality and correction of the relative hyposomatotropism of senescence due to high increase in REM sleep and a decrease in REM latency; treatment of hypothermia; treatment of congestive heart failure; treatment of lipodystrophy (e.g., in patients taking HIV or AIDS therapies such as protease inhibitors); treatment of muscular atrophy (e.g., due to physical inactivity, bed rest or reduced weight-bearing conditions); treatment of musculoskeletal impairment (e.g., in the elderly); improvement of the overall pulmonary function; treatment of sleep disorders; and the treatment of the catabolic state of prolonged critical illness; treatment of hirsutism, acne, seborrhea, androgenic alopecia, anemia, hyperpilosity, benign prostate hypertrophy, adenomas and neoplasies of the prostate (e.g., advanced metastatic prostate cancer) and malignant tumor cells containing the androgen receptor, such as is the case for breast, brain, skin, ovarian, bladder, lymphatic, liver and kidney cancers; cancers of the skin, pancreas, endometrium, lung and colon; osteosarcoma; hypercalcemia of malignancy; metastatic bone disease; treatment of spermatogenesis, endometriosis and polycystic ovary syndrome; conteracting preeclampsia, eclampsia of pregnancy and preterm labor; treatment of premenstrual syndrome; treatment of vaginal dryness; age related decreased testosterone levels in men, male menopause, hypogonadism, male hormone replacement, male and female sexual dysfunction (e.g., erectile dysfunction, decreased sex drive, sexual well-being, decreased libido), male and female contraception, hair loss, Reaven's Syndrome and the enhancement of bone and muscle performance/strength; and the conditions, diseases, and maladies collectively referenced to as "Syndrome X" or Metabolic Syndrome as detailed in Johannsson J. Clin. Endocrinol. Metab., 82, 727-34 (1997).

The present compounds have therapeutic utility in the modulation of immune cell activation/proliferation, e.g., as competitive inhibitors of intercellular ligand/receptor binding reactions involving CAMs (Cellular Adhesion Molecules) and Leukointegrins. For example, the present compounds modulate LFA-ICAM 1, and are particularly useful as LFA-ICAM 1 antagonists, and in the treatment of all conditions associated with LFA-ICAM 1 such as immunological disorders. Preferred utilities for the present compounds include, but are not limited to: inflammatory conditions such as those resulting from a response of the non-specific immune system in a mammal (e.g., adult respiratory distress syndrome, shock, oxygen toxicity, multiple organ injury syndrome secondary to septicemia, multiple organ injury syndrome secondary to trauma, reperfusion injury of tissue due to cardiopulmonary bypass, myocardial infarction or use with thrombolysis agents, acute glomerulonephritis, vasculitis, reactive arthritis, dermatosis with acute inflammatory components, stroke, thermal injury, hemodialysis, leukapheresis, ulcerative colitis, necrotizing enterocolitis and granulocyte transfusion associated syndrome) and conditions resulting from a response of the specific immune system in a mammal (e.g., psoriasis, organ/tissue transplant rejection, graft vs. host reactions and autoimmune diseases including Raynaud's syndrome, autoimmune thyroiditis, dermatitis, multiple sclerosis, rheumatoid arthritis, insulin-dependent diabetes mellitus, uveitis, inflammatory bowel disease including Crohn's disease and ulcerative colitis, and systemic lupus erythematosus). The present compounds can be used in treating asthma or as an adjunct to minimize toxicity with cytokine therapy in the treatment of cancers. The present compounds can be employed in the treatment of all diseases currently treatable through steroid therapy. The present compounds may be employed for the treatment of these and other disorders alone or with other immunosuppressive or antiinflammatory agents. In accordance with the invention, a compound of the formula I can be administered prior to the onset of inflammation (so as to suppress an anticipated inflammation) or after the initiation of inflammation. When provided prophylactically, the immunosupressive compound(s) are preferably provided in advance of any inflammatory response or symptom (for example, prior to, at, or shortly after the time of an organ or tissue transplant but in advance of any symptoms or organ rejection). The prophylactic administration of a compound of the formula I prevents or attenuates any subsequent inflammatory response (such as, for example, rejection of a transplanted organ or tissue, etc.) Administration of a compound of the formula I attenuates any actual inflammation (such as, for example, the rejection of a transplanted organ or tissue).

The compounds of the formula I can be administered for any of the uses described herein by any suitable

means, for example, orally, such as in the form of tablets, capsules, granules or powders; sublingually; bucally; parenterally, such as by subcutaneous, intravenous, intramuscular, or intrasternal injection or infusion techniques (e.g., as sterile injectable aqueous or non-aqueous solutions or suspensions); nasally, including administration to the nasal membranes, such as by inhalation spray; topically, such as in the form of a cream or ointment; or rectally such as in the form of suppositories; in dosage unit formulations containing non-toxic, pharmaceutically acceptable vehicles or diluents. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The present compounds can also be administered liposomally.

Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate and/or lactose and/or other excipients, binders, extenders, disintegrants, diluents and lubricants such as those known in the art. The compounds of formula I can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use.

Exemplary compositions for nasal aerosol or inhalation administration include solutions in saline which can contain, for example, benzyl alcohol or other suitable preservatives, absorption promoters to enhance bioavailability, and/or other solubilizing or dispersing agents such as those known in the art.

Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor.

Exemplary compositions for rectal administration include suppositories which can contain, for example, a suitable non-irritating excipient, such as cocoa butter, synthetic glyceride esters or polyethylene glycols, which are solid at ordinary temperatures, but liquify and/or dissolve in the rectal cavity to release the drug.

Exemplary compositions for topical administration include a topical carrier such as Plastibase (mineral oil gelled with polyethylene).

The effective amount of a compound of the present invention can be determined by one of ordinary skill in the art, and includes exemplary dosage amounts for an adult human of from about 1 to 100 (for example, 15 or lower, especially 1 to 3 or less) mg/kg of body weight of active compound per day, which can be administered in a single dose or in the form of individual divided doses, such as from 1 to 4 times per day. It will be understood that the specific dose level and frequency of dosage for any particular subject can be varied and will depend upon a variety of factors including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats and the like, subject to NHR-associated conditions.

As mentioned above, the compounds of the present invention can be employed alone or in combination with each other and/or other suitable therapeutic agents useful in the treatment of NHR-associated conditions, e.g., an antibiotic or other pharmaceutically active material.

For example, the compounds of the present invention can be combined with growth promoting agents, such as, but not limited to, TRH, diethylstilbesterol, theophylline, enkephalins, E series prostaglandins, compounds disclosed in U.S. Patent No. 3,239,345, e.g., zeranol, and compounds disclosed in U.S. Patent No. 4,036,979, e.g., sulbenox or peptides disclosed in U.S. Patent No. 4,411,890.

The compounds of the invention can also be used in combination with growth hormone secretagogues such as GHRP-6, GHRP-1 (as described in U.S. Patent No. 4,411,890 and publications WO 89/07110 and WO 89/07111), GHRP-2 (as described in WO 93/04081), NN703 (Novo Nordisk), LY444711 (Lilly), MK-677 (Merck), CP424391 (Pfizer) and B-HT920, or with growth hormone releasing factor and its analogs or growth hormone and its analogs or somatomedins including IGF-1 and IGF-2, or with alpha-adrenergic agonists, such as clonidine or serotinin 5-$HT_D$ agonists, such as sumatriptan, or agents which inhibit somatostatin or its release, such as physostigmine and pyridostigmine. A still further use of the disclosed compounds of the invention is in combination with parathyroid hormone, PTH(1-34) or

bisphosphonates, such as MK-217 (alendronate).

A still further use of the compounds of the invention is in combination with estrogen, testosterone, a selective estrogen receptor modulator, such as tamoxifen or raloxifene, or other androgen receptor modulators, such as those disclosed in Edwards, J. P. et al., Bio. Med. Chem. Let., 9, 1003-1008 (1999) and Hamann, L. G. et al., J. Med. Chem., 42, 210-212 (1999).

A further use of the compounds of this invention is in combination with progesterone receptor agonists ("PRA"), such as levonorgestrel, medroxyprogesterone acetate (MPA).

The compounds of the present invention can be employed alone or in combination with each other and/or other modulators of nuclear hormone receptors or other suitable therapeutic agents useful in the treatment of the aforementioned disorders including: anti-diabetic agents; anti-osteoporosis agents; anti-obesity agents; anti-inflammatory agents; anti-anxiety agents; anti-depressants; anti-hypertensive agents; anti-platelet agents; anti-thrombotic and thrombolytic agents; cardiac glycosides; cholesterol/lipid lowering agents; mineralocorticoid receptor antagonists; phospodiesterase inhibitors; protein tyrosine kinase inhibitors; thyroid mimetics (including thyroid receptor agonists); anabolic agents; HIV or AIDS therapies; therapies useful in the treatment of Alzheimer's disease and other cognitive disorders; therapies useful in the treatment of sleeping disorders; anti-proliferative agents; and anti-tumor agents.

Examples of suitable anti-diabetic agents for use in combination with the compounds of the present invention include biguanides (e.g., metformin), glucosidase inhibitors (e.g,. acarbose), insulins (including insulin secretagogues or insulin sensitizers), meglitinides (e.g., repaglinide), sulfonylureas (e.g., glimepiride, glyburide and glipizide), biguanide/glyburide combinations (e.g., Glucovance®), thiazolidinediones (e.g., troglitazone, rosiglitazone and pioglitazone), PPAR-alpha agonists, PPAR-gamma agonists, PPAR alpha/gamma dual agonists, SGLT2 inhibitors, glycogen phosphorylase inhibitors, inhibitors of fatty acid binding protein (aP2) such as those disclosed in U.S. Serial No. 09/519,079 filed March 6, 2000, glucagon-like peptide-1 (GLP-1), and dipeptidyl peptidase IV (DP4) inhibitors.

Examples of suitable anti-osteoporosis agents for use in combination with the compounds of the present invention include alendronate, risedronate, PTH, PTH fragment, raloxifene, calcitonin, steroidal or non-steroidal progesterone receptor agonists, RANK ligand antagonists, calcium sensing receptor antagonists, TRAP inhibitors, selective estrogen receptor modulators (SERM), estrogen and AP-1 inhibitors.

Examples of suitable anti-obesity agents for use in combination with the compounds of the present invention include aP2 inhibitors, such as those disclosed in U.S. Serial No. 09/519,079 filed March 6, 2000, PPAR gamma antagonists, PPAR delta agonists, beta 3 adrenergic agonists, such as AJ9677 (Takeda/Dainippon), L750355 (Merck), or CP331648 (Pfizer) or other known beta 3 agonists as disclosed in U.S. Patent Nos. 5,541,204, 5,770,615, 5,491,134, 5,776,983 and 5,488,064, a lipase inhibitor, such as orlistat or ATL-962 (Alizyme), a serotonin (and dopamine) reuptake inhibitor, such as sibutramine, topiramate (Johnson & Johnson) or axokine (Regeneron), a thyroid receptor beta drug, such as a thyroid receptor ligand as disclosed in WO 97/21993 (U. Cal SF), WO 99/00353 (KaroBio) and GB98/284425 (KaroBio), and/or an anorectic agent, such as dexamphetamine, phentermine, phenylpropanolamine or mazindol.

Examples of suitable anti-inflammatory agents for use in combination with the compounds of the present invention include prednisone, dexamethasone, Enbrel®, cyclooxygenase inhibitors (i.e., COX-1 and/or COX-2 inhibitors such as NSAIDs, aspirin, indomethacin, ibuprofen, piroxicam, Naproxen®, Celebrex®, Vioxx®), CTLA4- Ig agonists/antagonists, CD40 ligand antagonists, IMPDH inhibitors, such as mycophenolate (CellCept®) integrin antagonists, alpha-4 beta-7 integrin antagonists, cell adhesion inhibitors, interferon gamma antagonists, ICAM-1, tumor necrosis factor (TNF) antagonists (e.g., infliximab, OR1384), prostaglandin synthesis inhibitors, budesonide, clofazimine, CNI-1493, CD4 antagonists (e.g., priliximab), p38 mitogen-activated protein kinase inhibitors, protein tyrosine kinase (PTK) inhibitors, IKK inhibitors, and therapies for the treatment of irritable bowel syndrome (e.g., Zelmac® and Maxi-K® openers such as those disclosed in U.S. Patent No. 6,184,231 B1).

Example of suitable anti-anxiety agents for use in combination with the compounds of the present invention include diazepam, lorazepam, buspirone, oxazepam, and hydroxyzine pamoate.

Examples of suitable anti-depressants for use in combination with the compounds of the present invention include citalopram, fluoxetine, nefazodone, sertraline, and paroxetine.

Examples of suitable anti-hypertensive agents for use in combination with the compounds of the present invention include beta adrenergic blockers, calcium channel blockers (L-type and T-type; e.g. diltiazem, verapamil, nifedipine, amlodipine and mybefradil), diuretics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetanide, triamtrenene, amiloride, spironolactone), renin inhibitors, ACE inhibitors (e.g., captopril, zofenopril, fosinopril, enalapril, ceranopril, cilazopril, delapril, pentopril, quinapril, ramipril, lisinopril), AT-1 receptor antagonists (e.g., losartan, irbesartan, valsartan), ET receptor antagonists (e.g., sitaxsentan, atrsentan and compounds disclosed in U.S. Patent Nos. 5,612,359 and 6,043,265), Dual ET/AII antagonist (e.g., compounds disclosed in WO 00/01389), neutral endopeptidase (NEP) inhibitors, vasopepsidase inhibitors (dual NEP-ACE inhibitors) (e.g., omapatrilat and gemopatrilat), and nitrates.

Examples of suitable anti-platelet agents for use in combination with the compounds of the present invention

include GPIIb/IIIa blockers (e.g., abciximab, eptifibatide, tirofiban), P2Y12 antagonists (e.g., clopidogrel, ticlopidine, CS-747), thromboxane receptor antagonists (e.g., ifetroban), aspirin, and PDE-III inhibitors (e.g., dipyridamole) with or without aspirin.

Examples of suitable cardiac glycosides for use in combination with the compounds of the present invention include digitalis and ouabain.

Examples of suitable cholesterol/lipid lowering agents for use in combination with the compounds of the present invention include HMG-CoA reductase inhibitors (e.g., pravastatin, lovastatin, atorvastatin, simvastatin, NK-104 (a.k.a. itavastatin, or nisvastatin or nisbastatin) and ZD-4522 (a.k.a. rosuvastatin, or atavastatin or visastatin)), squalene synthetase inhibitors, fibrates, bile acid sequestrants, ACAT inhibitors, MTP inhibitors, lipooxygenase inhibitors, cholesterol absorption inhibitors, and cholesterol ester transfer protein inhibitors (e.g., CP-529414).

Examples of suitable mineralocorticoid receptor antagonists for use in combination with the compounds of the present invention include spironolactone and eplerinone.

Examples of suitable phospodiesterase inhibitors for use in combination with the compounds of the present invention include PDEIII inhibitors such as cilostazol, and PDE V inhibitors such as sildenafil.

Examples of suitable thyroid mimetics for use in combination with the compounds of the present invention include thyrotropin, polythyroid, KB-130015, and dronedarone.

Examples of suitable anabolic agents for use in combination with the compounds of the present invention include testosterone, TRH diethylstilbesterol, estrogens, β-agonists, theophylline, anabolic steroids, dehydroepiandrosterone, enkephalins, E-series prostagladins, retinoic acid and compounds as disclosed in U.S. Pat. No. 3,239,345, e.g., Zeranol®; U.S. Patent No. 4,036,979, e.g., Sulbenox® or peptides as disclosed in U.S. Pat. No. 4,411,890.

Examples of suitable HIV or AIDS therapies for use in combination with the compounds of the present invention include indinavir sulfate, saquinavir, saquinavir mesylate, ritonavir, lamivudine, zidovudine, lamivudine/zidovudine combinations, zalcitabine, didanosine, stavudine, and megestrol acetate.

Examples of suitable therapies for treatment of Alzheimer's disease and cognitive disorders for use in combination with the compounds of the present invention include donepezil, tacrine, revastigmine, 5HT6, gamma secretase inhibitors, beta secretase inhibitors, SK channel blockers, Maxi-K blockers, and KCNQs blockers.

Examples of suitable therapies for treatment of sleeping disorders for use in combination with the compounds of the present invention include melatonin analogs, melatonin receptor antagonists, ML1B agonists, and GABA/NMDA receptor antagonists.

Examples of suitable anti-proliferative agents for use in combination with the compounds of the present invention include cyclosporin A, paclitaxel, FK 506, and adriamycin.

Examples of suitable anti-tumor agents for use in combination with the compounds of the present invention include paclitaxel, adriamycin, epothilones, cisplatin and carboplatin.

Compounds of the present invention can further be used in combination with nutritional supplements such as those described in U.S. 5,179,080, especially in combination with whey protein or casin, amino acids (such as leucine, branched amino acids and hydroxymethylbutyrate), triglycerides, vitamins (e.g., A, B6, B12, folate, C, D and E), minerals (e.g., selenium, magnesium, zinc, chromium, calcium and potassium), carnitine, lipoic acid, creatine, and coenzyme Q-10.

In addition, compounds of the present invention can be used in combination with therapeutic agents used in the treatment of sexual dysfunction, including but not limited to PDE5 inhibitors, such as sildenafil or IC-351; with an antiresorptive agent, hormone replacement therapies, vitamin D analogues, calcitonins, elemental calcium and calcium supplements, cathepsin K inhibitors, MMP inhibitors, vitronectin receptor antagonists, Src $SH_2$ antagonists, vacular -$H^+$-ATPase inhibitors, progesterone receptor agonists, ipriflavone, fluoride, RANK antagonists, PTH and its analogues and fragments, Tibolone, HMG-CoA reductase inhibitors, SERM's, p38 inhibitors, prostanoids, 17-beta hydroxysteroid dehydrogenase inhibitors and Src kinase inhibitors.

Compounds of the present invention can be used in combination with male contraceptives, such as nonoxynol 9 or therapeutic agents for the treatment of hair loss, such as minoxidil and finasteride or chemotherapeutic agents, such as with LHRH agonists.

For their preferred anticancer or antiangiogenic use, the compounds of the present invention can be administered either alone or in combination with other anti-cancer and cytotoxic agents and treatments useful in the treatment of cancer or other proliferative diseases, for example, where the second drug has the same or different mechanism of action than the present compounds of formula I. Examples of classes of anti-cancer and cytotoxic agents useful in combination with the present compounds include but are not limited to: alkylating agents such as nitrogen mustards, alkyl sulfonates, nitrosoureas, ethylenimines, and triazenes; EGFR inhibitors such as small molecule EGFR inhibitors, EGFR antibodies such as C225 (Erbitux); antimetabolites such as folate antagonists, purine analogues, and pyrimidine analogues; antibiotics such as anthracyclines, bleomycins, mitomycin, dactinomycin, and plicamycin; enzymes such as L-asparaginase; farnesyl-protein transferase inhibitors; 5α reductase inhibitors; inhibitors of 17β-hydroxy steroid dehydrogenase type 3 or type 1; hormonal agents such as glucocorticoids, estrogens/ antiestrogens, androgens/ antiandrogens, progestins, and luteinizing hormone-releasing hormone antagonists, octreotide acetate; microtubule-disruptor

agents, such as ecteinascidins or their analogs and derivatives; microtubule-stabilizing agents such as taxanes, for example, paclitaxel (Taxol®), docetaxel (Taxotere®), and their analogs, and epothilones, such as epothilones A-F and their analogs; plant-derived products, such as vinca alkaloids, epipodophyllotoxins, taxanes; and topiosomerase inhibitors; prenyl-protein transferase inhibitors; and miscellaneous agents such as hydroxyurea, procarbazine, mitotane, hexamethylmelamine, platinum coordination complexes such as cisplatin and carboplatin; and other agents used as anti-cancer and cytotoxic agents such as biological response modifiers, growth factors; immune modulators and monoclonal antibodies. The compounds of the invention may also be used in conjunction with radiation therapy.

Representative examples of these classes of anti-cancer and cytotoxic agents include but are not limited to mechlorethamine hydrochloride, cyclophosphamide, chlorambucil, melphalan, ifosfamide, busulfan, carmustin, lomustine, semustine, streptozocin, thiotepa, dacarbazine, methotrexate, thioguanine, mercaptopurine, fludarabine, pentastatin, cladribin, cytarabine, fluorouracil, doxorubicin hydrochloride, daunorubicin, idarubicin, bleomycin sulfate, mitomycin C, actinomycin D, safracins, saframycins, quinocarcins, discodermolides, vincristine, vinblastine, vinorelbine tartrate, etoposide, etoposide phosphate, teniposide; paclitaxel, tamoxifen, estramustine, estramustine phosphate sodium, flutamide, buserelin, leuprolide, pteridines, diyneses, levamisole, aflacon, interferon, interleukins, aldesleukin, filgrastim, sargramostim, rituximab, BCG, tretinoin, irinotecan hydrochloride, betamethosone, gemcitabine hydrochloride, altretamine, and topoteca and any analogs or derivatives thereof.

Preferred member of these classes include, but are not limited to, paclitaxel, cisplatin, carboplatin, doxorubicin, carminomycin, daunorubicin, aminopterin, methotrexate, methopterin, mitomycin C, ecteinascidin 743, or porfiromycin, 5-fluorouracil, 6-mercaptopurine, gemcitabine, cytosine arabinoside, podophyllotoxin or podophyllotoxin derivatives such as etoposide, etoposide phosphate or teniposide, melphalan, vinblastine, vincristine, leurosidine, vindesine and leurosine.

Examples of anticancer and other cytotoxic agents include the following: epothilone derivatives as found in German Patent No. 4138042.8; WO 97/19086, WO 98/22461, WO 98/25929, WO 98/38192, WO 99/01124, WO 99/02224, WO 99/02514, WO 99/03848, WO 99/07692, WO 99/27890, WO 99/28324, WO 99/43653, WO 99/54330, WO 99/54318, WO 99/54319, WO 99/65913, WO 99/67252, WO 99/67253 and WO 00/00485; cyclin dependent kinase inhibitors as found in WO 99/24416 (see also U.S. Patent No. 6,040,321); and prenyl-protein transferase inhibitors as found in WO 97/30992 and WO 98/54966; and agents such as those described generically and specifically in U.S. Patent No. 6,011,029 (the compounds of which U.S. Patent can be employed together with any NHR modulators (including, but not limited to, those of present invention) such as AR modulators, ER modulators, with LHRH modulators, or with surgical castration, especially in the treatment of cancer).

The combinations of the present invention can also be formulated or co-administered with other therapeutic agents that are selected for their particular usefulness in administering therapies associated with the aforementioned conditions. For example, the compounds of the invention may be formulated with agents to prevent nausea, hypersensitivity and gastric irritation, such as antiemetics, and $H_1$ and $H_2$ antihistaminics.

As it pertains to the treatment of cancer, the compounds of this invention are most preferably used alone or in combination with anti-cancer treatments such as radiation therapy and/or with cytostatic and/or cytotoxic agents, such as, but not limited to; DNA interactive agents, such as cisplatin or doxorubicin; inhibitors of farnesyl protein transferase, such as those described in U.S. Patent No. 6,011,029; topoisomerase II inhibitors, such as etoposide; topoisomerase I inhibitors, such as CPT-11 or topotecan; tubulin stabilizing agents, such as paclitaxel, docetaxel, other taxanes, or epothilones; hormonal agents, such as tamoxifen; thymidilate synthase inhibitors, such as 5-fluorouracil; antimetabolites, such as methoxtrexate; antiangiogenic agents, such as angiostatin, ZD6474, ZD6126 and comberstatin A2; kinase inhibitors, such as her2 specific antibodies, Iressa and CDK inhibitors; histone deacetylase inhibitors, such as CI-994 and MS-27-275. Such compounds may also be combined with agents which suppress the production of circulating testosterone such as LHRH agonists or antagonists or with surgical castration. Exemplary combination therapies (e.g., for the treatment of prostate cancer) for use with a compound of the present invention include an LHRH modulator or prednisone.

The present invention also contemplates kits, for example, for the treatment of prostate cancer, comprising a first container (such as a vial) containing a pharmaceutical formulation comprising a compound of the present invention, said compound optionally in a pharmaceutically acceptable carrier, and a second container (such as a vial) containing a pharmaceutical formulation comprising one or more agents (such as an LHRH modulator) to be used in combination with said compound of the present invention, said agent(s) optionally in a pharmaceutically acceptable carrier.

For example, known therapies for advanced metastatic prostate cancer include "complete androgen ablation therapy" wherein tumor growth is inhibited by controlling the supply of androgen to the prostate tissues via chemical castration (castration serves to inhibit the production of circulating testosterone (T) and dihydrotestosterone (DHT)) followed by the administration of androgen receptor (AR) antagonists (which inhibit the function T/DHT derived from the conversion of circulating androgen precursors to T/DHT by the prostate tissue). The compounds of the present invention can be employed as AR antagonists in complete ablation therapy, alone or in combination with other AR antagonists such as Flutamide, Casodex, Nilutamide, or Cyproterone acetate.

The present invention provides compounds which can be used to treat patients suffering from prostate cancer

resistant to androgen receptor antagonists which are not within formula I of the invention (or salts thereof), such as bicalutimide. The invention thus further contemplates a method of treating prostate cancer resistant to an androgen receptor antagonist other than those of formula I or salts thereof, comprising the step of administering to a patient in need thereof a compound capable of reducing the growth rate of the tumor mass of said cancer in an amount effective therefor. The term "reducing the growth rate of said tumor mass" denotes reduction in the growth rate (including, of course, stabilization or reduction in size) of said tumor mass upon treatment relative to the growth rate upon treatment with said androgen receptor antagonist other than those of formula I or salts thereof. Compounds of the formula I and pharmaceutically acceptable salts thereof of the present invention are preferred such compounds.

The present invention also contemplates use of an antiestrogen and/or aromatase inhibitor in combination with a compound of the present invention, for example, to assist in mitigating side effects associated with antiandrogen therapy such as gynecomastia. Exemplary antiestrogen and/or aromatase inhibitors include anastrozole (Arimidex), tamoxifen citrate (Nolvadex), exemestane (Aromasin), toremifene citrate (Fareston), letrozole (Femara), raloxifene hydrochloride (Evista), Faslodex, or 923 (Wyeth Ayerst).

The compounds of the present invention may be employed adjuvant to surgery.

Another application of the present compounds is in combination with antibody therapy such as but not limited to antibody therapy against PSCA. An additional application is in concert with vaccine / immune modulating agents for the treatment of cancer.

Compounds of the present invention can be employed in accordance with the methods described in U.S. Provisional Patent Application Serial No. 60/284,438, entitled "Selective Androgen Receptor Modulators and Methods for Their Identification, Design and Use" filed April 18, 2001 by Mark E. Salvati *et al.* (Attorney Docket No. LD0250(PSP)), which Provisional Patent Application is incorporated herein by reference in its entirety (including, but not limited to, reference to all specific compounds within formula I of the present invention), and U.S. Patent Application Serial No. 09/885,827, entitled "Selective Androgen Receptor Modulators and Methods for Their Identification, Design and Use" filed June 20, 2001 by Mark E. Salvati *et al.* (Attorney Docket No. LD0250(NP)), which Patent Application is incorporated herein by reference in its entirety (including, but not limited to, reference to all specific compounds within formula I of the present invention).

For racemates of compounds of the present invention, one enantiomer can, for example be a full AR antagonist while the other can be an AR antagonist in tumor tissue while having no activity or agonist activity in nontumor tissue containing the androgen receptor.

The above other therapeutic agents, when employed in combination with the compounds of the present invention, can be used, for example, in those amounts indicated in the Physicians' Desk Reference (PDR) or as otherwise determined by one of ordinary skill in the art.

The following assays can be employed in ascertaining the activity of a compound as a NHR modulator. Preferred are those compounds with an activity greater than 20$\mu$m for binding or transactivation in any of these assays. Various compounds of the present invention were determined to have AR modulator activity utilizing the transactivation assay, and standard AR binding assays as described following.

**<u>Transactivation Assays:</u>**

**AR Specific Assay:**

Compounds of the present invention were tested in transactivation assays of a transfected reporter construct and using the endogenous androgen receptor of the host cells. The transactivation assay provides a method for identifying functional agonists and partial agonists that mimic, or antagonists that inhibit, the effect of native hormones, in this case, dihydrotestosterone (DHT). This assay can be used to predict *in vivo* activity as there is a good correlation in both series of data. See, e.g. T. Berger et al., J. Steroid Biochem. Molec. Biol. 773 (1992), the disclosure of which is herein incorporated by reference.

For the transactivation assay a reporter plasmid is introduced by transfection (a procedure to induce cells to take foreign genes) into the respective cells. This reporter plasmid, comprising the cDNA for a reporter protein, such as secreted alkaline phosphatase (SEAP), controlled by prostate specific antigen (PSA) upstream sequences containing androgen response elements (AREs). This reporter plasmid functions as a reporter for the transcription-modulating activity of the AR. Thus, the reporter acts as a surrogate for the products (mRNA then protein) normally expressed by a gene under control of the AR and its native hormone. In order to detect antagonists, the transactivation assay is carried out in the presence of constant concentration of the natural AR hormone (DHT) known to induce a defined reporter signal. Increasing concentrations of a suspected antagonist will decrease the reporter signal (e.g., SEAP production). On the other hand, exposing the transfected cells to increasing concentrations of a suspected agonist will increase the production of the reporter signal.

For this assay, LNCaP and MDA 453 cells were obtained from the American Type Culture Collection (Rockville,

MD), and maintained in RPMI 1640 or DMEM medium supplemented with 10% fetal bovine serum (FBS; Gibco) respectively. The respective cells were transiently transfected by electroporation according to the optimized procedure described by Heiser, 130 Methods Mol. Biol., 117 (2000), with the pSEAP2/PSA540/Enhancer reporter plasmid. The reporter plasmid, was constructed as follows: commercial human placental genomic DNA was used to generate by Polymerase Cycle Reaction (PCR) a fragment containing the BglII site (position 5284) and the Hind III site at position 5831 of the human prostate specific antigen promoter (Accession # U37672), Schuur, et al., J. Biol. Chem., 271 (12): 7043-51 (1996). This fragment was subcloned into the pSEAP2/basic (Clontech) previously digested with BglII and HindIII to generate the pSEAP2/PSA540 construct. Then a fragment bearing the fragment of human PSA upstream sequence between positions -5322 and -3873 was amplified by PCR from human placental genomic DNA. A XhoI and a BglII sites were introduced with the primers. The resulting fragment was subcloned into pSEAP2/PSA540 digested with XhoI and BglII respectively, to generate the pSEAP2/PSA540/Enhancer construct. LNCaP and MDA 453 cells were collected in media containing 10% charcoal stripped FBS. Each cell suspension was distributed into two Gene Pulser Cuvetts (Bio-Rad) which then received 8 μg of the reporter construct, and electoporated using a Bio-Rad Gene Pulser at 210 volts and 960 μFaraday. Following the transfections the cells were washed and incubated with media containing charcoal stripped fetal bovine serum in the absence (blank) or presence (control) of 1 nM dihydrotestosterone (DHT; Sigma Chemical) and in the presence or absence of the standard anti-androgen bicalutamide or compounds of the present invention in concentrations ranging from 10-10 to 10-5 M (sample). Duplicates were used for each sample. The compound dilutions were performed on a Biomek 2000 laboratory workstation. After 48 hours, a fraction of the supernatant was assayed for SEAP activity using the Phospha-Light Chemiluminescent Reporter Gene Assay System (Tropix, Inc). Viability of the remaining cells was determined using the CellTiter 96 Aqueous NonRadioactive Cell Proliferation Assay (MTS Assay, Promega). Briefly, a mix of a tetrazolium compound (3-(4,5-dimethylthiazol-2-yl)-5-(3-carboxymethoxyphenyl)- 2-(4-sulfophenyl)-2H-tetrazolium, inner salt; MTS) and an electron coupling reagent (phenazine methosulfate; PMS) are added to the cells. MTS (Owen's reagent) is bioreduced by cells into a formazan that is soluble in tissue culture medium, and therefore its absorbance at 490nm can be measured directly from 96 well assay plates without additional processing. The quantity of formazan product as measured by the amount of 490nm absorbance is directly proportional to the number of living cells in culture. For each replicate the SEAP reading was normalized by the Abs490 value derived from the MTS assay. For the antagonist mode, the % Inhibition was calculated as:

$$\% \text{ Inhibition} = 100 \text{ x } ( 1 - [\text{average control} - \text{average blank} / \text{average sample} - \text{average blank} ])$$

Data was plotted and the concentration of compound that inhibited 50% of the normalized SEAP was quantified ($IC_{50}$).

For the agonist mode % Control was referred as the effect of the tested compound compared to the maximal effect observed with the natural hormone, in this case DHT, and was calculated as:

$$\% \text{ Control} = 100 \text{ x average sample} - \text{average blank/ average control} - \text{average blank}$$

Data was plotted and the concentration of compound that activates to levels 50% of the normalized SEAP for the control was quantified ($EC_{50}$).

**GR Specificity Assay:**

The reporter plasmid utilized was comprised of the cDNA for the reporter SEAP protein, as described for the AR specific transactivation assay. Expression of the reporter SEAP protein was controlled by the mouse mammary tumor virus long terminal repeat (MMTV LTR) sequences that contains three hormone response elements (HREs) that can be regulated by both GR and PR see, e.g. G. Chalepakis et al., Cell, 53(3), 371 (1988). This plasmid was transfected into A549 cells, which expresses endogenous GR, to obtain a GR specific transactivation assay. A549 cells were obtained from the American Type Culture Collection (Rockville, MD), and maintained in RPMI 1640 supplemented with 10% fetal bovine serum (FBS; Gibco). Determination of the GR specific antagonist activity of the compounds of the present invention was identical to that described for the AR specific transactivation assay, except that the DHT was replaced with 5 nM dexamethasone (Sigma Chemicals), a specific agonist for GR. Determination of the GR specific agonist activity of the

compounds of the present invention was performed as described for the AR transactivation assay, wherein one measures the activation of the GR specific reporter system by the addition of a test compound, in the absence of a known GR specific agonists ligand.

**PR Specific Assay:**

The reporter plasmid utilized was comprised of the cDNA for the reporter SEAP protein, as described for the AR specific transactivation assay. Expression of the reporter SEAP protein was controlled by the mouse mammary tumor virus long terminal repeat (MMTV LTR) sequences that contains three hormone response elements (HREs) that can be regulated by both GR and PR. This plasmid was transfected into T47D, which expresses endogenous PR, to obtain a PR specific transactivation assay. T47D cells were obtained from the American Type Culture Collection (Rockville, MD), and maintained in DMEM medium supplemented with 10% fetal bovine serum (FBS; Gibco). Determination of the PR specific antagonist activity of the compounds of the present invention was identical to that described for the AR specific transactivation assay, except that the DHT was replaced with 1 nM Promegastone (NEN), a specific agonist for PR. Determination of the PR specific agonist activity of the compounds of the present invention was performed as described for the AR transactivation assay, wherein one measures the activation of the PR specific reporter system by the addition of a test compound, in the absence of a known PR specific agonists ligand.

**AR Binding Assay:**

For the whole cell binding assay, human LNCaP cells (T877A mutant AR) or MDA 453 (wild type AR) in 96-well microtiter plates containing RPMI 1640 or DMEM supplemented with 10% charcoal stripped CA-FBS (Cocaleco Biologicals) respectively, were incubated at 37°C to remove any endogenous ligand that might be complexed with the receptor in the cells. After 48 hours, either a saturation analysis to determine the $K_d$ for tritiated dihydrotestosterone, [$^3$H]-DHT, or a competitive binding assay to evaluate the ability of test compounds to compete with [$^3$H]-DHT were performed. For the saturation analysis, media (RPMI 1640 or DMEM - 0.2% CA-FBS) containing [$^3$H]-DHT (in concentrations ranging from 0.1 nM to 16 nM) in the absence (total binding) or presence (non-specific binding) of a 500-fold molar excess of unlabeled DHT were added to the cells. After 4 hours at 37°C, an aliquot of the total binding media at each concentration of [$^3$H]-DHT was removed to estimate the amount of free [$^3$H]-DHT. The remaining media was removed, cells were washed three times with PBS and harvested onto UniFilter GF/B plates (Packard), Microscint (Packard) was added and plates counted in a Top-Counter (Packard) to evaluate the amount of bound [$^3$H]-DHT.

For the saturation analysis, the difference between the total binding and the non-specific binding, was defined as specific binding. The specific binding was evaluated by Scatchard analysis to determine the $K_d$ for [$^3$H]-DHT. See e.g. D. Rodbard, Mathematics and statistics of ligand assays: an illustrated guide: In: J. Langon and J. J. Clapp, eds., Ligand Assay, Masson Publishing U.S.A., Inc., New York, pp. 45-99, (1981), the disclosure of which is herein incorporated by reference.

For the competition studies, media containing 1 nM [$^3$H]-DHT and compounds of the invention ("test compounds") in concentrations ranging from $10^{-10}$ to $10^{-5}$ M were added to the cells. Two replicates were used for each sample. After 4 hours at 37°C, cells were washed, harvested and counted as described above. The data was plotted as the amount of [$^3$H]-DHT (% of control in the absence of test compound) remaining over the range of the dose response curve for a given compound. The concentration of test compound that inhibited 50% of the amount of [$^3$H]-DHT bound in the absence of competing ligand was quantified ($IC_{50}$) after log-logit transformation. The $K_I$ values were determined by application of the Cheng-Prusoff equation to the $IC_{50}$ values, where:

$$K_I = \frac{IC_{50}}{(1 + (^3\text{H-DHT}) / K_d \text{ for } ^3\text{H-DHT})}.$$

After correcting for non-specific binding, $IC_{50}$ values were determined. The $IC_{50}$ is defined as the concentration of competing ligand needed to reduce specific binding by 50%. The $K_d$s for [$^3$H]-DHT for MDA 453 and LNCaP were 0.7 and 0.2 nM respectively.

**Human Prostate Cell Proliferation Assay:**

Compounds of the present invention were tested ("test compounds") on the proliferation of human prostate cancer cell lines. For that, MDA PCa2b cells, a cell line derived from the metastasis of a patient that failed castration, Navone et al., Clin. Cancer Res., 3, 2493-500 (1997), were incubated with or without the test compounds for 72 hours

and the amount of [$^3$H]-thymidine incorporated into DNA was quantified as a way to assess number of cells and therefore proliferation. The MDA PCa2b cell line was maintained in BRFF-HPC1 media (Biological Research Faculty & Facility Inc., MD) supplemented with 10% FBS. For the assay, cells were plated in Biocoated 96-well microplates and incubated at 37°C in 10% FBS (charcoal-stripped)/BRFF-BMZERO (without androgens). After 24 hours, the cells were treated in the absence (blank) or presence of 1 nM DHT (control) or with test compounds (sample) of the present invention in concentrations ranging from $10^{-10}$ to $10^{-5}$ M. Duplicates were used for each sample. The compound dilutions were performed on a Biomek 2000 laboratory work station. Seventy two hours later 0.44 uCi. of [$^3$H]-Thymidine (Amersham) was added per well and incubated for another 24 h followed by tripsinization, harvesting of the cells onto GF/B filters. Micro-scint PS were added to the filters before counting them on a Beckman TopCount.

The % Inhibition was calculated as:

$$\% \text{ Inhibition} = 100 \text{ x } ( 1 - [\text{average}_{\text{control}} - \text{average}_{\text{blank}} / \text{average}_{\text{sample}} - \text{average}_{\text{blank}}])$$

Data was plotted and the concentration of compound that inhibited 50% of the [$^3$H]-Thymidine incorporation was quantified ($IC_{50}$).

<u>**C2C12 Mouse Myoblast Transactivation Assay:**</u>

Two functional transactivation assays were developed to assess the efficacy of androgen agonists in a muscle cell background using a luciferase reporter. The first assay (ARTA Stable 1) uses a cell line, Stable 1 (clone #72), which stably expresses the full length rat androgen receptor but requires the transient transfection of an enhancer/reporter. This cell line was derived from C2C12 mouse moyoblast cells. The second assay (ARTA Stable 2) uses a cell line, Stable 2 (clone #133), derived from Stable 1 which stably expresses both rAR and the enhancer/luciferase reporter.

The enhancer/reporter construct used in this system is pGL3/2XDR-1/luciferase. 2XDR-1 was reported to be an AR specific response element in CV-1 cells, Brown et. al. The Journal of Biological Chemisty 272, 8227-8235, (1997). It was developed by random mutagenesis of an AR/GR consensus enhancer sequence.

**ARTA Stable 1:**

1. Stable 1 cells are plated in 96 well format at 6,000 cells/well in high glucose DMEM without phenol red (Gibco BRL, Cat. No.: 21063-029) containing 10% charcoal and dextran treated FBS (HyClone Cat. No.: SH30068.02), 50 mM HEPES Buffer (Gibco BRL, Cat. No.: 15630-080), 1X MEM Na Pyruvate (Gibco BRL, Cat. No.: 11360-070), 0.5X Antibiotic-Antimycotic, and 800 μg/ml Geneticin (Gibco BRL, Cat. No.: 10131-035).
2. 48 hours later, cells are transfected with pGL3/2XDR-1/luciferase using LipofectAMINE Plus™ Reagent (Gibco BRL, Cat. No.: 10964-013). Specifically, 5 ng/well pGL3/2XDR-1/luciferase DNA and 50 ng/well Salmon Sperm DNA (as carrier) are diluted with 5 μl/well Opti-MEMem media (Gibco BRL, Cat. No.: 31985-070). To this, 0.5 μl/well Plus reagent is added. This mixture is incubated for 15 minutes at room temperature. In a separate vessel, 0.385 μl/well LipofectAMINE reagent is diluted with 5 μl/well Opti-MEM. The DNA mixture is then combined with the LipofectAMINE mixture and incubated for an additional 15 minutes at room temperature. During this time, the media from the cells is removed and replaced with 60 μl/well of Opti-MEM. To this is added 10 μl/well of the DNA/LipofectAMINE transfection mixture. The cells are incubated for 4 hours.
3. The transfection mixture is removed from the cells and replaced with 90 μl of media as in #1 above.
4. 10 μl/well of appropriate drug dilution is placed in each well.
5. 24 hours later, the Steady-Glo™ Luciferase Assay System is used to detect activity according to the manufacturer's instructions (Promega, Cat. No.: E2520).

**ARTA stable 2**

1. Stable 2 cells are plated in 96 well format at 6,000 cells/well in high glucose DMEM without phenol red (Gibco BRL, Cat. No.: 21063-029) containing 10% charcoal and dextran treated FBS (HyClone Cat. No.: SH30068.02), 50 mM HEPES Buffer (Gibco BRL, Cat. No.: 15630-080), 1X MEM Na Pyruvate (Gibco BRL, Cat. No.: 11360-070), 0.5X Antibiotic-Antimycotic, 800 μLg/ml Geneticin (Gibco BRL, Cat. No.: 10131-035) and 800 μg/ml Hygromycin β

(Gibco BRL, Cat. No.: 10687-010).

2. 48 hours later, the media on the cells is removed and replaced with 90 μl fresh. 10 μl/well of appropriate drug dilution is placed in each well.

3. 24 hours later, the Steady-GloTM Luciferase Assay System is used to detect activity according to the manufacturer's instructions (Promega, Cat. No.: E2520).

See U.S. Patent Application Serial No. 09/885,831, entitled "Cell Lines and Cell-BasedAssays for Identification of Androgen Receptor Modulators" filed June 20, 2001 by Jacek Ostrowski *et al.* (Attorney Docket No. D0177), which Patent Application is incorporated herein by reference in its entirety.

**Proliferation Assays**

**Murine Breast Cell Proliferation Assay:**

The ability of compounds of the present invention ("test compounds") to modulate the function of the AR was determined by testing said compounds in a proliferation assay using the androgen responsive murine breast cell line derived from the Shionogi tumor, Hiraoka et al., Cancer Res., 47, 6560-6564 (1987). Stable AR dependent clones of the parental Shionogi line were established by passing tumor fragments under the general procedures originally described in Tetuo, et. al., Cancer Research 25, 1168-1175 (1965). From the above procedure, one stable line, SC114, was isolated, characterized and utilized for the testing of example compounds. SC114 cells were incubated with or without the test compounds for 72 hours and the amount of [3H]-thymidine incorporated into DNA was quantified as a surrogate endpoint to assess the number of cells and therefore the proliferation rate as described in Suzuki et. al., J. Steroid Biochem. Mol. Biol. 37, 559-567 (1990). The SC114 cell line was maintained in MEM containing $10^{-8}$ M testosterone and 2% DCC-treated FCS. For the assay, cells were plated in 96-well microplates in the maintenance media and incubated at 37°C. On the following day, the medium was changed to serum free medium [Ham's F-12:MEM (1;1, v/v) containing 0.1% BSA] with (antagonist mode) or without (agonist mode) $10^{-8}$ M testosterone and the test compounds of the present invention in concentrations ranging from $10^{-10}$ to $10^{-5}$ M. Duplicates were used for each sample. The compound dilutions were performed on a Biomek 2000 laboratory work station. Seventy two hours later 0.44uCi of [3H]-Thymidine (Amersham) was added per well and incubated for another 2 hr followed by tripsinization, and harvesting of the cells onto GFB filters. Micro-scint PS were added to the filters before counting them on a Beckman TopCount.

For the antagonist mode, the % Inhibition was calculated as:

$$\% \text{ Inhibition} = 100 \text{ x } ( 1 - [\text{average}_{\text{sample}} - \text{average}_{\text{blank}} / \text{average}_{\text{control}} - \text{average}_{\text{blank}} ])$$

Data was plotted and the concentration of compound that inhibited 50% of the [$^3$H]-Thymidine incorporation was quantified ($IC_{50}$).

For the agonist mode % Control was referred as the effect of the tested compound compared to the maximal effect observed with the natural hormone, in this case DHT, and was calculated as:

$$\% \text{ Control} = 100 \text{ x } (\text{average}_{\text{sample}} - \text{average}_{\text{blank}}) / (\text{average}_{\text{control}} - \text{average}_{\text{blank}})$$

Data was plotted and the concentration of compound that inhibited 50% of the [$^3$H]-Thymidine incorporation was quantified ($EC_{50}$).

***In Vitro* Assay to Measure GR Induced AP-1 Transrepression:**

The AP-1 assay is a cell based luciferase reporter assay. A549 cells, which contain endogenous glucocorticoid receptor, were stably transfected with an AP-1 DNA binding site attached to the luciferase gene. Cells are then grown in RPMI + 10% fetal calf serum (charcoal-treated) + Penicillin/Streptomycin with 0.5mg/ml geneticin. Cells are plated the day before the assay at approximately 40000 cells/well. On assay day, the media is removed by aspiration and 20

μl assay buffer (RPMI without phenol red + 10% FCS (charcoal-treated) + Pen/Strep) is added to each well. At this point either 20 μl assay buffer (control experiments), the compounds of the present invention ("test compounds") (dissolved in DMSO and added at varying concentrations) or dexamethasome (100 nM in DMSO, positive control) are added to each well. The plates are then pre-incubated for 15 minutes at 37°C, followed by stimulation of the cells with 10 ng/ml PMA. The plates are then incubated for 7 hrs at 37°C after which 40 μl luciferase substrate reagent is added to each well. Activity is measured by analysis in a luminometer as compared to control experiments treated with buffer or dexamethasome. Activity is designated as % inhibition of the reporter system as compared to the buffer control with 10 ng/ml PMA alone. The control, dexamethasone, at a concentration of ≤10 μM typically suppresses activity by 65%. Test compounds which demonstrate an inhibition of PMA induction of 50% or greater at a concentration of test compound of ≤10 μM are deemed active.

**Wet Prostate Weight Assay AR Antagonist Assay:**

The activity of compounds of the present invention as AR antagonists was investigated in an immature male rat model, a standard, recognized test of antiandrogen activity of a given compound, as described in L. G. Hershberger et al., Proc. Soc. Expt. Biol. Med., 83, 175 (1953); P. C. Walsh and R. F. Gittes, "Inhibition of extratesticular stimuli to prostate growth in the castrated rat by antiandrogens", Endocrinology, 86, 624 (1970); and B. J. Furr et al., "ICI 176,334: A novel non-steroid, peripherally selective antiandrogen", J. Endocrinol., 113, R7-9 (1987), the disclosures of which are herein incorporated by reference.

The basis of this assay is the fact that male sexual accessory organs, such as the prostate and seminal vesicles, play an important role in reproductive function. These glands are stimulated to grow and are maintained in size and secretory function by the continued presence of serum testosterone (T), which is the major serum androgen (>95%) produced by the Leydig cells in the testis under the control of the pituitary luteinizing hormone (LH) and follicle stimulating hormone (FSH). Testosterone is converted to the more active form, dihydrotestosterone, (DHT), within the prostate by 5α-reductase. Adrenal androgens also contribute about 20% of total DHT in the rat prostate, compared to 40% of that in 65-year-old men. F. Labrie et al. Clin. Invest. Med.,16, 475-492 (1993). However, this is not a major pathway, since in both animals and humans, castration leads to almost complete involution of the prostate and seminal vesicles without concomitant adrenalectomy. Therefore, under normal conditions, the adrenals do not support significant growth of prostate tissues. M. C. Luke and D. S. Coffey, "The Physiology of Reproduction" ed. By E. Knobil and J. D. Neill,1, 1435-1487 (1994). Since the male sex organs are the tissues most responsive to modulation of the androgen activity, this model is used to determine the androgen dependent growth of the sex accessory organs in immature castrated rats.

Male immature rats (19-20 days old Sprague-Dawley, Harlan Sprague-Dawely) were castrated under metofane ansestesia. Five days after surgery these castrated rats (60-70g, 23-25 day-old) were dosed for 3 days. Animals were dosed sub-cutaneously (s.c.) 1mg/kg with Testosterone Proprionate (TP) in arachis oil vehicle and anti-androgen test compounds (compounds of the present invention) were dosed orally by gavage (p.o.) in dissolved/suspensions of 80% PEG 400 and 20% Tween 80 (PEGTW). Animals were dosed (v/w) at 0.5 ml of vehicle /100g body weight. Experimental groups were as follows:

1. Control vehicle
2. Testosterone Propionate (TP) (3 mg/rat/day, subcutaneous)
3. TP plus Casodex (administered p.o. in PEGTW, QD) , a recognized antiandrogen, as a reference compound.
4. To demonstrate antagonist activity; a compound of the present invention ("test compound") was administered (p.o. in PEGTW, QD) with TP (s.c. as administered in group 2) in a range of doses.
5. To demonstrate agonist activity a compound of the present invention ("test compound") was administered alone (p.o.. in PEGTW, QD) in a range of doses.

At the end of the 3-day treatment, the animals were sacrificed, and the ventral prostate weighed. To compare data from different experiments, the sexual organs weights were first standardized as mg per 100 g of body weight, and the increase in organ weight induced by TP was considered as the maximum increase (100%). ANOVA followed by one-tailed Student or Fischer's exact test was used for statistical analysis.

The gain and loss of sexual organ weight reflect the changes of the cell number (DNA content) and cell mass (protein content), depending upon the serum androgen concentration. See Y. Okuda et al., J. Urol., 145, 188-191 (1991), the disclosure of which is herein incorporated by reference. Therefore, measurement of organ wet weight is sufficient to indicate the bioactivity of androgens and androgen antagonist. In immature castrated rats, replacement of exogenous androgens increases seminal vesicles (SV) and the ventral prostate (VP) in a dose dependent manner.

The maximum increase in organ weight was 4 to 5-fold when dosing 3 mg/rat/day of testosterone (T) or 1 mg/rat/day of testosterone propionate (TP) for 3 days. The $EC_{50}$ of T and TP were about 1 mg and 0.03 mg, respectively. The increase in the weight of the VP and SV also correlated with the increase in the serum T and DHT concentration.

Although administration of T showed 5-times higher serum concentrations of T and DHT at 2 hours after subcutaneous injection than that of TP, thereafter, these high levels declined very rapidly. In contrast, the serum concentrations of T and DHT in TP-treated animals were fairly consistent during the 24 hours, and therefore, TP showed about 10-30-fold higher potency than free T.

In this immature castrated rat model, a known AR antagonist (Casodex) was also administered simultaneously with 0.1 mg of TP ($ED_{80}$), inhibiting the testosterone-mediated increase in the weights of the VP and SV in a dose dependent manner. The antagonist effects were similar when dosing orally or subcutaneously. Compounds of the invention also exhibited AR antagonist activity by suppressing the testosterone-mediated increase in the weights of VP and SV.

**Levator Ani & Wet Prostate Weight Assay AR Agonist Assay:**

The activity of compounds of the present invention as AR agonists was investigated in an immature male rat model, a recognized test of anabolic effects in muscle and sustaining effects in sex organs for a given compound, as described in L. G. Hershberger et al., Proc. Soc. Expt. Biol. Med., 83, 175 (1953); B. L. Beyler et al, "Methods for evaluating anabolic and catabolic agents in laboratory animals", J. Amer. Med. Women's Ass., 23, 708 (1968); H. Fukuda et al., "Investigations of the levator ani muscle as an anabolic steroid assay", Nago Dai. Yak. Ken. Nem. 14, 84 (1966) the disclosures of which are herein incorporated by reference.

The basis of this assay lies in the well-defined action of androgenic agents on the maintenance and growth of muscle tissues and sexual accessory organs in animals and man. Androgenic steroids, such as testosterone (T), have been well characterized for their ability to maintain muscle mass. Treatment of animals or humans after castrations with an exogenous source of T results in a reversal of muscular atrophy. The effects of T on muscular atrophy in the rat levator ani muscle have been well characterized. M. Masuoka et al., "Constant cell population in normal, testosterone deprived and testosterone stimulated levator ani muscles" Am. J. Anat. 119, 263 (1966); Z. Gori et al., "Testosterone hypertrophy of levator ani muscle of castrated rats. I. Quantitative data" Boll. -Soc. Ital. Biol. Sper. 42, 1596 (1966); Z. Gori et al., "Testosterone hypertrophy of levator ani muscle of castrated rats. II. Electron-microscopic observations" Boll. -Soc. Ital. Biol. Sper. 42, 1600 (1966); A. Boris et al., Steroids 15, 61 (1970). As described above, the effects of androgens on maintenance of male sexual accessory organs, such as the prostate and seminal vesicles, is well described. Castration results in rapid involution and atrophy of the prostate and seminal vesicles. This effect can be reversed by exogenous addition of androgens. Since both the levator ani muscle and the male sex organs are the tissues most responsive to the effects of androgenic agents, this model is used to determine the androgen dependent reversal of atrophy in the levator ani muscle and the sex accessory organs in immature castrated rats. Sexually mature rats (200-250 g, 6-8 weeks-old, Sprague-Dawley, Harlan) were acquired castrated from the vendor (Taconic). The rats were divided into groups and treated daily for 7 to 14 days with one of the following:

1. Control vehicle
2. Testosterone Propionate (TP) (3 mg/rat/day, subcutaneous)
3. TP plus Casodex (administered p.o. in PEGTW, QD) , a recognized antiandrogen, as a reference compound.
4. To demonstrate antagonist activity, a compound of the present invention ("test compound") was administered (p.o. in PEGTW, QD) with TP (s.c. as administered in group 2) in a range of doses.
5. To demonstrate agonist activity a compound of the present invention ("test compound") was administered alone (p.o. in PEGTW, QD) in a range of doses.

At the end of the 7-14-day treatment, the animals were sacrificed by carbon dioxide, and the levator ani, seminal vesicle and ventral prostate weighed. To compare data from different experiments, the levator ani muscle and sexual organ weights were first standardized as mg per 100 g of body weight, and the increase in organ weight induced by TP was considered as the maximum increase (100%). Super-anova (one factor) was used for statistical analysis.

The gain and loss of sexual organ weight reflect the changes of the cell number (DNA content) and cell mass (protein content), depending upon the serum androgen concentration: See Y. Okuda et al., J. Urol., 145, 188-191 (1991), the disclosure of which is herein incorporated by reference. Therefore, measurement of organ wet weight is sufficient to indicate the bioactivity of androgens and androgen antagonist. In immature castrated rats, replacement of exogenous androgens increases levator ani, seminal vesicles (SV) and prostate in a dose dependent manner.

The maximum increase in organ weight was 4 to 5-fold when dosing 3 mg/rat/day of testosterone (T) or 1 mg/rat/day of testosterone propionate (TP) for 3 days. The $EC_{50}$ of T and TP were about 1 mg and 0.03 mg, respectively. The increase in the weight of the VP and SV also correlated with the increase in the serum T and DHT concentration. Although administration of T showed 5-times higher serum concentrations of T and DHT at 2 hours after subcutaneous injection than that of TP, thereafter, these high levels declined very rapidly. In contrast, the serum concentrations of T and DHT in TP-treated animals were fairly consistent during the 24 hours, and therefore, TP showed about 10-30-fold

higher potency than free T.

**MDA PCa2b Human Prostate Zenograft Assay:**

In Vivo Antitumor Testing: MDA-PCa-2b human prostate tumors were maintained in Balb/c nu/nu nude mice. Tumors were propagated as subcutaneous transplants in adult male nude mice (4-6 weeks old) using tumor fragments obtained from donor mice. Tumor passage occurred every 5-6 weeks.

For antitumor efficacy trial, the required number of animals needed to detect a meaningful response were pooled at the start of the experiment and each was given a subcutaneous implant of a tumor fragment (~50 mg) with a 13-gauge trocar. Tumors were allowed to grow to approx. 100-200 mg (tumors outside the range were excluded) and animals were evenly distributed to various treatment and control groups. Treatment of each animal was based on individual body weight. Treated animals were checked daily for treatment related toxicity/mortality. Each group of animals was weighed before the initiation of treatment (Wt1) and then again following the last treatment dose (Wt2). The difference in body weight (Wt2-Wt1) provides a measure of treatment-related toxicity.

Tumor response was determined by measurement of tumors with a caliper twice a week, until the tumors reach a predetermined "target" size of 0.5 gm. Tumor weights (mg) were estimated from the formula: Tumor weight = (length x width2) ÷ 2

Tumor response end-point was expressed in terms of tumor growth inhibition (%T/C), defined as the ratio of median tumor weights of the treated tumors (T) to that of the control group (C).

To estimate tumor cell kill, the tumor volume doubling time was first calculated with the formula:

TVDT = Median time (days) for control tumors to reach target size –

Median time (days) for control tumors to reach half the target size s

And, Log cell kill = (T-C) ÷ (3.32 x TVDT)

Statistical evaluations of data were performed using Gehan's generalized Wilcoxon test.

**Dunning Prostate Tumor:**

Dunning R3327H prostate tumor is a spontaneously derived, well differentiated androgen responsive adenocarcinoma of the prostate (Smolev JK, Heston WD, Scott WW, and Coffey DS, Cancer Treat Rep. 61, 273-287 (1977)). The growth of the R3327H subline has been selected for its highly androgen-dependent and reproducible growth in intact male rats. Therefore, this model and other sublines of this tumor have been widely used to evaluate *in vivo* antitumor activities of antiandrogens such as flutamide and bacilutamide/Casodex (Maucher A., and von Angerer, J. Cancer Res. Clin. Oncol., 119, 669-674 (1993), Furr B.J.A. Euro. URL. 18 (suppl. 3), 2-9 (1990), Shain S.A. and Huot RI. J. Steriod Biochem. 31, 711-718 (1988)).

At the beginning of the study, the Dunning tumor pieces (about 4 x 4 mm) are transplanted subcutaneously to the flank of mature male Copenhagen rats (6-7 weeks old, Harlan-Sprague Dawley, Indianapolis, MD). About 6 weeks after the implantation, the animals with tumors of measurable size (about 80 - 120 $mm^2$) are randomized into treatment groups (8-10 rats/group) and the treatments are initiated. One group of the rats are castrated to serve as the negative control of tumor growth. Animals are treated daily with compounds of the current invention, standard antiandrogens such as bacilutamide or vehicle (control) for an average of 10 to 14 weeks. Test compounds are dissolved in a vehicle of (2.5 ml/kg of body weight) 10% polyethylene glycol and 0.05% Tween-80 in 1% carboxymethyl cellulose, PEG/CMC, (Sigma, St Louis, MO). Typical therapeutic experiments would include three groups of three escalating doses for each standard or test compound (in a range of 300-3 mg/kg).

Tumors in the vehicle (control) group reach a size of 1500 to 2500 $mm^3$, whereas the castrated animal group typically shows tumor stasis over the 14 weeks of observation. Animals treated orally with 20 mg/kg of bicalutamide or flutamide would be expected to show a 40% reduction in tumor volumes compared to control after 14 weeks of treatment. The size of tumors are measured weekly by vernier caliper (Froboz, Switzerland), taking perpendicular measurements of length and width. Tumor volumes are measured in $mm^3$ using the formula: Length x Width x Height = Volume. Statistical differences between treatment groups and control are evaluated using multiple ANOVA analysis followed by one tail non-parametric Student t test.

**Mature Rat Prostate Weight Assay:**

The activity of compounds of the present invention were investigated in a mature male rat model, which is a variation of the Levator ani & wet prostate weight assay described above. The above *in vivo* assays are recognized assays for determining the anabolic effects in muscle and sustaining effects in sex organs for a given compound, as described in L. G. Hershberger et al., 83 Proc. Soc. Expt. Biol. Med., 175 (1953); B. L. Beyler et al, "Methods for evaluating anabolic and catabolic agents in laboratory animals", 23 J. Amer. Med. Women's Ass., 708 (1968); H. Fukuda et al., "Investigations of the levator ani muscle as an anabolic steroid assay", 14 Nago Dai. Yak. Ken. Nem. 84 (1966) the disclosures of which are herein incorporated by reference. The basis of this assay lies in the well-defined action of androgenic agents on the maintenance and growth of muscle tissues and sexual accessory organs in animals and man.

The male sexual accessory organs, such as the prostate and seminal vesicles, play an important role in reproductive function. These glands are stimulated to grow and are maintained in size and secretory function by the continued presence of serum testosterone (T), which is the major serum androgen (>95%) produced by the Leydig cells in the testis under the control of the pituitary luteinizing hormone (LH) and follicle stimulating hormone (FSH). Testosterone is converted to the more active form, dihydrotestosterone, (DHT), within the prostate by 5$\alpha$-reductase. Adrenal androgens also contribute about 20% of total DHT in the rat prostate, compared to 40% of that in 65-year-old men. F. Labrie et. al. 16 Clin. Invest. Med., 475-492 (1993). However, this is not a major pathway, since in both animals and humans, castration leads to almost complete involution of the prostate and seminal vesicles without concomitant adrenalectomy. Therefore, under normal conditions, the adrenals do not support significant growth of prostate tissues, M. C. Luke and D. S. Coffey, "The Physiology of Reproduction" ed. By E. Knobil and J. D. Neill, 1, 1435-1487 (1994). Since the male sex organs and the levator ani are the tissues most responsive to modulation of the androgen activity, this model is used to determine the activity of compounds that modulate the androgen receptor pathway in mature rats.

Along with its mitogenic activity on tissues such as prostate, seminal vesicle and muscle, testosterone also serves as a negative regulator for its own biosynthesis. Testosterone production in the Leydig cells of the testis is controlled by the level of circulating LH released from the pituitary gland. LH levels are themselves controlled by the level of LHRH produced in the hypothalmic region. Testosterone levels in the blood serve to inhibit the secretion of LHRH and subsequently reduce levels of LH and ultimately the levels of circulating testosterone levels. By measuring blood levels of LH as they are effected by compounds of the present invention ("test compounds"), it is possible to determine the level of agonist or antagonist activity of said compounds at the hypothalamic axis of this endocrine cycle.

Matched sets of Harlan Sprague-Dawely rats (40-42 days old, 180-220 g), were dosed orally by gavage (p.o.) with the test compounds in dissolved/suspensions of 80% PEG 400 and 20% Tween 20 (PEGTW) for 14 days. Two control groups, one intact and one castrated were dose orally only with the PEGTW vehicle. Animals were dosed (v/w) at 0.5 ml of vehicle /100g body weight. Experimental groups were as follows:

1. Intact vehicle (p.o., PEGTW, QD)
2. Control vehicle (p.o., PEGTW, QD)
3. Bicalutamide (Casodex, a recognized antiandrogen, as a reference compound) or a compound of the present invention, p.o. in PEGTW QD. (in a range of doses).

At the end of the 14-day treatment, the animals were sacrificed, and the ventral prostate, the seminal vesicles, and the levator ani were removed surgically and weighed. To compare data from different experiments, the organs weights were first standardized as mg per 100 g of body weight, and expressed as a percentage of the value of the respective organ in the intact group.

Rat luteinizing hormone (rLH) is quantitatively determined with the Biotrak [125 I] kit (Amersham Pharmacia Biotek), following the manufacturer directions. The assay is based on the competition by the LH present in the serum of the binding of [$^{125}$I] rLH to an Amerlex-M bead/antibody suspension. The radioactivity that remains after incubation with the serum and subsequent washes is extrapolated into a standard curve to obtain a reading in ng/ml.

The gain and loss of sexual organ and levator ani weight reflect the changes of the cell number (DNA content) and cell mass (protein content), depending upon the serum androgen concentration, see Y. Okuda et al., J. Urol., 145, 188-191 (1991), the disclosure of which in herein incorporated by reference. Therefore, measurement of organ wet weight is sufficient to indicate the bioactivity of androgens and androgen antagonist. In the mature rats assay, active agonist agents will have no effect or will increase the weight of one or more of the androgen responsive organs (levator ani, prostate, seminal vessicle) and will have no effect or a suppressive effect on LH secretion. Compounds with antagonist activity will decrease the weight of one or more of the androgen responsive organs (levator ani, prostate, seminal vesicle) and will have no effect or a reduced suppressive effect on LH secretion.

**CWR22 Human Prostate Zenograft Assay:**

*In Vivo* Antitumor Testing: CWR22 human prostate tumors were maintained in Balb/c nu/nu nude mice. Tumors were propagated as subcutaneous transplants in adult male nude mice (4-6 weeks old) using tumor fragments obtained from donor mice. Tumor passage occurred every 5-6 weeks.

For antitumor efficacy trial, the required number of animals needed to detect a meaningful response were pooled at the start of the experiment and each was given a subcutaneous implant of a tumor fragment (~50 mg) with a 13-gauge trocar. Tumors were allowed to grow to approx. 100-200 mg (tumors outside the range were excluded) and animals were evenly distributed to various treatment and control groups. Treatment of each animal was based on individual body weight. Treated animals were checked daily for treatment related toxicity/mortality. Each group of animals was weighed before the initiation of treatment (Wt1) and then again following the last treatment dose (Wt2). The difference in body weight (Wt2-Wt1) provides a measure of treatment-related toxicity.

Tumor response was determined by measurement of tumors with a caliper twice a week, until the tumors reach a predetermined "target" size of 0.5 gm. Tumor weights (mg) were estimated from the formula: Tumor weight = (length × width2) ÷ 2.

Tumor response end-point was expressed in terms of tumor growth inhibition (%T/C), defined as the ratio of median tumor weights of the treated tumors (T) to that of the control group (C).

To estimate tumor cell kill, the tumor volume doubling time was first calculated with the formula:

$$\text{TVDT} = \text{Median time (days) for control tumors to reach target size} - \text{Median time (days) for control tumors to reach half the target size}$$

$$\text{And, Log cell kill} = (\text{T-C}) \div (3.32 \times \text{TVDT})$$

Statistical evaluations of data were performed using Gehan's generalized Wilcoxon test.

The following Examples illustrate embodiments of the present invention, and are not intended to limit the scope of the claims. Within certain Examples, one compound of the formula I is prepared and then employed to further prepare one or more additional compounds of the formula I or salts thereof. Methods employed to prepare one compound of the formula I or salt thereof as described herein can be employed as appropriate to prepare other compounds of the invention.

**Abbreviations**

The following abbreviations are used herein:

DBU = 1,8-diazabicyclo[5.4.0]undec-7-ene
4-DMAP = 4-dimethylaminopyridine
ee = enantiomeric excess
DMF = dimethylformamide
EtOAc = ethyl acetate
LDA = lithium diisopropylamide
Hünig's Base = N,N-diisopropylethylamine
Me = methyl
RT = retention time
TFA = trifluoroacetic acid
THF = tetrahydrofuran
TLC = thin layer chromatography
TMS = trimethylsilyl
pTSA = para-toluenesulfonic acid
Δ = heat
*t*-Bu = *tert*-butyl
$PhCH_3$ = toluene
Pd/C = palladium on activated charcoal
TsCl = tosyl chloride
TBSOTf = *tert*-butyldimethylsilyl trifluoromethane sulfonate
TBS = *tert*-butyldimethylsilane
MeI = methyl iodide

(BOC)$_2$O = di-*tert*-butyl dicarbonate
TEA = triethylamine
*n*-BuLi = *n*-butyllithium
rt = room temperature
LC = liquid chromatography
Ts = tosyl
Ph = phenyl
EtOH = ethanol
DCE = dichloroethane
DMSO = dimethylsulfoxide
Ra-Ni = Raney Nickel
MS = molecular sieves
MS(ES) = Electro-Spray Mass Spectrometry
mCPBA = m-chloroperoxybenzoic acid
sat = saturated
AcOH = acetic acid
MeOH = methanol
Et$_2$O = diethyl ether
Ac = acetyl
DEAD = diethyl azodicarboxylate
h = hours
Et = ethyl
WSDCC = water soluble dicarbonyl diimide, 1-(3-dimethylaminopropyl)-3-ethylcarbodiimide hydrochloride
TBAF = tetrabutylammonium fluoride
DBAD = di-terbutylazodicarboxylate
DCC = Dicyclohexylcarbodiimide
Wilkinson's catalyst = RhCl(PPh3)3
ADDP = 1,1-[azodicarbonyl]dipiperidine
DMA = dimethylacetamide
DME = 1,2-dimethoxyethane
BOP = benzotriazol-1-yloxytris(dimethylamino)-phosphonium hexafluorophosphate
HRMS = high resolution mass spectrometry
TBME = MTBE = methyl *tert*-butyl ether (i.e., 2-methoxy-2-methyl-propane)
TiCl$_2$Cp$_2$ = bis(cyclopentadienyl)titanium dichloride
DPPA = diphenylphosphoryl azide
HMPA = hexamethylphosphoryl amide
V% = volume percent
BH$_3$ DMS = borane dimethylsulfate
vvm = volume gas per volume liquid per minute

**Reference example 1**

**(3aα,4β-5α-7β,7aα)-4-(Octahydro-5-hydroxy-4,7-dimethyl-1,3-dioxo-4,7-epoxy-2H-isoindol-2-yl)-2-(trifluoromethyl)benzonitrile (1D)**

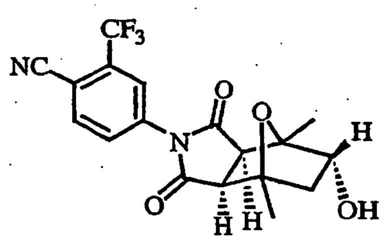

**A. 3-[[(1,1-Dimethylethyl)dimethylsilyl]oxy]-2,5-dimethylfuran (1A)**

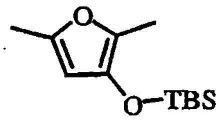

2,5-Dimethyl-3(3H)-furanone (2.00 g, 17.8 mmol) was dissolved in methylene chloride (180 mL). TEA (7.43 mL, 53.5 mmol) was added followed by TBSOTf (4.92 mL, 21.4 mmol) at 25°C. After 1 h, the reaction was concentrated *in vacuo* and the resulting slurry was run through a silica gel column conditioned with 3% TEA in hexanes. The product was eluted with 3% TEA/hezanes to give 3.6 g (89%) of compound 1A as an orange oil which was used directly in subsequent reactions.

**B. (3aα,4β,7β,7aα)-4-[5-[[(1,1-Dimethylethyl)dimethylsilyl]oxy]-1,3,3a,4,7,7a-hexahydro-4,7-dimethyl-1,3-dioxo-4,7-epoxy-2H-isoindol-2-yl]-2-(trifluoromethyl)benzonitrile (1B)**

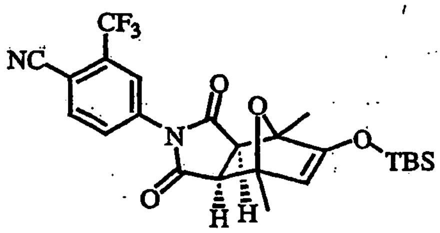

4-(2,5-Dihydro-2,5-dioxo-1H-pyrrol-1-yl)-2-trifluoromethylbenzonitrile (1.00 g, 3.85 mmol) was dissolved in benzene (5.0 mL) and the compound 1A (1.30 g, 5.77 mmol) was added. The reaction mixture was wanned to 60°C for 2 h and then cooled to 25°C. The solution was then concentrated *in vacuo* to give compound 1B as a yellow oil which was carried on to the next reaction without purification. HPLC: 60% at 4.013 min (retention time) (YMC S5 ODS column 4.6 × 50 mm eluting with 10-90% aqueous methanol over 4 minutes containing 0.2% phosphoric acid, 4 mL/min, monitoring at 220 nm).

**C. (3aα,4β,5α,7β,7aα)-4-[5-[[(1,1-Dimethylethyl)dimethylsilyl]oxy-octahydro-4,7-dimethyl-1,3-diozo-4,7-epoxy-2H-isoindol-2-yl]-2-(trifluoromethyl)benzonitrile (1C)**

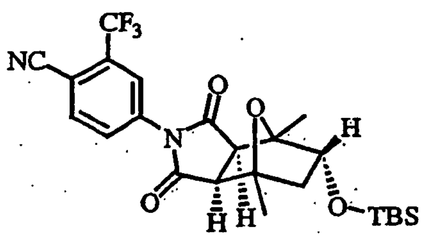

Crude compound 1B (3.85 mmol) was dissolved in ethyl acetate (75 mL) and 10% Pd/C (1.20 g) was added. Hydrogen was then introduced via a balloon. After 24 h, the reaction was filtered through Celite rinsing with ethyl acetate and concentrated *in vacuo* to give a yellow oil. The crude product was purified by flash chromatography on silica gel

eluting with methylene chloride/acetone (0%. - 1% - 2% acetone) to give 0.710 g (35%) compound 1C as a yellow solid. HPLC: 100% at 4.160 min (retention time) (YMC S5 ODS column 4.6 x 50 mm eluting with 10-90% aqueous methanol over 4 minutes containing 0.2% phosphoric acid, 4 mL/min, monitoring at 220 nm). MS (ES): m/z 517.6 $[M+Na]^+$.

**D. (3aα,4β,5α,7β,7aα)-4-(Octahydro-5-hydroxy-4,7-dimethyl-1,3-dioxo-4,7-epoxy-2H-isoindol-2-yl)-2-(trifluoromethyl)benzonitrile (1D)**

Compound 1C (0.040 g, 0.081 mmol) was dissolved in THF (1.0 mL) and HF•Pyridine (0.5 mL) was added. After 2 h, the reaction was carefully poured into cold saturated aq. $NaHCO_3$. The mixture was then extracted with methylene chloride (3 x 10 mL). The combined organics were washed with 1 N HCl (1 x 10 mL) and dried over anhydrous sodium sulfate. Concentration *in vacuo* gave 0.031 g (10%) compound 1D as a yellow solid. NOE experiments confirmed the assigned isomer. HPLC: 98% at 2.777 min (retention time) (YMC S5 ODS column 4.6 x 50 mm eluting with 10-90% aqueous methanol over 4 minutes containing 0.2% phosphoric acid, 4 mL/min, monitoring at 220 nm). MS (ES): m/z 403.06 $[M+Na]^+$.

**Reference Example 2**

**[3aR-(3aα,4β,5β,7β.7aα)]-4-(Octahydro-5-hydroxy-4,7-dimethyl-1,3-dioxo-4.7-epoxy-2H-isoindol-2-yl-2-(trifluoromethyl)benzonitrile (2Di) & [3a*S*-(3aα,4β,5β,7β,7aα)]-4-(Octahydro-5-hydroxy-4,7-dimethyl-1,3-dioxo-4,7-epoxy-2H-isoindol-2-yl)-2-(trifluoromethyl)benzonitrile (2Dii)**

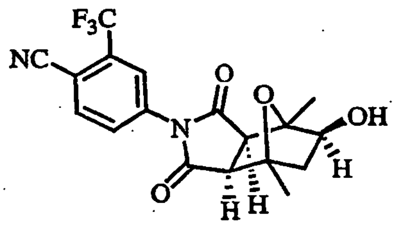
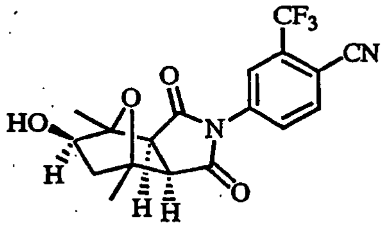

**A. 4-(2,5-Dioxo-2,5-dihydro-pyrrol-1-yl)-2-trifluoromethyl-benzonitrile (471A)**

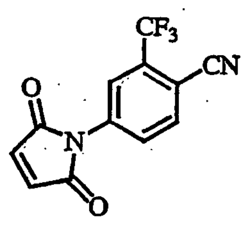

A mixture of 3-trifluoromethyl-4-cyano-aniline (24.0 g, 129 mmol) and maleic anhydride (14.0 g, 143 mmol) in 50 mL of acetic acid was heated at 115°C overnight. A precipitate was obtained during the heating period. The reaction was allowed to stand at rt for an additional overnight period. The solid was removed by filtration, the filter cake was washed with diethyl ether and dried to give 21 g (79 mmol), 61%) of compound **2A** as an off white solid. HPLC: 100% at 2.11 min (retention time) (YMC S5 ODS column, 4.6 x 50 mm, eluting with 10-90% aqueous methanol over 4 min containing 0.2% phosphoric acid, 4 mL/min, monitoring at 220 nm).

**B. (3aα,4β,β,7aα)-4-(1,3,3a,4,7,7a-Hexahydro-4,7-dimethyl-1,3-dioxo=4,7-epoxy-2H-isoindol-2-yl)-2-(trifluoromethyl)benzonitrile (2B)**

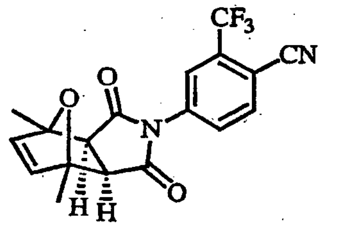

A suspension of compound **2A** (13.0 g, 48.8 mmol) and 2,5-dimethylfuran (10.5 mL, 98.6 mmol) in 50 mL of toluene was heated at 60°C, under argon. A solution was obtained on initial heating and a precipitate was observed after approximately 1 h. Heating was continued overnight. After cooling to rt, the suspension was allowed to stand at 4°C overnight. The resulting solid was filtered and the filter cake was washed with cold toluene followed by air drying to give 13.2 g of pure compound **2B** as a white solid. The filtrate volume was reduced *in vacuo* by one half and the resulting solution was treated as above to yield an additional 2.8 g of pure compound **2B** (total 16.0 g, 90%). HPLC: 90% at 3.65 min (retention time) (YMC S5 ODS column, 4.6 x 50 mm, eluting with 10-90% aqueous methanol over 4 min containing 0.2% phosphoric acid, 4 mL/mm, monitoring at 220 nm).

**C. (3aα4β,5β,7β,7aα)-4-(Octahydro-5-hydroxy-4,7-dhnethyl-1,3-dioxo-4,7-epoxy-2H-isoindol-2-yl)-2-(trifluoromethyl)benzonitrile (2C)**

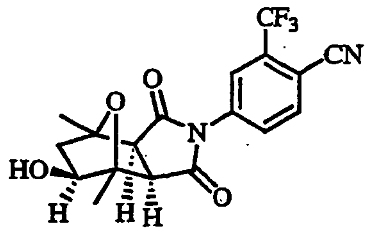

A solution of compound **2B** (25 g, 69 mmol) in 125 mL of THF, in a dry flask under nitrogen, was cooled to 10°C with an ice bath. To this solution was added neat borane-dimethylsulfide complex (13.0 mL, 138 mmol) dropwise over 10 min, while maintaining a reaction temperature of <15°C. The reaction mixture was stirred for 30 min at rt and then in an ice bath cooled to 10°C. To the cool solution was slowly added 480 mL of pH 7 phosphate buffer, which resulted in a strong exothermic reaction and vigorous gas evolution. The solution was maintained at <21°C throughout the addition by means of an ice bath. To the resulting solution was added 240 mL of ethanol and the resulting mixture was cooled to 5°C with an ice bath. To the cooled solution was added 50 mL of 30% hydrogen peroxide and the resulting mixture was stirred at 10-20°C for 1.5 h. The mixture was extracted with ethyl acetate (2 x 1 L) and the combined organic layers were washed with 10% sodium sulfite (1 x 500 mL) and brine (2 x 300 mL) and dried over $MgSO_4$. Concentration *in vacuo* afforded 29 g of crude product as a white solid. This material was subjected to flash chromatography on a 1.2 L column of silica gel equilibrated with 100% $CH_2Cl_2$-The material was applied to the column as a solution consisting of 100 mL EtOAc (warm) and 400 mL $CH_2Cl_2$. Initial elution with $CH_2Cl_2$ (3 L), followed by 25% EtOAc/75% $CH_2Cl_2$ (3 L) and finally 50% EtOAc/50% $CH_2Cl_2$ (6 L) gave 11.8 g (45%) of compound **2C** which is a racemic mixture.

Alternatively compound **2C** can be made by the following approach: A dry flask containing compound **2B** (8.90 g, 24.6 mmol) and Wilkinson's catalyst (0.57 mg, 0.62 mmol) was degassed 4x with vacuum/argon. THF (40 mL) was added to the flask and the mixture was stirred until a clear brown solution was obtained. Catecholborane (49 mL, 49 mmol, 1 M in THF) was then added dropwise over 20 min and a slight exotherm was observed. Stirring was continued for 45 min followed by cooling of the reaction mixture with an ice bath. pH 7 phosphate buffer (175 mL) was slowly added, followed by the consecutive addition of ethanol (87 mL) and 30% hydrogen peroxide (18 mL). Stirring was continued

with cooling and the reaction progress was monitored by HPLC for 4 h. The reaction was extracted with $CH_2Cl_2$ (3 x 250 mL). The combined extracts were washed with 1:1 1N NaOH:15% sodium sulfite (300 mL) and brine, dried over $MgSO_4$, and the solvent was removed *in vacuo* to afford 8.5 g of a tan solid The crude product was subjected to flash chromatography on a 500 $cm^3$ silica gel column eluting with a gradient of 25-50% EtOAc/$CH_2Cl_2$ to give 6.00 g compound **2C** (15.8 mmol, 64%) as a white solid. HPLC: 90% at 2.45 min (retention time) (YMC S5 ODS column, 4.6 x 50 mm, eluting with 10-90% aqueous methanol over 4 min containing 0.2% phosphoric acid, 4 mL/min, monitoring at 220 nm). MS (ES): m/z 381.11 $[M+H]^+$.

**D. [3aR-(3aα,4β,5β,7β,7aα)]-4-(Octahydro-5-hydroxy-4,7-dimethyl-1,3-dioxo-4,7-epoxy-2H-isoindol-2-yl)-2-(trifluoromethyl)benzonitrile (2Di) & [3aS-(3aα,4β,5β,7β,7aα)]-4-(Octahydro-5-hydroxy-4,7-dimethyl-1,3-dioxo-4,7-epoxy-2H-isoindol-2-yl)-2-(trifluoromethyl)benzonitrile (2Dii)**

The individual antipodes of compound **2C** were separated by normal phase preparative chiral HPLC (CHIRALPAK AD, 5 × 50 cm column). A 2.5 g portion of **2C** was dissolved into 25 mL of warm acetone and diluted to 50-75 mL with hexane for injection. Isocratic elution with 20% MeOH/EtOH (1:1) in heptane at 50 mL/min gave the faster eluting compound **2Di** (Chiral HPLC: 10.02 min; CHIRALPAK AD 4.6 x 250 mm column; isocratic elution with 20% MeOH/EtOH (1:1) in heptane at 1 mL/min) and the slower eluting compound **2Dii** (Chiral HPLC: 14.74 min; CHIRALPAK AD 4.6 x 250 mm column; isocratic elution with 20% MeOH/EtOH (1:1) in heptane at 1 mL/min). Compounds **2Di** & **2Dii**: HPLC: 90% at 2.45 min (retention time) (YMC S5 ODS column, 4.6 x 50 mm, eluting with 10-90% aqueous methanol over 4 min containing 0.2% phosphoric acid, 4 mL/min, monitoring at 220 nm). MS (ES): m/z 381.11 $[M+H]^+$. The absolute stereochemistry of compounds 2**Di** & **2Dii** was determined by single crystal X-ray diffraction studies and is as described by the designated nomenclature.

The resulting HPLC purified fractions of compounds **2Di** & **2Dii** were further purified by crystallization using any one of the procedures described below.

1) From Ethyl Acetate

A 700 mg portion of compound **2Di**, obtained after chiral chromatography as described above, was dissolved in ethyl acetate (10 mL) at rt. The solution was diluted with small portions of hexane (20 mL) until cloudiness was observed. The solution was allowed to stand overnight at rt The resulting white solid was filtered and air dried to afford 430 mg of compound **2Di** as a white powder. This sample was further dried at 60°C (3 h. 0.5 Torr), then at 70°C, (12 h, 0.5 Torr).

2) From Acetone

A 500 mg portion of compound **2Di**, obtained after chiral chromatography as described above, was dissolved in a minimal amount of acetone (3 mL) and slowly diluted with hexane (1 mL). The clear colorless solution was allowed to stand overnight at rt. The resulting white solid was filtered and air dried to afford 440 mg of compound **2Di** as a white powder. This sample was further dried at 60°C, (3 h, 0.5 Torr) then at 70°C, (12 h, 0.5 Torr).

3) From Methanol

A 500 mg portion of compound **2Di**, obtained after chiral chromatography as described above, was dissolved in 5 mL of hot (steam bath) methanol. The clear colorless solution was allowed to stand at rt for 2 h, then at 4°C overnight. The resulting solid was filtered, washed with minimal cold methanol and air dried for to afford 360 mg of compound **2Di** as a white powder. This sample was further dried at 70°C, (12 h, 0.5 Torr).

4) From $CH_2Cl_2$

A 7.00 g portion of compound **2Di**, obtained after chiral chromatography as described above, was dissolved in 75 mL of $CH_2Cl_2$ at rt. The clear and colorless solution was slowly diluted with hexane (48 mL) until crystallization was observed. The solution was allowed to stand at rt for 1 h, then at 4°C overnight. The resulting crystalline material was filtered and then washed with a minimal amount of cold 2:1 $CH_2Cl_2$:hexane. The large crystals were ground to a fine powder and dried at 50°C (12 h, 0.5 Torr) to yield 5.96 g of compound **2Di** as a white powder

## Reference Example 3

**(3aα,4β,5β,6α,7β,7aα)-4-(Octahydro-5,6-dihydroxy-4,7-dimethyl-1,3-dioxo-4,7-epoxy-2H-isoindol-2-yl)-2-(trifluoromethyl)benzonitrile (3B)**

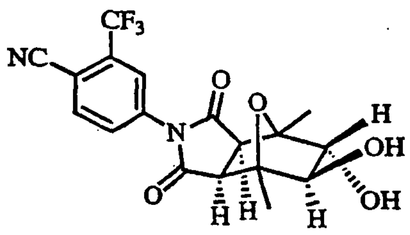

**A. (3aα,4β,5α,6β,7β,7aα)-4-(Octahydro-5-[[(1,1-dimethylethyl)dimethylsilyl]oxy]-6-hydroxy-4,7-dimethyl-1,3-dioxo-4,7-epoxy-2H-isoindol-2-yl)-2-(trifluoromethyl)benzonitrile (2A)**

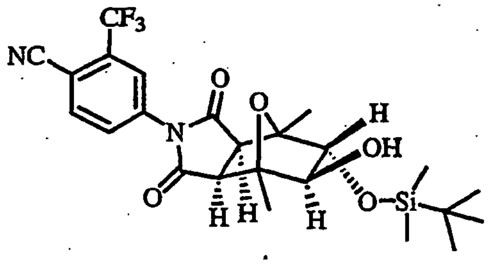

To a stirred solution of compound **1B** (1.49 g, 3 mmol) in anhydrous THF (31 mL) was added borane-methyl sufide complex (0.61 mL, 6.1 mmol) dropwise at rt under argon. After the reaction mixture was stirred at rt for 1.5 h, EtOH (20 mL) was added slowly at 0°C, followed by phosphate buffer (pH = 7.2, 39 mL), and $H_2O2$ (30% aqueous, 12 mL). The mixture was vigorously stirred at 0°C for 30 min, at rt for 21 h, and then concentrated under reduced pressure at rt to remove THF. The residue was partitioned between EtOAc (160 mL) and brine (160 mL). The organic solution was separated, washed with 5% $Na_2SO_3$ solution (120 mL), dried over $Na_2SO_4$, and concentrated under reduced pressure. Purification by silica gel flash chromatography on $SiO_2$ eluting with EtOAc/heptane (gradient from 1:8 to 1:0 ratio) compound **3A** (1.15 g, 75%) as a foam solid.

**B. [3a*S*-(3aα,4β,5β,6α,7β,7aα)]-4-(Octahydro-5,6-dihydroxy-4,7-dimethyl-1,3-dioxo-4,7-epoxy-2H-isoindol-2-yl)-2-(trifluoromethyl)benzonitrile (3B)**

To a stirred solution of compound **3A** (0.67 g, 1.3 mmol) in EtOH (100%, 19 mL) was added concentrated HCl (3.7 mL). The mixture was stirred at rt for 21 h, at 40°C for 3 h, and then concentrated under reduced pressure. Purification by silica gel flash chomatography eluting with EtOAc/heptane (gradient from 1:1 to 1:0 ratio) gave compound **3B** (0.47 mg, 92%) as a glassy solid. HPLC: 98% at 3.07 min (retention time) (YMC S5 ODS-A column 4.6 x 50 mm eluting with 10-90% aqueous methanol over 4 mincontaining 0.2% phosphoric 3 acid, 4 mL/min, monitoring at 220 nm). MS (ES): m/z 397 $[M+H]^+$. Compound **3B** represents a racemic mixture of antipodes. The nomenclature and structure shown does not reflect the absolute stereochemistry of the compound.

**Reference Example 4**

**[3a*S*-(3aα,4β,5β,6α,7β,7aα)]-4-(Octahydro-5,6-dihydroxy-4,7-dimethyl-1,3-dioxo-4,7-epoxy-2H-isoindol-2-yl)-2-(trifluoromethyl)benzonitrile & [3a*R*-(3aα,4β,5β,6β,7β,7aα)]-4-(Octahydro-5,6-dihydroxy-4,7-dimethyl-1,3-dioxo-4,7-epoxy-2H-isoindol-2-yl)-2-trifluoromethyl)benzonitrile 4i &4ii)**

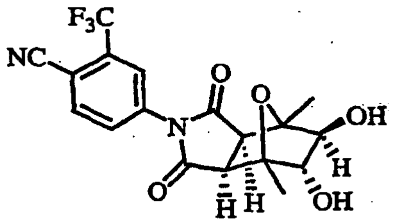

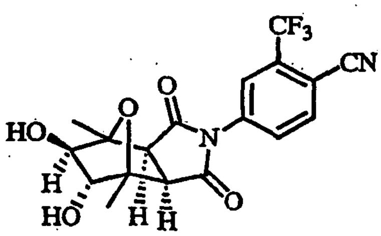

Racemic compound **3B** (1g) was separated into its enantiomers by normal phase preparative chiral HPLC (CHIRALPAK OJ 5 x 50 cm column; eluting with 20% MeOH/EtOH (1:1) in heptane (isocratic) + 0.1% diethylamine at 50 mL/min) to give 296 mg of faster eluting compound **4i** (Chiral HPLC: 8.92 min; CHIRALPAK OJ 4.6 x 250 mm column; eluting with 20% MeOH/EtOH (1:1) in heptane + 0.1% diethylamine at mLmin); HPLC: 95% at 1.25 min (retention time) (Phenomenex S5 ODS column 4.6 x 50 mm eluting with 10-90% aqueous methanol over 2 mincontaining 0.1% TFA, 4 mL/min, monitoring at 254 nm); MS (ES): m/z 397.37 $[M+H]^+$and 274 mg of the slower eluting **4ii** (Chiral HPLC: 11.25 min; CHIRALPAK OJ 4.6 x 250 mm column; eluting with 20% MeOH/EtOH (1:1) in heptane + 0.1% diethylamine at 1 mL/min); HPLC: 95% at 1.25 min (retention time) (Phenomenex S5 ODS column 4.6 x 50 mm eluting with 10-90% aqueous methanol over 2 mincontaining 0.1% TFA, 4 mL/min, monitoring at 254 nm); MS (ES): m/z 397.43 $[M+H]^+$. The absolute stereochemistry of compounds **4i** & **4ii** has not been established. Although each compound represents a single antipode, the nomenclature and structure shown does not reflect the absolute stereochemistry of the compound.

**Example 1**

**[3a*R*-(3aα,4β,7β,7aα)]-4-(Octahydro-4,7-dimethyl-1,3,5-trioxo-4,7-epoxy 2H-isoindol-2-yl)-2-(trifluoromethyl) benzonitrile (A)**

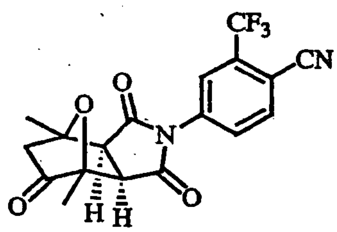

Compound **2Di** (0.10 g, 0.263 mmol) was dissolved in a mixture of $CH_2Cl_2$ (1.0 mL) and THF (2.0 mL) at 22°C. Dess-Martin periodinane (0.279 g, 0.658 mmol) was added with stirring. After 3 h, the reaction was quenched with a 1: 1 mixture of sat aq $NaHCO_3$ and sat aq $NaHSO_3$ (5 mL). After stirring for 15 min, the mixture was extracted with $CH_2Cl_2$ (3 x 10 mL) and the combined organics were dried over $Na_2SO_4$. The crude material was purified by silica gel flash chomatography eluting with 5 - 10 - 20% acetone in chloroform to give compound **A** (0.090 g) as a white solid. HPLC: 100% at 3.170 min (retention time) (YMC S5 ODS column 4.6 x 50 mm eluting with 10-90% aqueous methanol over 4 mincontaining 0.2% phosphoric acid, 4 mL/min, monitoring at 220 nm), MS (ES): m/z 379.11 $[M+H]^+$. Chiral analytical HPLC using a Chiracel OD column, 4.6X250 mm, eluting with 20% (1:1) EtOH/MeOH in hexanes at 2.0 mL/min and monitoring at 220 nm gave a retention time of 9.097 mins. The absolute stereochemistry of compound **A** is established by the known stereochemistry of the intermediate compound **2Di** and the retention of configuration therein. The absolute stereochemistry is as drawn in the above figure and rendered by the nomenclature.

**Reference Example 5**

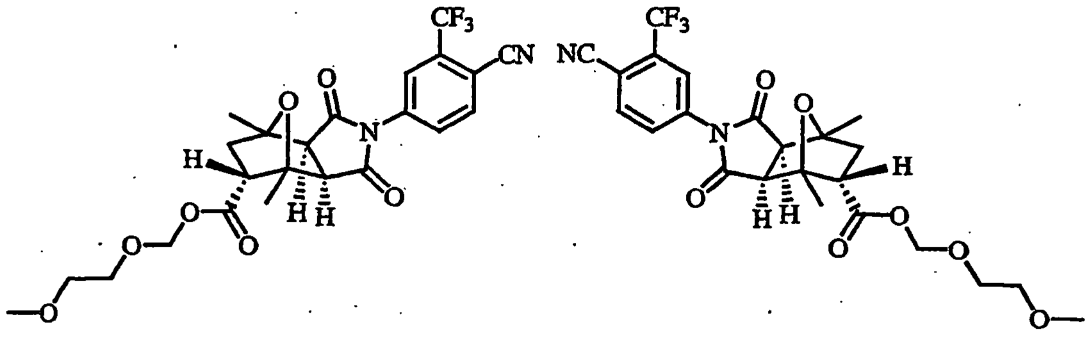

**A. 2,5-Dimethylfuran-3-carboaylic Acid 2-Methozyethoxymethyl Ester** (5**A)**

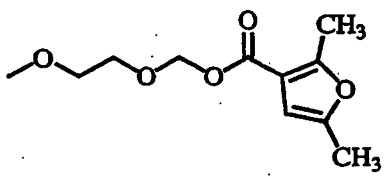

A 2 L round-bottomed flask equipped with a mechanical stirrer was charged with 2,5-dimethyl-3-furoic acid (81.0 g, 0.58 mol) and potassium carbonate (95.9 g, 0.69 mol) in DMF (500 mL). An ice bath was used to cool the reaction as 2-methoxyethoxymethyl chloride (72.2 g, 0.58 mol) was added portionwise. The reaction was stirred for 5 h at rt. The mixture was then diluted with water (1.5 L) and extracted with EtOAc (2 x 600 mL). The combined organics were washed with water (2 x 900 mL) and saturated sodium chloride solution (1 L), dried over $MgSO_4$, filtered and concentrated to afford compound 5**A** (103 g, 82%) as a yellow oil. $^1H$ NMR($CDCl_3$): δ = 6.23 (s, 1H), 5.47 (s, 1H), 3.84 (m, 2H), 3.56 (m, 2H), 3.38 (s, 3H), 2.51 (s, 3H) and 2.20 ppm (s, 3H).

**B.** (5**B**)

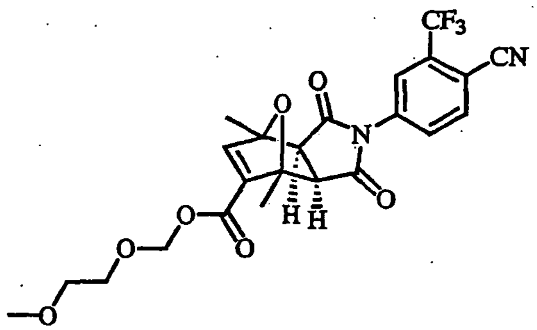

A 250 mL round-bottomed flask was charged with 4-(2,5-dioxo-2,5-dihydropyrrol-1-yl)-2-trifluoromethylbenzonitrile (15.0 g, 0.056 mol) and 2,5-dimethylfuran-3-carboxylic acid 2-methoxyethoxymethyl ester (22.8 g, 0.10 mol).

The mixture was heated to 125 °C for 1.5 h then stirred at rt overnight. The crude product was dissolved in EtOAc and adsorbed onto silica gel. Purification by silica gel chomatography, eluting with 30% EtOAc in hexanes, afforded compound **5B** (8.21 g, 30%) as a viscous oil. $^1$H NMR($CDCl_3$): δ = 7.95 (d, *J* = 8.3 Hz, 1H), 7.86 (d, *J* = 1.9 Hz, 1H), 7.75 (m, 1H), 7.13 (s, 1H), 5.44 (m, 2H), 3.83 (m, 2H), 3.57 (m, 2H); 3.39 (s, 3H), 3.19 (d, *J* = 6.5 Hz, 1H), 3.09 (d, *J* = 6.5 Hz, 1H), 2.04 (s, 3H) and . 1.91 ppm (s, 3H).

**C.** (5**C**)

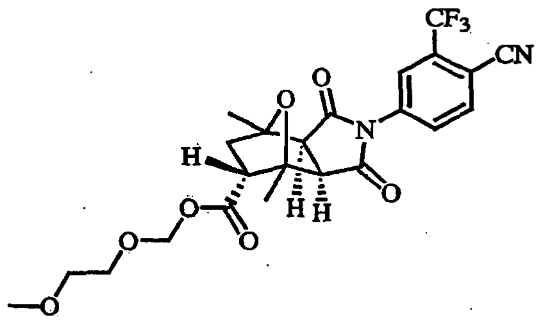

A 250 mL Parr hydrogenation bottle was charged with compound **5B** (7.75 g, 0.015 mol), EtOAc (80 mL) and palladium on carbon (0.40 g, 10% Pd, 50% wet). The bottle was placed on a Parr hydrogenation apparatus, pressurized with hydrogen to 5 psi, and shook until the uptake of hydrogen ceased. Filtration of the contents over celite and concentration afforded a yellow oil. Purification by silica gel chomatography, eluting with 30% EtOAc in hexanes, afforded compound **5C** (3.92 g, 50%) as a white solid. HPLC: 100% at 14.5 min (retention time) (Hypersil C18 BDS column, 250 x 4.6 mm 5 μm, a detection wavelength of 254 mn, and a flow rate of 1 mL/min. A linear gradient of 90% of 0.1% trifluoroacetic acid in water, 10% acetonitrile'(start) to 100% acetonitrile over 15 min, then 100% acetonitrile for 5 min was used). $R_f$ = 0.61 ($SiO_2$, 60% EtOAc in hexanes). Mpt >300 °C. $^1$H NMR($CDCl_3$): δ = 7.94 (d, *J* = 8.3 Hz, 1H), 7.84 (d, *J* = 1.9 Hz, 1H), 7.74 (m, 1H), 5.46 (d, *J* = 6.1 Hz, 1H), 5.34 (d, *J* = 6.1 Hz, 2H), 3.85 (m, 2H), 3.57 (t, *J* = 4.5 Hz, 2H), 3.38 (s, 3H), 3.33 (d, *J* = 7.2 Hz, 1H), 3.19 (d, *J* = 7.2 Hz, 1H). 3.04 (m, 1H), 2.28 (m, 1H), 2.05 (t, *J* = 12.2 Hz, 1H), 1.78 (s, 3H) and 1.64 ppm (s, 3H). m/z = 496 $[M+H]^+$.

**D. (**5**Di and** 5**Dii).**

Racemic compound **5C** was separated into its two enantiomers by preparative chiral HPLC using a Chiracel OD column (50x500 mm) eluting with 50% EtOH in Heptane at 100 mL/min and 290 nm detection. Compound **5Di** had a retention time of 6.68 min and $[\alpha]_{25}^{D}$ = +28.7° (c = 1.0, MeOH). Compound **5D**i**i** had a retention time of 13.9 min and $[\alpha]_{25}^{D}$ = -29.2° (c = 1.0, MeOH). The absolute stereochemistry of compounds **5Di** & **5Dii** has not been established. Although, each compound represents a single antipode, the nomenclature and structure shown does not reflect the absolute stereochemistry of the compound.

<u>**Reference Example 6**</u>

<u>**[3a*R*-(3aα,4β,5α,7β,7aα)]-2-(4-Cyano-3-(trifluoromethyl)phenyl)hexahydro-4,7-dimethyl-1,3-dioxo-4,7-epoxy-1H-isoindole-5-carboxylic acid (**6)</u>

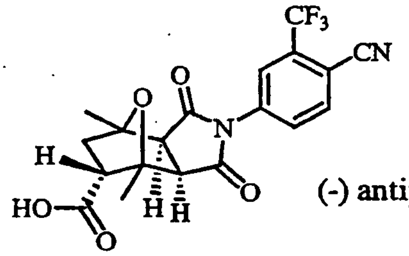

(-) antipode

A 250 mL round-bottomed flask was charged with compound 5**Di** (9.85 g, 19.8 mmol) and THF (60 mL). A solution of 3N hydrochloric acid (50 mL) was added and the reaction mixture shirred for 16 h at rt. Water (60 mL) was then added and the aqueous layer extracted with EtOAc (3 x 120 mL). The combined organics were dried over sodium sulfate, filtered and concentrated to afford compound 6 (8.00 g, 98%) which was shown to be identical to compound 4. HPLC: 100% at 13.3 min (retention time) (Hypersil C18 BDS column, 250 x 4.6 mm, 5 μm, a detection wavelength of 254 nm, and a flow rate of 1 mL/min. A linear gradient of 90% of 0.1% trifluoroacetic acid in water, 10% acetonitrile (start) to 100% acetonitrile over 15 min, then 100% acetonitrile for 5 min was used), MS (ES): m/z 409 $[M+H]^+$. $R_f$ = 0.41 ($SiO_2$, 10% MeOH in $CH_2Cl_2$). Mpt >300 °C. $[\alpha]_{25}{}^{D}$ = - 28.5° (c = 1.0, MeOH). $^1H$ NMR ($CD_3OD$): δ = 8.12 (d, *J* = 8.3 Hz, 1H), 7.92 (s, 1H), 7.82 (dd, *J* = 8.3 and 2.0 Hz, 1H), 3.39 (t, *J* = 6.7 Hz, 1H), 3.30 (d, *J* = 6.7 Hz, 1H), 3.00 (dd, *J* = 12.0 and 5.2 Hz, 1H), 2.23 (dd, *J* = 12.0 and 5.2 Hz, 1H), 2.01 (t, *J* = 12.0 Hz, 1H), 1.70 (s, 3H) and 1.57 ppm (s, 3H). The absolute stereochemistry of compound 6 has not been established. Although the compound represent a single antipode the nomenclature and structure shown does not reflect the absolute stereochemistry of the compound.

**Reference Example 7**

**[3*S*-(3aα,4β,5α-7β,7aα)-2-(4-Cyano-3-(trifluoromethyl)phenyl)hexahydro-4,7-dimethyl-1,3-dioxo-4,7-epoxy-1H-isoindole-5-carboxylic acid (7)**

(+) antipode

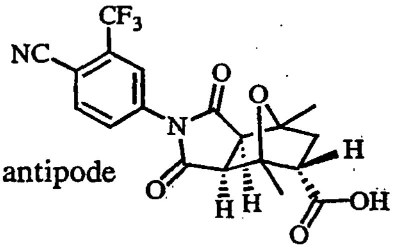

Compound 7 was prepared in identical fashion as described in reference example **6** with the exception that compound 5**Dii** was the starting material in place of compound 5**Di**. Compound 7 was obtained in 98% yield. $[\alpha]^{25}{}_{D}$ = +28.0° (c = 1.0, MeOH). The absolute stereochemistry of compound 7 has not been established. Although the compound represent a single antipode the nomenclature and structure shown does not reflect the absolute stereochemistry of the compound.

**Reference Example 8**

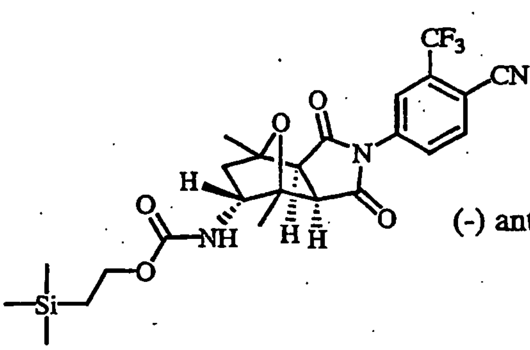

(-) antipode

A 50 mL round-bottomed flask was charged with dioxane (6 mL), compound 6 (400 mg, 0.984 mmol), diphenylphosphoryl azide (334 mg, 1.21 mmol), triethylamine (123 mg, 1.22 mmol) and powdered 4Å molecular sieves (400 mg). The resulting suspension was heated at 50 °C for 1.5 h, the temperature was then raised to 75 °C and 2-(trimethylsilyl) ethanol (590 mg, 5.02 mmol) was added. Heating was continued for an additional 1.5 h. The reaction mixture was then cooled, filtered though a celite pad and the filtrate concentrated under reduced pressure. Purification of the residue by silica gel chomatography, eluting with 20% then 40% EtOAc in hexanes, afforded compound 8 (400 mg, 78%) as a white solid. HPLC: 100% at 15.3 min (retention time) (Hypersil C18 BDS column, 250 x 4.6 mm, 5 $\mu$m, a detection wavelength of 254 nm, and a flow rate of 1 mL/min. A linear gradient of 90% of 0.1% trifluoroacetic acid in water, 10% acetonitrile (start) to 100% acetonitrile over 15 min, then 100% acetonitrile for 5 min was used), MS (ES): m/z 524 $[M+H]^+$. $R_f$ = 0.79 ($SiO_2$, 50% EtOAc in hexanes). Mpt >300 °C. $[\alpha]_{25}^D$ = -1.8° (c = 1.0, MeOH). $^1H$ NMR($CDCl_3$): $\delta$ = 7.96 (d, *J* = 8.3 Hz, 1H), 7.83 (s, 1H), 7.73 (dd, *J* = 8.3 and 2.0 Hz, 1H), 4.72 (bs, 1H), 4.19 (t, *J* = 12.0 Hz, 2H), 4.05 (m, 1H), 3.45 (d, *J* = 8.3 Hz, 1H), 3.12 (d, *J* = 8.3 Hz, 1H), 2.36 (t, *J* = 12.0 Hz, 1H), 1.61 (s, 6H), 1.04 (t, *J* = 12.0 Hz, 2H) and 0.05 ppm (s, 6H). The absolute stereochemistry of compound 8 has not been established. Although the compound represents a single antipode, the nomenclature and structure shown does not reflect the absolute stereochemistry of the compound.

## Reference Example 9

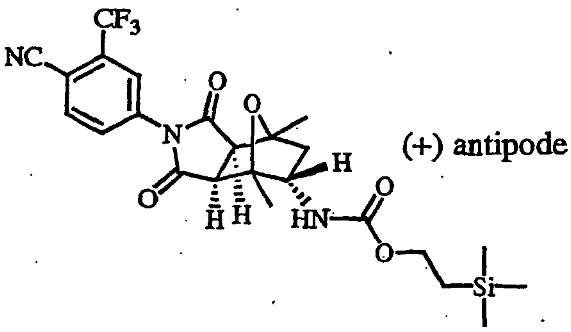

(+) antipode

Compound 9 was prepared in identical fashion as described in reference example 8 with the exception that compound 7 was the starting material in place of compound 6. Compound 9 was obtained in 80% yield. $[\alpha]^{25}_D$ = +1.1° (c = 1.0, MeOH) The absolute stereochemistry of compound 9 has not been established. Although the compound represents a single antipode, the nomenclature and structure shown does not reflect the absolute stereochemistry of the compound.

## Reference Example 10

**[3a*R*-(3a$\alpha$,4$\beta$,5$\alpha$,7$\beta$,7a$\alpha$)]-4-(Octahydro-5-amino-4,7-dimethyl-1,3-dioxo-4,7-epoxy-2H-isoindol-2-yl)-2-trifluoromethylbenzonitrile (10)**

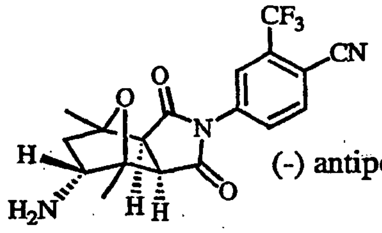

(-) antipode

A solution of compound 8 (400 mg, 0.76 mmol) in methylene chloride (10 mL) was treated with trifluoroacetic acid (2 mL) and the mixture stirred at rt for 2 h. After this time the reaction was rendered basic (pH = 9) by the addition of saturated aqueous sodium carbonate solution (20 mL) and solid potassium carbonate. The organic phase was then separated and the aqueous layer extracted with methylene chloride (3 x 40 mL). The combined organics were dried over sodium sulfate, filtered, and concentrated to afford compound 10 (283 mg, 99%) as a white solid. HPLC: 100% at 10.5 min (retention time) (Hypersil C18 BDS column, 250 x 4.6 mm, 5 μm, a detection wavelength of 254 nm, and a flow rate of 1 mL/min. A linear gradient of 90% of 0.1% trifluoroacetic acid in water, 10% acetonitrile (start) to 100% acetonitrile over 15 min, then 100% acetonitrile for 5 min was used), MS (ES): m/z 380 $[M+H]^+$. $R_f$ = 0.59 ($SiO_2$, 5% MeOH in $CH_2Cl_2$). $[\alpha]_{25}^{D}$ = -26.7° (c = 1.0, MeOH). Mpt = 150-152 °C. $^1$H NMR ($CD_3OD$): δ = 8.12 (d, *J* = 8.3 Hz, 1H), 7.94 (s, 1H), 7.83 (dd, *J* = 8.3 and 2.0 Hz, 1H), 3.66 (d, *J* = 7.2 Hz, 1H), 3.21 (m, 4H), 2.16 (t, *J* =12.0 Hz, 1H), 1.51 (s, 3H), 1.49 (s, 3H) and 1.42 ppm (dd, *J* = 12.0 and 5.0 Hz, 1H). The absolute stereochemistry of compound 10 has not been established. Although the compound represents a single antipode, the nomenclature and structure shown does not reflect the absolute stereochemistry of the compound.

**Reference Example 11**

**[3a*S*-(3aα,4β,5α,7β,7aα)]-4-(Octahydro-5-amino-4,7-dimethyl-1,3-dioxo-4,7-epoxy-2H-isoindol-2-yl)-2-(trifluoromethyl)benzonitrile (**11)

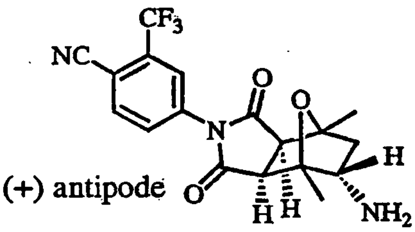

(+) antipode

Compound 11 was prepared in identical fashion as described in example 10 with the exception that compound 9 was the starting material in place of compound 8. Compound 11 was isolated in 94% overall yield. $[\alpha]^{25}_{D}$ = +27.3° (c = 1.0, MeOH). The absolute stereochemistry of compound 11 has not been established. Although the compound represents a single antipode, the nomenclature and structure shown does not reflect the absolute stereochemistry of the compound.

**Example 2**

**(3aα,4β,5α,7β,7aα)-4-(Octahydro-5-ethylsulfonamido-4,7-dimethyl-1,3-dioxo-4,7-epoxy-2H-isoindol-2-yl-2-trifluoromethyl)benzonitrile** (B)

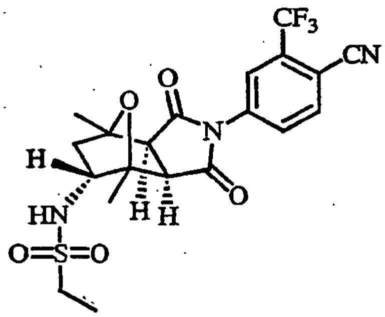

A 25 mL round-bottomed flask was charged with a racemic mixture of compounds 10 & 11 (102 mg, 0.27 mmol) and methylene chloride (10 mL). Ethanesulfonyl chloride (54.3 mg, 0.42 mmol) and triethylamine (43.6 mg, 0.43 mmol) were added and the resulting mixture stirred at rt overnight. Analysis of the reaction mixture by TLC ($SiO_2$, EtOAc) indicated the presence of starting material so an additional equivalent of ethanesulfonyl chloride (54.3 mg, 0.42 mmol) was added and stirring continued at rt for 4 h. The reaction mixture was then diluted with $CH_2Cl_2$ (25 mL) and washed with water (10 mL). Drying over magnesium sulfate, filtration, and concentration afforded a yellow oil. Purification of this oil by silica gel chomatography, eluting with 25% EtOAc in hexanes, then 50% EtOAc in hexanes and finally EtOAc, afforded compound B (55.9 mg, 44%) as a white solid. HPLC: 100% at 13.8 min (retention time) (Hypersil C18 BDS column, 250 x 4.6 mm, 5 μm, a detection wavelength of 254 nm, and a flow rate of 1 mL/min. A linear gradient of 90% of 0.1% trifluoroacetic acid in water, 10% acetonitrile (start) to 100% acetonitrile over 15 min, then 100% acetonitrile for 5 min was used), MS (ES): m/z 472 $[M+H]^+$. $R_f$ = 0.72 (EtOAc). Mpt = 189-192 °C. $^1H$ NMR($CDCl_3$): δ = 7.94 (d, *J* = 8.3 Hz, 1H), 7.85 (s, 1H), 7.74 (dd, *J* = 8.3 and 2.0 Hz, 1H), 5.72 (d, *J* = 8.3 Hz. 1H), 3.71 (m, 1H), 3.51 (d, *J* = 7.2 Hz, 1H), 3.18 (d, *J* = 7.2 Hz, 1H), 3.11 (q, *J* = 7.2 Hz, 2H), 2.37 (t, *J* = 12.0 Hz, 1H), 1.67 (m, 1H), 1.62 (s, 3H), 1.61 (s, 3H) and 1.40 ppm (t, *J* = 7.2 Hz, 3H). The absolute stereochemistry of compound B has not been established. Although the compound represents a single antipode, the nomenclature and structure shown does not reflect the absolute stereochemistry of the compound.

## Claims

**1.** A compound having the formula:

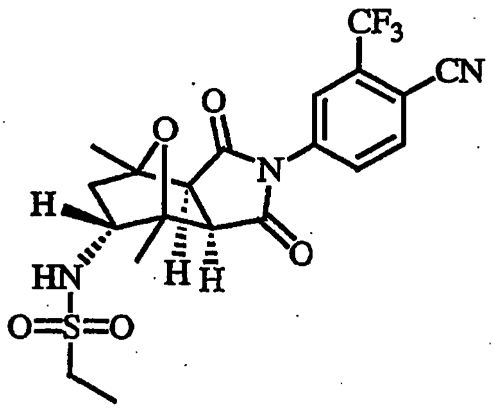

**2.** A compound having the formula:

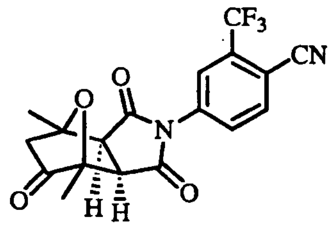

3. A pharmaceutical composition comprising at least one compound according to claim 1 or 2 or a pharmaceutically acceptable salt, or solvate, thereof; and a pharmaceutically acceptable carrier.

4. A pharmaceutical composition according to claim 3 further comprising another anti-cancer agent.

5. Use of at least one compound as defined in claim 1 or 2 or a pharmaceutically acceptable salt, or solvate, thereof for the preparation of a medicament for treating a condition or disorder selected from the group consisting of proliferate diseases, cancers, benign prostate hypertrophia, adenomas and neoplasies of the prostate, benign or malignant tumor cells containing the androgen receptor, heart disease, angiogenic conditions or disorders, hirsutism, acne, hyperpilosity, inflammation, immune modulation, seborrhea, endometriosis, polycystic ovary syndrome, androgenic alopecia, hypogonadism, osteoporosis, suppressing spermatogenisis, libido, cachexia, anorexia, inhibition of muscular atrophy in ambulatory patients, androgen supplementation for age related decreased testosterone levels in men, cancers expressing the estrogen receptor, prostate cancer, breast cancer, endometrial cancer, hot flushes, vaginal dryness, menopause, amennoreahea, dysmennoreahea, contraception, pregnancy termination, cancers containing the progesterone receptor, cyclesynchrony, meniginoma, fibroids, labor induction, autoimmune diseases, Alzheimer's disease, psychotic disorders, drug dependence, non-insulin dependent Diabetes Mellitus, dopamine receptor mediated disorder, congestive heart failure, disregulation of cholesterol homeostasis, and attenuating the metabolism of a pharmaceutical agent.

6. Use according to claim 5, wherein the condition or disorder is prostate cancer.

7. A compound as defined in claim 1 or 2 or a pharmaceutically acceptable salt or solvate thereof for treating a condition or disorder selected from the group consisting of proliferate diseases, cancers, benign prostate hypertrophia, adenomas and neoplasies of the prostate, benign or malignant tumor cells containing the androgen receptor, heart disease, angiogenic conditions or disorders, hirsutism, acne, hyperpilosity, inflammation, immune modulation, seborrhea, endometriosis, polycystic ovary syndrome, androgenic alopecia, hypogonadism, osteoporosis, suppressing spermatogenisis, libido, cachexia, anorexia, inhibition of muscular atrophy in ambulatory patients, androgen supplementation for age related decreased testosterone levels in men, cancers expressing the estrogen receptor, prostate cancer, breast cancer, endometrial cancer, hot flushes, vaginal dryness, menopause, amennoreahea, dysmennoreahea, contraception, pregnancy termination, cancers containing the progesterone receptor, cyclesynchrony, meniginoma, fibroids, labor induction, autoimmune diseases Alzheimer's disease psychotic disorders drug dependence, non-insulin dependent Diabetes Mellitus, dopamine receptor mediated disorders, congestive heart failure, disregulation of cholesterol homeostasis, and attenuating the metabolism of a pharmaceutical agent.

8. A compound as defined in claim 1 or 2 or a pharmaceutically acceptable salt, or solvate thereof for treating prostate cancer.

## Patentansprüche

1. Verbindung mit der Formel:

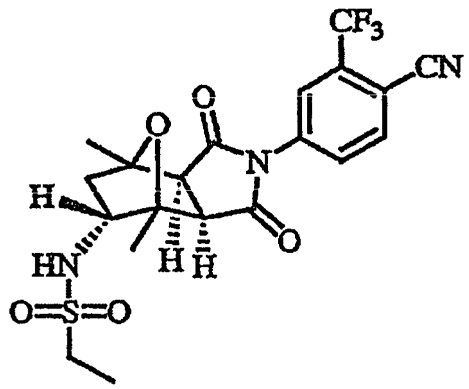

2. Verbindung mit der Formel:

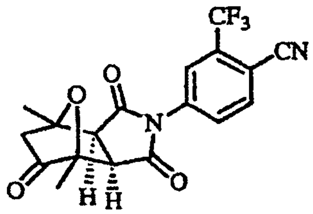

3. Pharmazeutische Zusammensetzung, umfassend mindestens eine Verbindung nach Anspruch 1 oder 2 oder ein pharmazeutisch verträgliches Salz oder Solvat davon; und einen pharmazeutisch verträglichen Träger.

4. Pharmazeutische Zusammensetzung nach Anspruch 3, ferner umfassend ein anderes Antikrebsmittel.

5. Verwendung von mindestens einer Verbindung nach Anspruch 1 oder 2 oder eines pharmazeutisch verträglichen Salzes, oder Solvats davon für die Herstellung eines Arzneimittels zur Behandlung eines Zustands oder einer Störung, die aus der Gruppe ausgewählt ist, bestehend aus: proliferativen Erkrankungen, Krebserkrankungen, benigner Prostatahypertrophie, Adenomen und Neoplasien der Prostata, den Androgenrezeptor enthaltenden benignen oder malignen Tumorzellen, Herzerkrankung, angiogenen Zuständen oder Störungen, Hirsutismus, Akne, Hyperpilosität, Entzündung, Immunmodulation, Seborrhoe, Endometriose, polyzystischem Ovarialsyndrom, androgenetischer Alopezie, Hypogonadismus, Osteoporose, Suppression der Spermatogenese, Libido, Kachexie, Anorexie, Inhibition der Muskelatrophie bei gehfähigen Patienten, Androgensupplementierung bei altersbedingten verringerten Testosteronspiegeln bei Männern, den Estrogenrezeptor exprimierenden Krebserkrankungen, Prostatakrebs, Brustkrebs, Endometrialkrebs, Hitzewallungen, vaginaler Trockenheit, Menopause, Amenorrhoe, Dysmenorrhoe, Kontrazeption, Schwangerschaftsabbruch, den Progesteronrezeptor enthaltenden Krebserkrankungen, Zyklus-Synchronie, Meningeom, Fibroiden, Geburtseinleitung, Autoimmunerkrankungen, Alzheimer-Krankheit, psychotischen Störungen, Arzneimittel-/Substanzabhängigkeit, nicht insulinabhängigem Diabetes mellitus, Dopaminrezeptor-vermittelten Störungen, dekompensierter Herzinsuffizienz, Dysregulation der Cholesterinhomöostase und Abschwächung des Metabolismus eines Pharmazeutikums.

6. Verwendung nach Anspruch 5, worin der Zustand oder die Störung Prostatakrebs darstellt.

7. Verbindung wie in Anspruch 1 oder 2 definiert oder ein pharmazeutisch verträgliches Salz oder Solvat davon zur Behandlung eines Zustands oder einer Störung, die aus der Gruppe ausgewählt ist, bestehend aus: proliferativen Erkrankungen, Krebserkrankungen, benigner Prostatahypertrophie, Adenomen und Neoplasien der Prostata, den Androgenrezeptor enthaltenden benignen oder malignen Tumorzellen, Herzerkrankung, angiogenen Zuständen oder Störungen, Hirsutismus, Akne, Hyperpilosität, Entzündung, Immunmodulation, Seborrhoe, Endometriose, polyzystischem Ovarialsyndrom, androgenetischer Alopezie, Hypogonadismus, Osteoporose, Suppression der Spermatogenese, Libido, Kachexie, Anorexie, Inhibition der Muskelatrophie bei gehfähigen Patienten, Androgensupplementierung bei altersbedingten verringerten Testosteronspiegeln bei Männern, den Estrogenrezeptor exprimierenden Krebserkrankungen, Prostatakrebs, Brustkrebs, Endometrialkrebs, Hitzewallungen, vaginaler Trockenheit, Menopause, Amenorrhoe, Dysmenorrhoe, Kontrazeption, Schwangerschaftsabbruch, den Progesteronrezeptor enthaltenden Krebserkrankungen, Zyklus-Synchronie, Meningeom, Fibroiden, Geburtseinleitung, Autoimmunerkrankungen, Alzheimer-Krankheit, psychotischen Störungen, Arzneimittel-/Substanzabhängigkeit, nicht insulinabhängigem Diabetes mellitus, Dopaminrezeptor-vermittelten Störungen, dekompensierter Herzinsuffizienz, Dysregulation der Cholesterinhomöostase und Abschwächung des Metabolismus eines Pharmazeutikums.

8. Verbindung wie in Anspruch 1 oder 2 definiert oder ein pharmazeutisch verträgliches Salz oder Solvat davon zur Behandlung des Prostatakrebses.

## Revendications

1. Composé ayant pour formule:

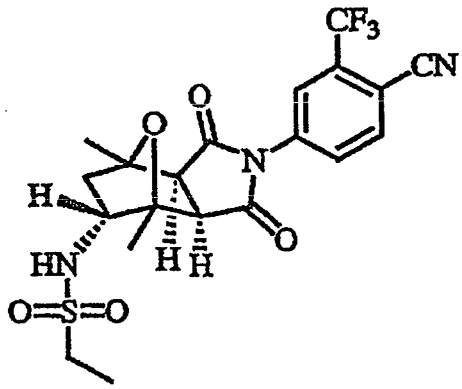

2. Composé ayant pour formule:

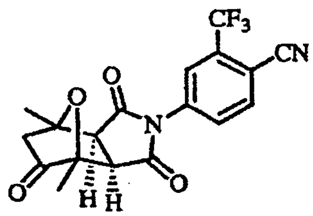

3. Composition pharmaceutique comprenant au moins un composé selon la revendication 1 ou 2 ou un de ses sels ou solvates pharmaceutiquement acceptables; et un véhicule pharmaceutiquement acceptable.

4. Composition pharmaceutique selon la revendication 3 comprenant en outre un autre agent anticancéreux.

5. Utilisation d'au moins un composé tel que défini dans la revendication 1 ou 2 ou d'un de ses sels ou solvates pharmaceutiquement acceptables, pour la préparation d'un médicament indiqué pour traiter un état ou un trouble choisi dans le groupe comprenant les maladies prolifératives, les cancers, l'hypertrophie prostatique bénigne, les adénomes et les néoplasies prostatiques, les cellules des tumeurs bénignes ou malignes contenant le récepteur des androgènes, la cardiopathie, les états ou troubles angiogéniques, l'hirsutisme, l'acné, l'hyperpilosité, l'inflammation, la modulation immunitaire, la séborrhée, l'endométriose, le syndrome des ovaires polykystiques, l'alopécie androgénique, l'hypogonadisme, l'ostéoporose, la suppression de la spermatogenèse, la libido, la cachexie, l'anorexie, l'inhibition de l'atrophie musculaire chez les patients ambulatoires, la supplémentation en androgènes pour lutter contre la diminution des taux de testostérone liée à l'âge chez l'homme, les cancers exprimant le récepteur des oestrogènes, le cancer de la prostate, le cancer du sein, le cancer endométrial, les bouffées de chaleur, la sécheresse vaginale, la ménopause, l'aménorrhée, la dysménorrhée, la contraception, l'interruption de grossesse, les cancers contenant le récepteur de la progestérone, la synchronisation du cycle, le méningiome, les fibromes, le déclenchement du travail, les maladies autoimmunes, la maladie d'Alzheimer, les troubles psychotiques, la pharmacodépendance, le diabète sucré non insuline-dépendant, les troubles médiés par le récepteur de la dopamine,

l'insuffisance cardiaque congestive, le dérèglement de l'homéostase du cholestérol, et l'atténuation du métabolisme d'un agent pharmaceutique.

**6.** Utilisation selon la revendication 5, dans laquelle l'état ou le trouble est le cancer de la prostate.

**7.** Composé tel que défini dans la revendication 1 ou 2 ou un de ses sels ou solvates pharmaceutiquement acceptables, pour traiter un état ou un trouble choisi dans le groupe comprenant les maladies prolifératives, les cancers, l'hypertrophie prostatique bénigne, les adénomes et les néoplasies prostatiques, les cellules des tumeurs bénignes ou malignes contenant le récepteur des androgènes, la cardiopathie, les états ou troubles angiogéniques, l'hirsutisme, l'acné, l'hyperpilosité, l'inflammation, la modulation immunitaire, la séborrhée, l'endométriose, le syndrome des ovaires polykystiques, l'alopécie androgénique, l'hypogonadisme, l'ostéoporose, la suppression de la spermatogenèse, la libido, la cachexie, l'anorexie, l'inhibition de l'atrophie musculaire chez les patients ambulatoires, la supplémentation en androgènes pour lutter contre la diminution des taux de testostérone liée à l'âge chez l'homme, les cancers exprimant le récepteur des oestrogènes, le cancer de la prostate, le cancer du sein, le cancer endométrial, les bouffées de chaleur, la sécheresse vaginale, la ménopause, l'aménorrhée, la dysménorrhée, la contraception, l'interruption de grossesse, les cancers contenant le récepteur de la progestérone, la synchronisation du cycle, le méningiome, les fibromes, le déclenchement du travail, les maladies autoimmunes, la maladie d'Alzheimer, les troubles psychotiques, la pharmacodépendance, le diabète sucré non insulino-dépendant, les troubles médiés par le récepteur de la dopamine, l'insuffisance cardiaque congestive, le dérèglement de l'homéostase du cholestérol, et l'atténuation du métabolisme d'un agent pharmaceutique.

**8.** Composé tel que défini dans la revendication 1 ou 2 ou un de ses sels ou solvates pharmaceutiquement acceptables, pour traiter le cancer de la prostate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9935246 A, Evans
- WO 9749709 A
- US 5696133 A
- US 5696130 A
- US 5696127 A
- US 5693647 A
- US 5693646 A
- US 5688810 A
- US 5688808 A
- WO 9619458 A
- US 5817679 A, Shen
- US 4476184 A, Lubowitz
- WO 9827423 A, Buchmeiser
- JP 07144477 B
- JP 63170383 B
- JP 62053963 B
- JP 62053964 B
- JP 53086035 B
- JP 51088631 B
- JP 49124225 B
- DD 101271
- FR 2031538
- WO 9829495 A, Amos
- US 4670536 A, Odagiri
- EP 355435 A
- US 3821232 A, Redmore, D.
- JP 01061457 A
- JP 09194458 B
- US 5523277 A, Condon
- JP 04290868 B
- JP 04149173 B
- JP 04134063 B
- JP 04134062 B
- US 3995099 A, Norman G. Gaylord
- US 5961925 A, Waldemar Ruediger, Wen-Jeng Li, John W., Allen Jr., and Harold N. Weller III
- US 3239345 A
- US 4036979 A
- US 4411890 A
- WO 8907110 A
- WO 8907111 A
- WO 9304081 A
- US 09519079 B
- US 5541204 A
- US 5770615 A
- US 5491134 A
- US 5776983 A
- US 5488064 A
- WO 9721993 A
- WO 9900353 A
- GB 98284425 A
- US 6184231 B1
- US 5612359 A
- US 6043265 A
- WO 0001389 A
- US 5179080 A
- DE 4138042
- WO 9719086 A
- WO 9822461 A
- WO 9825929 A
- WO 9838192 A
- WO 9901124 A
- WO 9902224 A
- WO 9902514 A
- WO 9903848 A
- WO 9907692 A
- WO 9927890 A
- WO 9928324 A
- WO 9943653 A
- WO 9954330 A
- WO 9954318 A
- WO 9954319 A
- WO 9965913 A
- WO 9967252 A
- WO 9967253 A
- WO 0000485 A
- WO 9924416 A
- US 6040321 A
- WO 9730992 A
- WO 9854966 A
- US 6011029 A
- US 28443801 P
- US 88582701 A
- US 88583101 A


### Non-patent literature cited in the description


- **EVANS.** *Science,* 1988, vol. 240, 889-895
- **JONAT et al.** *Cell,* 1990, vol. 62, 1189-1204
- **SCHUELE et al.** *Cell,* 1990, vol. 62, 1217-1226

- **YANG-YEN et al.** *Cell,* 1990, vol. 62, 1205-1215
- **MANGELSDORF et al.** *Cell,* 1995, vol. 83, 835-839
- **O'MALLEY et al.** *Mol. Endocrinol.,* 1996, vol. 10, 1293
- **ENMARK et al.** *Mol. Endocrinol.,* 1996, vol. 10, 1293-1307
- **GIGUERE.** *Endocrin. Rev.,* 1999, vol. 20, 689-725
- **WURTZ et al.** *Nature Struc. Biol.,* 1996, vol. 3, 87-94
- **PARKER et al.** *Nature Struc. Biol.,* 1996, vol. 3, 113-115
- **KUMAR et al.** *Steroids,* 1999, vol. 64, 310-319
- **WALLER et al.** *Toxicol. Appl. Pharmacol.,* 1996, vol. 137, 219-227
- **MEKENYAN et al.** *Environ. Sci. Technol.,* 1997, vol. 31, 3702-3711
- **VEGETO et al.** *Cell,* 1992, vol. 69, 703-713
- **NERI et al.** *Endo.,* 1972, vol. 91, 427-437
- **SMIGEL.** *J. Natl. Cancer Inst.,* 1998, vol. 90, 647-648
- **GRESE et al.** *Proc. Natl. Acad. Sci. USA,* 1997, vol. 94, 14105-14110
- **REGAL et al.** *Proc. Soc. Exp. Biol. Med.,* 2000, vol. 223, 372-378
- **HEMPSTOCK et al.** *J. Med. Food,* 1999, vol. 2, 267-269
- **QUELLA et al.** *J. Clin. Oncol.,* 2000, vol. 18, 1068-1074
- **BANZ et al.** *J. Med. Food,* 1999, vol. 2, 271-273
- **BOURGUET et al.** *Nature,* 1995, vol. 375, 377-382
- **BRZOZOWSKI et al.** *Nature,* 1997, vol. 389, 753-758
- **SHIAU et al.** *Cell,* 1998, vol. 95, 927-937
- **TANENBAUM et al.** *Proc. Natl. Acad. Sci. USA,* 1998, vol. 95, 5998-6003
- *J. Med. Chem.,* 1999, vol. 41, 623
- *J. Med. Chem.,* 1963, vol. 6 (6), 802-805
- **GREENE, T. W. et al.** Protective Groups in Organic Synthesis. Wiley, 1991
- **LI et al.** *Eur. J. Org. Chem,* 1998, vol. 9, 1841-1850
- **LI, Y-Q.** *Synlett.,* 1996, vol. 5, 461-464
- **THIEMANN et al.** *Bull. Chem. Soc. Jpn.,* 1994, vol. 67, 1886-1893
- **TSUGE et al.** *Heterocycles,* 1980, vol. 14, 423-428
- **WARD et al.** *Can J. Chem.,* 1997, vol. 75, 681-693
- **WARD et al.** *Can J. Chem.,* 1991, vol. 69, 1487-1497
- **WARD et al.** *Tetrahedron Lett.,* 1990, vol. 31, 845-848
- **FLEMING et al.** *J. Org. Chem.,* 1979, vol. 44, 2280-2282
- **JANKOWSKI et al.** *J. Organomet. Chem.,* 2000, vol. 595, 109-113
- **KEGLEVICH et al.** *J. Organomet. Chem.,* 1999, vol. 579, 182-189
- **KEGLEVICH et al.** *J. Organomet. Chem.,* 1998, vol. 570, 49-539
- **JANKOWSKI et al.** *Hetroat. Chem.,* 1996, vol. 7, 369-374
- **JANKOWSKI et al.** *J. Am. Chem. Soc.,* 1991, vol. 113, 7011-7017
- **QUIN et al.** *Tetrahedron Lett.,* 1990, vol. 31, 6473-6476
- **QUIN et al.** *J. Org. Chem.,* 1994, vol. 59, 120-129
- **QUIN et al.** *J. Org. Chem.,* 1993, vol. 58, 6212-6216
- **QUIN et al.** *Phosphorous, Sulfur Silicon Relat. Elem.,* 1991, vol. 63, 349-362
- **QUIN et al.** *Hetroat. Chem.,* 1991, vol. 2, 359-367
- **HUSSONG et al.** *Phosphorus Sulfur.,* 1985, vol. 25, 201-212
- **QUIN et al.** *J. Org. Chem.,* 1986, vol. 51, 3341-3347
- **MYERS et al.** *J. Am. Chem. Soc.,* 1992, vol. 114, 5684-5692
- **MYERS et al.** *J. Am. Chem. Soc.,* 1991, vol. 113, 6682-6683
- **CORDONE et al.** *J. Am. Chem. Soc.,* 1989, vol. 111, 5969-5970
- **JUNG et al.** *J. Chem. Soc. Commun.,* 1984, 630-632
- **LAY et al.** *J. Am. Chem. Soc.,* 1982, vol. 104, 7658-7659
- **GONZALEZ et al.** *J. Am. Chem. Soc.,* 1995, vol. 117, 3405-3421
- **KREHER et al.** *Chem. Ber.,* 1992, vol. 125, 183-189
- **SIMIG et al.** *Synlett.,* 1990, vol. 7, 425-426
- **SHA et al.** *J. Org. Chem.,* 1990, vol. 55, 2446-2450
- **DREW et al.** *J. Chem. Soc., Perkin Trans.,* 1985, vol. 17, 1277-1284
- **KREHER et al.** *Anorg. Chem., Org Chem.,* 1976, vol. 31B, 599-604
- **AVALOS et al.** *Tetrahedron Lett.,* 1998, vol. 39, 9301-9304
- **GOUSSE et al.** *Macromolecules,* 1998, vol. 31, 314-321
- **MIKHAILYUCHENKO et al.** *Khim. Geterotsikl. Soedin.,* 1993, vol. 6, 751-758
- **PADWA et al.** *J. Org. Chem.,* 1996, vol. 61, 3706-3714
- **SCHLESSINGER et al.** *J. Org. Chem.,* 1994, vol. 59, 3246-3247

- **GOUSSE et al.** *Polym. Int.,* 1999, vol. 48, 723-731
- **PADWA et al.** *J. Org. Chem.,* 1997, vol. 62, 4088-4096
- **THEURILLAT-MORITZ et al.** *Tetrahedron: Asymmetry,* 1996, vol. 7, 3163-3168
- **MATHEWS et al.** *J. Carbohydr. Chem.,* 1995, vol. 14, 287-97
- **SRIVASTAVA et al.** *Natl. Acad. Sci. Lett. (India),* 1992, vol. 15, 41-44
- **MAYORGA et al.** *Rev. Cubana Quim.,* 1988, vol. 4, 1-6
- **KONDOLI et al.** *J. Chem. Res., Synop.,* 1987, vol. 3, 76
- **PRIMELLES et al.** *Cent. Azucar,* 1985, 7-14
- **SOLOV'EVA et al.** *Khim. Geterotsikl. Soedin.,* 1984, vol. 5, 613-15
- **LIU et al.** *Yaoxue Xuebao,* 1983, vol. 18, 752-759
- **JOSHI et al.** *Indian J. Chem, Sect. B.,* 1983, vol. 22B, 131-135
- **NAKANO et al.** *Heterocycles,* 1993, vol. 35, 37-40
- **TOMISAWA et al.** *Chem. Pharm. Bull.,* 1988, vol. 36, 1692-1697
- **KROW et al.** *J. Heterocycl. Chem.,* 1985, vol. 22, 131-135
- **KROW et al.** *J. Org. Chem.,* vol. 47, 1989-1993
- **RICE et al.** *J. Med. Chem.,* 1968, vol. 11, 183-185
- **HOFMAN et al.** *J. Agric. Food Chem.,* 1997, vol. 45, 898-906
- **BACIOCCHI et al.** *J. Chem. Soc., Perkin Trans.,* 1975, vol. 2 (8), 821-824
- **WU et al.** *J. Heterocycles,* 1994, vol. 38, 1507-1518
- **YIN et al.** *Tetrahedron Lett.,* 1997, vol. 38, 5953-5954
- **MIC'OVIC et al.** *Tetrahedron,* 1964, vol. 20, 2279-2287
- **GORBUNOVA et al.** *J. Org. Chem.,* 1999, vol. 35, 1557-1566
- **RASSU et al.** *Chem. Soc. Rev.,* 2000, vol. 29, 109-118
- **KABERDIN et al.** *Chem. Rev.,* 1999, vol. 68, 765-779
- **BARLUENGA et al.** *Aldrichimica Acta,* 1999, vol. 32, 4-15
- **BOGDANOWICZ-SZWED et al.** *Pol. Wiad. Chem.,* 1998, vol. 52, 821-842
- **CASIRAGHI et al.** *Adv. Asymmetric Synth.,* 1998, vol. 3, 113-189
- **BAECKVALL et al.** *Chem. Rev.,* 1998, vol. 98, 2291-2312
- **DESHPANDE et al.** *Heterocycles,* 1999, vol. 51, 2159-2162
- **SEIJAS et al.** *J. Chem. Res., Synop.,* 1999, vol. 7, 420-421
- **LANGER et al.** *Eur. J. Org. Chem.,* 1998, vol. 7, 1467-1470
- **LOPEZ-ALVARADO et al.** *J. Org. Chem.,* 1996, vol. 61, 5865-5870
- **PASTOR et al.** *J. Org. Chem.,* 1988, vol. 53, 5776-5779
- **TAKAHASHI et al.** *Chem. Lett.,* 1987, vol. 6, 1229-1232
- **KOHLER, E. P. ; TISHLER, M. ; POTTER, H. ; THOMPSON, H. T.** *J. Am. Chem. Soc.,* 1939, 1057-1061
- **YUR'EV, Y. K. ; ZEFIROV, N. S.** *J. Gen. Chem. U.S.S.R. (Engl. Transl.),* 1961, vol. 31, 772-5
- **SCHUELER, P. E. ; RHODES, Y. E.** *J. Org. Chem.,* 1974, vol. 39, 2063-9
- **ISHITOBI, H. ; TANIDA, H ; TSUJI, T.** *Bull. Client. Soc. Japan,* 1971, vol. 44, 2993-3000
- **STÁJER, G. ; VIRÁG, M.; ; SZABÓ, A. E. ; BERNÁTH, G. ; SOHÁR, P. ; SILLANPÄÄ, R.** *Acta. Chem. Scand.,* 1996, vol. 50, 922-30
- **HART, H. ; GHOSH, T.** *Tetrahedron Lett.,* 1988, vol. 29, 881-884
- **KATO, M. ; YAMAMOTO, S. ; YOSHIHARA, T. ; FURUICHI, K ; MIWA, T.** *Chem. Lett.,* 1987, 1823-1826
- **KOTTWITZ, J. ; VORBRÜGGEN, H.** *Synthesis,* 1995, 636-637
- **CREARY, X.** *J. Org. Chem.,* 1975, vol. 40, 3326-3331
- **ALDER, K. ; ACHE, H.-J. ; FLOCK, F. H.** *Chem. Ber.,* 1960, vol. 93, 1888-1895
- **TODER, B. H. ; BRANCA, S. J. ; DIETER, R. K. ; SMITH, A. B.** *III Synth. Commun.,* 1975, vol. 5, 435-439
- **SPRAGUE, P. W. ; HEIKES, J. E. ; GOUGOUTAS, J. Z. ; MALLEY, M. F. ; HARRIS, D. N. ; GREENBERG, R.** *J. Med. Chem.,* 1985, vol. 28, 1580-1590
- **SPRAGUE et al.** *J. Med. Chem.,* 1985, vol. 28, 1580-1590
- **RETEMI et al.** *J. Org. Chem.,* 1996, vol. 61, 6296-6301
- Advanced Organic Chemistry, Reactions, Mechanisms, and Structure. John Wiley & Sons, 1992, 293-500
- *Biophys. Acta,* 1990, vol. 187, 1052
- *J. Urol.,* 1999, vol. 162, 2192
- *Mol. Eildo.,* 1997, vol. 11, 450
- *J. Clin. Endocrinol. Metab.,* 1997, vol. 82, 727-34
- **EDWARDS, J. P. et al.** *Bio. Med. Chem. Let.,* 1999, vol. 9, 1003-1008
- **HAMANN, L. G. et al.** *J. Med. Chem.,* 1999, vol. 42, 210-212
- **T. BERGER et al.** *J. Steroid Biochem. Molec. Biol.,* 1992, 773

- **SCHUUR et al.** *J. Biol. Chem.,* 1996, vol. 271 (12), 7043-51
- **G. CHALEPAKIS et al.** *Cell,* 1988, vol. 53 (3), 371
- **D. RODBARD.** Mathematics and statistics of ligand assays: an illustrated guide. Masson Publishing U.S.A., Inc, 1981, 45-99
- **NAVONE et al.** *Clin. Cancer Res.,* 1997, vol. 3, 2493-500
- **BROWN.** *The Journal of Biological Chemisty,* 1997, vol. 272, 8227-8235
- **HIRAOKA et al.** *Cancer Res.,* 1987, vol. 47, 6560-6564
- **TETUO.** *Cancer Research,* 1965, vol. 25, 1168-1175
- **SUZUKI.** *J. Steroid Biochem. Mol. Biol.,* 1990, vol. 37, 559-567
- **L. G. HERSHBERGER et al.** *Proc. Soc. Expt. Biol. Med.,* 1953, vol. 83, 175
- **P. C. WALSH ; R. F. GITTES.** Inhibition of extratesticular stimuli to prostate growth in the castrated rat by antiandrogens. *Endocrinology,* 1970, vol. 86, 624
- **B. J. FURR et al.** *ICI,* vol. 176, 334
- *J. Endocrinol.,* 1987, vol. 113, R7-9
- **F. LABRIE et al.** *Clin. Invest. Med.,* 1993, vol. 16, 475-492
- **M. C. LUKE ; D. S. COFFEY.** The Physiology of Reproduction. 1994, vol. 1, 1435-1487
- **Y. OKUDA et al.** *J. Urol.,* 1991, vol. 145, 188-191
- **L. G. HERSHBERGER et al.** *Proc. Soc. Expt. Biol. Med.,* 1953, vol. 83, 175
- **B. L. BEYLER et al.** Methods for evaluating anabolic and catabolic agents in laboratory animals. *J. Amer. Med. Women's Ass.,* 1968, vol. 23, 708
- **H. FUKUDA et al.** Investigations of the levator ani muscle as an anabolic steroid assay. *Nago Dai. Yak. Ken. Nem.,* 1966, vol. 14, 84
- **M. MASUOKA et al.** Constant cell population in normal, testosterone deprived and testosterone stimulated levator ani muscles. *Am. J. Anat.,* 1966, vol. 119, 263
- **Z. GORI et al.** Testosterone hypertrophy of levator ani muscle of castrated rats. I. Quantitative data. *Boll. -Soc. Ital. Biol. Sper.,* 1966, vol. 42, 1596
- **Z. GORI et al.** Testosterone hypertrophy of levator ani muscle of castrated rats. II. Electron-microscopic observations. *Boll. -Soc. Ital. Biol. Sper.,* 1966, vol. 42, 1600
- **A. BORIS et al.** *Steroids,* 1970, vol. 15, 61
- **SMOLEV JK ; HESTON WD ; SCOTT WW ; COFFEY DS.** *Cancer Treat Rep.,* 1977, vol. 61, 273-287
- **MAUCHER A. ; ANGERER.** *J. Cancer Res. Clin. Oncol.,* 1993, vol. 119, 669-674
- **FURR B.J.A.** *Euro. URL.,* 1990, vol. 18 (3), 2-9
- **SHAIN S.A. ; HUOT RI.** *J. Steriod Biochem.,* 1988, vol. 31, 711-718
- **F. LABRIE.** *Clin. Invest. Med.,* 1993, vol. 16, 475-492